# EUROPEAN PATENT APPLICATION

(11) **EP 2 270 056 A2**
(43) Date of publication of application: **05.01.2011**
(21) Application number: 10179778.5
(22) Date of filing: 01.02.2006
(51) Int. Cl.: C08B 37/00, C12P 19/04

(54) **Purification of streptococcal capsular polysaccharide**

(30) Priority: 01.02.2005 GB 0502096
(62) Divisional of application: 06710574.2
(71) Applicant: Novartis Vaccines and Diagnostics S.r.l., 53100 Siena (SI) (IT)
(72) Inventor: Costantino, Paolo, I-53100 Siena (IT)
(74) Representative: Marshall, Cameron John

(57) **Abstract**

A purification process for the capsular polysaccharide of *S.agalactiae* in which the saccharide is initially treated with an aqueous mixture of an alcohol and a calcium salt, followed by precipitation with a cationic detergent. The process can be completed in less than three days and has a yield of around 60%. It avoids the need for DNase, RNase and/or protease treatment. The saccharides of the process have a very low protein contamination and a very low absorbance at 280nm.

## Description

All documents cited herein are incorporated by reference in their entirety.

### TECHNICAL FIELD

This invention is in the field of purifying bacterial capsular polysaccharides, particularly those of *Streptococcus agalactiae,* and particularly for use in the preparation of vaccines.

### BACKGROUND ART

The capsular saccharides of bacteria have been used for many years in vaccines against capsulated bacteria. As saccharides are T-independent antigens, however, they are poorly immunogenic. Conjugation to a carrier can convert T-independent antigens into T-dependent antigens, thereby enhancing memory responses and allowing protective immunity to develop. The most effective saccharide vaccines are therefore based on glycoconjugates, and the prototype conjugate vaccine was against *Haemophilus influenzae* type b ('Hib') [*e.g.* see chapter 14 of ref. 79].

Another bacterium for which conjugate vaccines have been described is *Streptococcus agalactiae,* also known as 'group B streptococcus', or simply as 'GBS'. Much of this work has been performed by Dennis Kasper and colleagues, and is described in documents such as references 1 to 9.

The starting point for saccharide-based vaccines is the saccharide itself, and this is generally purified from the target bacterium. The Kasper process for purification of the GBS saccharide is described in detail in references 2 and 10, and involves the following basic steps after bacterial culture: filtration to remove bacteria; ultrafiltration with a 10kDa cut-off membrane; addition of ethanol to 30% to precipitate contaminants; increase ethanol to 80% to precipitate the GBS saccharide overnight; collect and dry the precipitate; treat with RNase, then DNase, then pronase; treatment with sodium hydroxide; dialysis; DEAE-Sephacel ion-exchange chromatography; dialysis; lyophilisation; acetic anhydride treatment; conconavilin affinity chromatography to remove mannan; Ultragel size exclusion chromatography; and final lyophilisation.

This process is very slow, with the RNase, DNase, pronase and sodium hydroxide treatments each lasting overnight, and can take well over a week to complete. Furthermore, the yield is well below 50%. There is thus a need for further and improved processes for purifying GBS capsular polysaccharides, and particularly for quicker processes with higher yields.

### DISCLOSURE OF THE INVENTION

The invention is based on a purification process in which the saccharide is initially treated with an aqueous mixture of an alcohol (*e.g*. ethanol) and a metal cation (*e.g*. as a calcium salt), followed by precipitation with a cationic detergent (*e.g*. CTAB). The process can be completed in less than three days after release of saccharide from the bacteria and has a yield of around 60%.

The invention provides a process for purifying a *Streptococcus agalactiae* capsular polysaccharide, comprising the steps of: (a) treating a suspension comprising streptococcal proteins, nucleic acids and capsular polysaccharide with an aqueous metal cation and an alcohol in order to precipitate nucleic acids and proteins; (b) separating the precipitated material from the aqueous material; and (c) treating the aqueous material with a cationic detergent in order to precipitate the capsular polysaccharide. The precipitated polysaccharide can then be separated and re-solubilised for subsequent vaccine preparation. Other processing steps may be included in the process, such as ultrafiltration to remove low molecular weight contaminants (such as fragments of group-specific carbohydrate), further precipitation and re-solubilisation, and/or drying steps.

The invention also provides a process for purifying a *Streptococcus agalactiae* capsular polysaccharide, comprising the step of precipitating the saccharide using a cationic detergent. Similarly, the invention provides, in a process for purifying the *Streptococcus agalactiae* capsular polysaccharide, the improvement consisting of the use of a cationic detergent to precipitate the saccharide. Precipitation with a cationic detergent simplifies separation of the capsular saccharide from other saccharides that are present *e.g*. the group-specific saccharide.

The invention also provides a process for purifying a *Streptococcus agalactiae* capsular polysaccharide, comprising the step of removing contaminating nucleic acids and/or proteins by the use of precipitation. Similarly, the invention provides, in a process for purifying the *Streptococcus agalactiae* capsular polysaccharide, the improvement consisting of the use of precipitation to remove contaminating nucleic acids. Precipitation avoids the need for DNase or RNase enzymatic treatments.

The invention also provides a process for purifying the *Streptococcus agalactiae* capsular polysaccharide, wherein the process does not involve a step of DNase treatment. Similarly, the invention provides a process for purifying the *Streptococcus agalactiae* capsular polysaccharide, wherein the process does not involve a step of RNase treatment. Similarly, the invention provides a process for purifying the *Streptococcus agalactiae* capsular polysaccharide, wherein the process does not involve a step of protease treatment. Preferably, the process of the invention does not involve the use of two or three of DNase, RNase and/or protease *e.g*. it uses none of the three.

The invention also provides a process for purifying capsular saccharide from *Streptococcus agalactiae* bacteria, wherein the yield of the process (starting with the bacteria and ending with the capsular polysaccharide) is at least 40% *(e.g.* >50%, >60%, >70%, >80%, >90%). Practical limitations mean that the yield might not exceed 90% (*e.g*. might be <90%, <80%, <70%, *etc*.).

The invention also provides a process for purifying capsular saccharide from *Streptococcus agalactiae* bacteria, wherein the process provides a composition comprising the saccharide in which purity of the saccharide is at least 89% (*e.g.* ≥90%, ≥92%, ≥94%, ≥96%, ≥98%, *etc.)* relative to the total weight of saccharide, protein and nucleic acid in the composition.

The invention also provides a process for purifying capsular saccharide from *Streptococcus agalactiae* bacteria, wherein (a) the yield of the process is at least 40% (as described above) and (b) the purity of the saccharide is at least 89% (as described above).

The invention also provides a process for releasing the capsular polysaccharide from *Streptococcus agalactiae* bacteria, comprising the step of treating the bacteria with a type II phosphodiesterase. These enzymes can cleave the same phosphates as sodium hydroxide, but offer the advantage of not having sodium hydroxide's de-acetylating reactivity.

The invention also provides a composition comprising a *Streptococcus agalactiae* capsular polysaccharide, wherein UV absorbance at 280nm is less than 0.20. An absorbance of <0.15 or even <0.10 is preferred. The processes of the invention have been found to give compositions with very little protein contamination, leading to very little absorbance at 280nm. This is a particular advantage over the methods of the prior art, which give material that shows an absorbance peak at ∼280nm.

The invention also provides a composition comprising a *Streptococcus agalactiae* capsular polysaccharide, wherein the ratio of UV absorbance at 280nm to the UV absorbance at 260nm is greater than 0.85. A ratio of >0.90, >0.95 or even >1.0 is preferred. The ratio will typically be less than 1.2. A ratio of 1.0±0.1 is preferred.

The invention also provides a composition comprising a *Streptococcus agalactiae* capsular polysaccharide, wherein the UV absorbance spectrum of the composition between 220nm and 300nm does exhibit either a shoulder or peak at around 270nm. The invention also provides a composition comprising a *Streptococcus agalactiae* serotype Ia or serotype III capsular polysaccharide, wherein the UV absorbance spectrum between 250nm and 275nm does not increase. The invention also provides a composition comprising a *Streptococcus agalactiae* capsular polysaccharide, wherein the UV spectrum of the composition between 250nm and 275nm has neither a maximum point nor a point of inflexion.

The invention also provides a composition comprising *Streptococcus agalactiae* capsular saccharide, wherein the purity of the saccharide is at least 89% (*e.g.* ≥90%, ≥92%, ≥94%, ≥96%, ≥98%, *etc*.) relative to the total weight of saccharide, protein and nucleic acid in the composition.

The invention also provides a composition comprising *Streptococcus agalactiae* serotype Ia capsular saccharide, wherein the saccharide has monosaccharide subunits, and wherein no more than 93% *(e.g.* ≤92%, ≤90%, ≤85%, ≤80%, ≤75%, ≤70%, ≤65%, ≤60%, ≤55%, ≤50%, ≤45%, ≤40%, *etc*.) of the monosaccharide subunits have N-acetyl groups.

The invention also provides a composition comprising *Streptococcus agalactiae* serotype Ib capsular saccharide, wherein the saccharide has monosaccharide subunits, and wherein at least 78% *(e.g.* ≥80%, ≥85%, ≥90%, ≥92%, ≥94%, ≥96%, ≥98%, *etc.*) of the monosaccharide subunits have N-acetyl groups.

The invention also provides a composition comprising *Streptococcus agalactiae* serotype III capsular saccharide, wherein the saccharide has monosaccharide subunits, and wherein no more than 76% (*e.g.* ≤74%, ≤72%, ≤70%, ≤65%, ≤60%, ≤55%, ≤50%, ≤45%, ≤40%, *etc*.) of the monosaccharide subunits have N-acetyl groups.

The invention also provides a composition comprising *Streptococcus agalactiae* serotype Ia capsular saccharide, wherein the saccharide has a molecular weight of at least 100kDa.

The invention also provides a composition comprising *Streptococcus agalactiae* serotype Ib capsular saccharide, wherein the saccharide has a molecular weight of at least 40kDa.

The invention also provides a composition comprising *Streptococcus agalactiae* serotype III capsular saccharide, wherein the saccharide has a molecular weight of at least 40kDa.

### The capsular saccharide

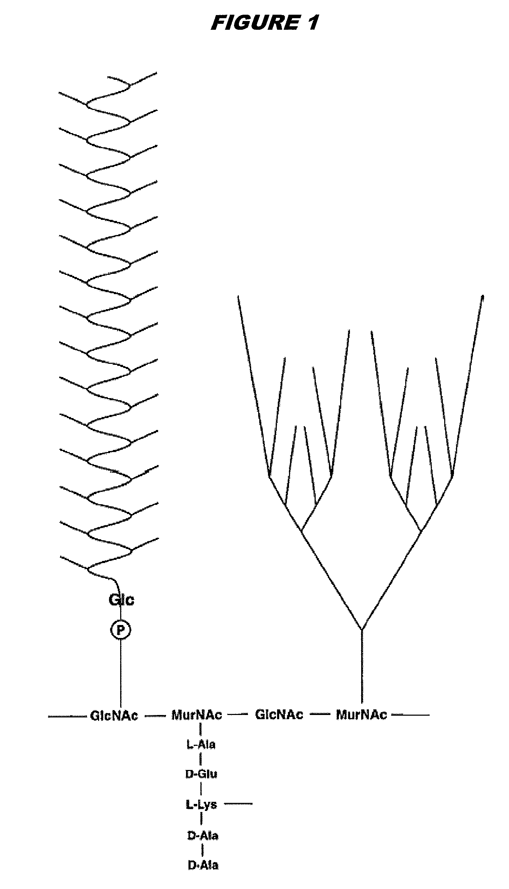

The *S.agalactiae* capsular polysaccharide is covalently linked to GlcNAc residues in the bacterium's peptidoglycan backbone, and is distinct from the group B antigen, which is separate saccharide that is attached to MurNAc residues on the same peptidoglycan backbone (Figure 1 [12]). The capsular polysaccharides of different serotypes are chemically related, but are antigenically very different. All GBS capsular polysaccharides share the following trisaccharide core:

β-D-Glc*p*NAc(1→3)β-D-Gal*p*(1→4)β-D-Glc*p*

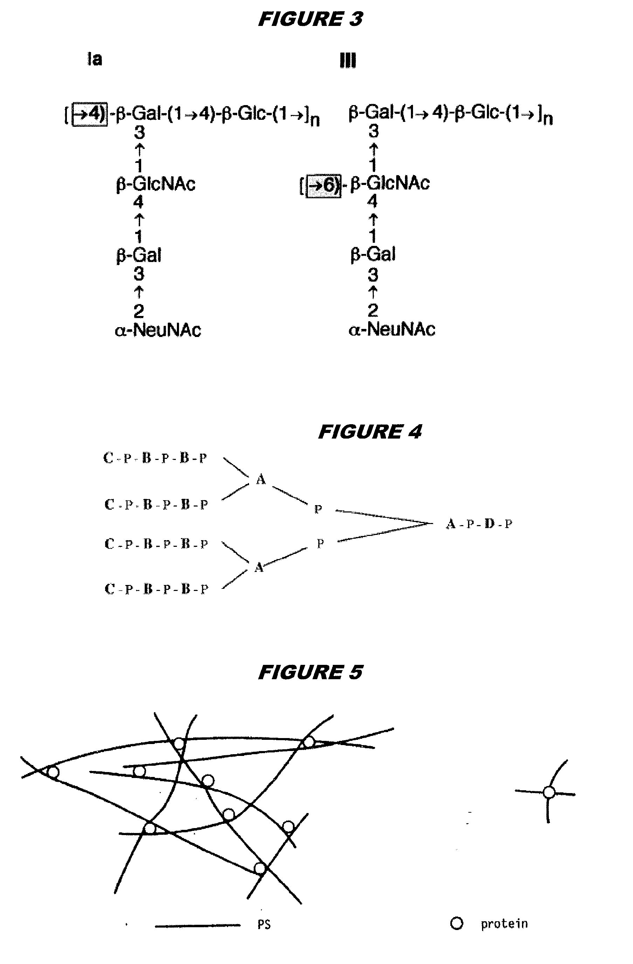

The various GBS serotypes differ by the way in which this core is modified. The difference between serotypes Ia and III, for instance, arises from the use of either the GlcNAc (Ia) or the Gal (III) in this core for linking consecutive trisaccharide cores (Figure 3). Serotypes Ia and Ib both have a [α-D-Neu*p*NAc(2→3)β-D-Gal*p*-(1→] disaccharide linked to the GlcNAc in the core, but the linkage is either 1→4 (Ia) or 1→3 (Ib).

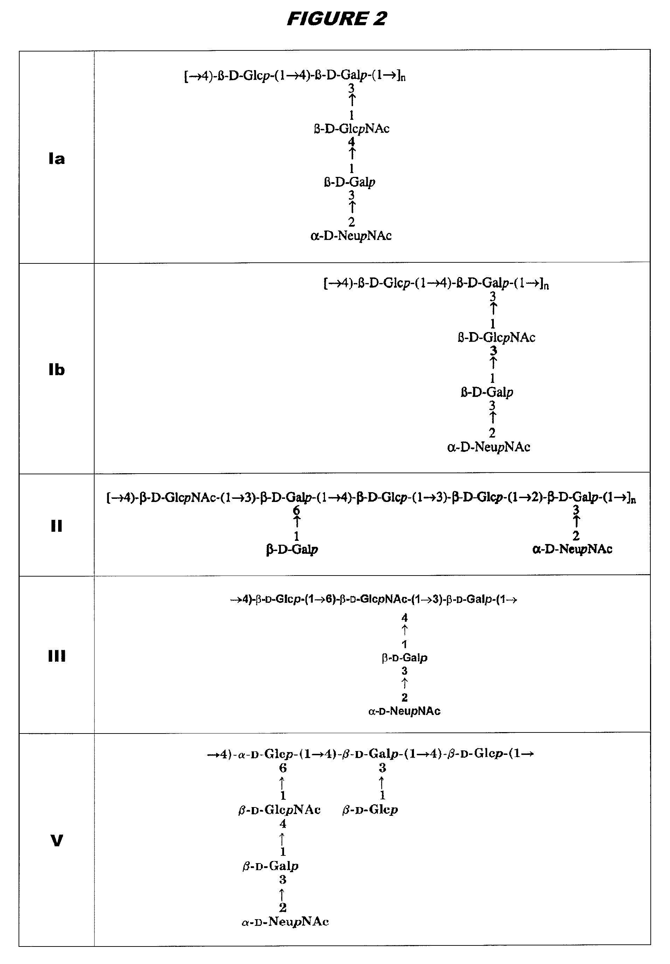

GBS-related disease arises primarily from serotypes Ia, Ib, II, III, IV, V, VI, VII, and VIII, with over 90% being caused by five serotypes: Ia, Ib, II, III & V. The invention preferably uses a saccharide from one of these five serotypes. As shown in Figure 2, the capsular saccharides of each of these five serotypes include: (a) a terminal *N*-acetyl-neuraminic acid (NeuNAc) residue (commonly referred to as sialic acid), which in all cases is linked 2→3 to a galactose residue; and (b) a N-acetylglucosamine residue (GlcNAc) within the trisaccharide core.

All five saccharides include galactose residues within the trisaccharide core, but serotypes Ia, Ib, II & III also contain additional galactose residues in each repeating unit, with the serotype II saccharide containing three galactose residues per repeating unit. Saccharides purified according to the invention will generally be in their native form, but they may have been modified. For example, the saccharide may be shorter than the native capsular saccharide, or may be chemically modified.

Thus the saccharide used according to the invention may be a substantially full-length capsular polysaccharide, as found in nature, or it may be shorter than the natural length. Full-length polysaccharides may be depolymerised to give shorter fragments for use with the invention *e.g*. by hydrolysis in mild acid, by heating, by sizing chromatography, *etc.* Chain length has been reported to affect immunogenicity of GBS saccharides in rabbits [4].

Depolymerisation of the serotype III capsular saccharide by endo-β-galactosidase has been reported [refs. 1 & 4-6]. Ozonolysis of capsular polysaccharides from GBS serotypes II, III and VIII has also been used for depolymerisation [13]. It is preferred to use saccharides with MW>30kDa, and substantially full-length capsular polysaccharides can be used. For serotype Ia, it is preferred to use polysaccharides with a MW up to ∼145kDa. For serotype Ib, it is preferred to use polysaccharides with a MW up to ∼50kDa. For serotype III, it is preferred to use polysaccharides with a MW up to ∼50kDa. These molecular masses can be measured by gel filtration relative to dextran standards, such as those available from Polymer Standard Service [14].

The saccharide may be chemically modified relative to the capsular saccharide as found in nature. For example, the saccharide may be de-O-acetylated (partially or fully), de-N-acetylated (partially or fully), N-propionated (partially or fully), *etc.* Depending on the particular saccharide, de-acetylation may or may not affect immunogenicity *e.g*. the NeisVac-C™ vaccine uses a de-O-acetylated saccharide, whereas Menjugate™ is acetylated, but both vaccines are effective. The relevance of O-acetylation on GBS saccharides in various serotypes is discussed in reference 15, and it is preferred to retain O-acetylation of sialic acid residues at positions 7, 8 and/or 9 before during and after purification *e.g*. by using formaldehyde for extraction of the saccharide and/or bacterial inactivation, by protection/de-protection, by re-acetylation, *etc.* The effect of de-acetylation *etc.* can be assessed by routine assays.

### Starting material

The process of the invention starts with the capsular saccharide in aqueous form, typically as a suspension comprising streptococcal proteins, nucleic acids and capsular polysaccharide.

A small amount of capsular polysaccharide is released into the culture medium during bacterial growth, and so the starting material for alcoholic precipitation of contaminating proteins and/or nucleic acids may thus be the supernatant from a centrifuged bacterial culture. More typically, however, the starting material will be prepared by treating the capsulated bacteria themselves (or material containing the bacterial peptidoglycan), such that the capsular saccharide is released. Reference 10 characterises saccharides prepared from both of these two sources.

Capsular polysaccharide can be released from bacteria by various methods, including chemical, physical or enzymatic treatment. Thus an aqueous preparation of polysaccharide can be treated prior to the initial protein/nucleic acid precipitation reaction.

A typical chemical treatment is base extraction [16] (*e.g*. using sodium hydroxide), which can cleave the phosphodiester linkage between the capsular saccharide and the peptidoglycan backbone. Base extraction is advantageous because it inactivates the bacteria at the same time as releasing the capsular polysaccharide. Moreover, base treatment releases the polysaccharide intact and causes extensive cleavage of the group B antigen due to its multiple phosphodiester linkages (Figure 4 [12]), facilitating later separation of the capsular and group-specific saccharide antigens. Sodium hydroxide treatment is therefore a preferred method for releasing the capsular polysaccharide. As hydroxide treatment de-N-acetylates the capsular saccharide, however, later re-N-acetylation may be necessary.

A typical enzymatic treatment involves the use of both mutanolysin and β-N-acetylglucosaminidase [12]. These act on the GBS peptidoglycan to release the capsular saccharide for use with the invention, but also lead to release of the group-specific carbohydrate antigen. An alternative enzymatic treatment involves treatment with a type II phosphodiesterase (PDE2). PDE2 enzymes can cleave the same phosphates as sodium hydroxide (see above) and can release the capsular saccharide without cleaving the group-specific carbohydrate antigen and without de-N-acetylating the capsular saccharide, thereby simplifying downstream steps. PDE2 enzymes are therefore a preferred option for preparing GBS capsular saccharides for use in the processes of the invention.

A preferred starting material for the process of the invention is thus de-N-acetylated capsular polysaccharide, which can be obtained by base extraction as described in reference 16. Another preferred starting material is thus the product of PDE2 treatment of GBS. Such materials can be subjected to concentration (*e.g*. ultrafiltration) prior to precipitation by the processes of the invention.

### Alcoholic precipitation and cation exchange

The GBS capsular saccharide obtained after culture will generally be impure and will be contaminated with bacterial nucleic acids and proteins. The prior art removes these contaminants by sequential overnight treatments with RNase, DNase and protease. In contrast, the overall purification process of the invention can be performed in a shorter time than these three individual steps from the prior art process. Rather than remove these contaminants enzymatically, the process of the invention utilises alcoholic precipitation. If necessary (*e.g*. after base extraction), materials will usually be neutralised prior to the precipitation.

The alcohol used to precipitate contaminating nucleic acids and/or proteins is preferably a lower alcohol, such as methanol, ethanol, propan-1-ol, propan-2-ol, butan-1-ol, butan-2-ol, 2-methyl-propan-1-ol, 2-methyl-propan-2-ol, diols, *etc.* The selection of an appropriate alcohol can be tested empirically, without undue burden, but alcohols such as ethanol and isopropanol (propan-2-ol) are preferred, rather than alcohols such as phenol.

The alcohol is preferably added to the polysaccharide suspension to give a final alcohol concentration of between 10% and 50% (*e.g*. around 30%). The most useful concentrations are those which achieve adequate precipitation of contaminants without also precipitating the polysaccharide. The optimum final alcohol concentration may depend on the GBS serotype from which the polysaccharide is obtained, and can be determined by routine experiments without undue burden. Precipitation of polysaccharides as ethanol concentrations >50% has been observed.

The alcohol may be added in pure form or may be added in a form diluted with a miscible solvent (*e.g*. water). Preferred solvent mixtures are ethanol:water mixtures, with a preferred ratio of between around 70:30 and around 95:5 (*e.g*. 75:25, 80:20, 85:15, 90:10).

The saccharide is also treated with an aqueous metal cation. Monovalent and divalent metal cations are preferred, and divalent cations are particularly preferred, such as Mg⁺⁺, Mn⁺⁺, Ca⁺⁺, *etc,* as they are more efficient at complex formation. Calcium ions are particularly useful, and so the alcohol mixture preferably includes soluble calcium ions. These may be added to a saccharide/ alcohol mixture in the form of calcium salts, either added as a solid or in an aqueous form. The calcium ions are preferably provided by the use of calcium chloride.

The calcium ions are preferably present at a final concentration of between 10 and 500 mM *e.g*. about 0.1 M. The optimum final Ca⁺⁺ concentration may depend on the GBS serotype from which the polysaccharide is obtained, and can be determined by routine experiments without undue burden.

The alcohol and the cation play different roles (the alcohol is used to precipitate contaminants, whereas the cation stabilises and complexes the saccharide in soluble form) but produce a combined effect. Although the aim is to prepare a mixture of the saccharide, the alcohol and the cation, these three components need not be mixed together simultaneously. Thus the alcohol and cation can be used sequentially or simultaneously. Sequential treatment is preferred, and a particularly preferred process involves addition of the cation to the saccharide followed by addition of the alcohol to the cation/saccharide mixture, although the alcohol can be used before the cation if desired.

After alcoholic precipitation of contaminating proteins and/or nucleic acids, the GBS capsular polysaccharide is left in solution. The precipitated material can be separated from the polysaccharide by any suitable means, such as by centrifugation. The supernatant can be subjected to microfiltration, and in particular to dead-end filtration (perpendicular filtration) in order to remove particles that may clog filters in later steps (*e.g*. precipitated particles with a diameter greater than 0.22µm). As an alternative to dead-end filtration, tangential microfiltration can be used.

### Diafiltration

The process of the invention may involve a step of diafiltration after the precipitation of proteins and/or nucleic acids, and before the detergent-mediated precipitation. This diafiltration step is particularly advantageous if base extraction or phosphodiesterase was used for release of the capsular saccharide, as the group-specific saccharide will also have been hydrolysed, to give fragments much smaller than the intact capsular saccharide. These small fragments can be removed by the diafiltration step.

Tangential flow diafiltration is typical. The filtration membrane should thus be one that allows passage of hydrolysis products of the group-specific antigen while retaining the capsular polysaccharide. A cut-off in the range 10kDa-30kDa is typical. Smaller cut-off sizes can be used, as the hydrolysis fragments of the group-specific antigen are generally around 1kDa (5-mer, 8-mer and 11-mer saccharides), but the higher cut-off advantageously allows removal of other contaminants without leading to loss of the capsular saccharide.

At least 5 cycles of tangential flow diafiltration are usually performed *e.g*. 6, 7, 8, 9, 10, 11 or more.

### Cationic detergent treatment

Many techniques for precipitating soluble polysaccharides are known in the art. According to the invention, the GBS saccharide is precipitated using one or more cationic detergents. The inventors have found that treating a mixture of the GBS capsular saccharide and group-specific saccharide with a cationic detergent leads to preferential precipitation of the capsular saccharide, thereby advantageously and conveniently minimising contamination by the group-specific saccharide.

The cationic detergent preferably has the following general formula: 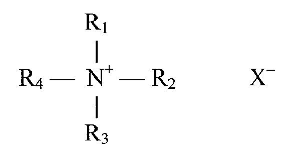 wherein:
R₁, R₂ and R₃ are the same or different and each signifies alkyl or aryl; or R₁ and R₂ together with the nitrogen atom to which they are attached form a 5- or 6-membered saturated heterocyclic ring, and R₃ signifies alkyl or aryl; or R₁, R₂ and R₃ together with the nitrogen atom to which they are attached form a 5- or 6-membered heterocyclic ring, unsaturated at the nitrogen atom,
R₄ signifies alkyl or aryl, and
X⁻ signifies an anion.

Particularly preferred detergents for use in the process of the invention are tetrabutylammonium and cetyltrimethylammonium salts (*e.g*. the bromide salts). Cetyltrimethylammonium bromide ('CTAB') is particularly preferred [17]. CTAB is also known as hexadecyltrimethylammonium bromide, cetrimonium bromide, Cetavlon and Centimide. Other detergents include hexadimethrine bromide and myristyltrimethylammonium salts.

The detergent-mediated precipitation step is preferably selective for the capsular polysaccharide. Advantageously, the invention uses a detergent such as CTAB that interacts with sialic acid residues in the saccharide *e.g*. via carboxyl groups in the sialic acid. The detergent will thus preferentially precipitate the sialic acid-containing capsular saccharides, and particularly longer saccharides within a mixed population, thus minimising contamination by saccharides whose antigenically-important sialic acids may have been damaged in earlier treatment steps. For GBS, longer saccharides tend to be more immunogenic than shorter ones [18], and so the invention offers advantages over prior art methods which led to shorter depolymerised saccharides.

After precipitation, the capsular saccharides can be separated by centrifugation. The inventors have found that centrifugation after CTAB-mediated precipitation does not give a simple pellet, but instead gives a pellet that appears to have two phases. The bottom of these is typically chosen for further use with the invention.

### Re-solubilisation

After precipitation, the polysaccharide (typically in the form of a complex with the cationic detergent) can be re-solubilised, either in aqueous medium or in alcoholic medium. For aqueous re-solubilisation, the CTA⁻ cation in the precipitate will generally be replaced by a metal cation; for alcoholic re-solubilisation, the CTA⁻ cation will generally be retained. The choice of aqueous or alcoholic re-solubilisation may depend on the GBS serotype from which the polysaccharide is obtained, and on any contaminants still present at this stage. For example, pigments are sometimes present in the precipitated pellet, and these can effectively be removed by alcoholic re-solubilisation followed by carbon filtration.

A typical aqueous medium for re-solubilisation will include a metal cation. Monovalent and divalent metal cations are preferred, and divalent cations are particularly preferred, such as Mg⁺⁺, Mn⁺⁺, Ca⁺⁺, *etc.* Calcium ions are particularly useful, and so re-solubilisation preferably uses Ca⁺⁺, provided provided by the use of calcium chloride. A Ca⁺⁺ concentration of between 10 and 500 mM (*e.g*. about 0.1M) is preferred. The optimum final Ca⁺⁺ concentration may depend on the GBS serotype from which the polysaccharide is obtained, and can be determined by routine experiments without undue burden.

A typical alcoholic medium for re-solubilisation is based on ethanol. The same alcohols used for precipitation of nucleic acids and/or proteins can be used, but the concentration required for precipitation of the capsular saccharide will generally be higher *e.g*. the alcohol is preferably added to give a final alcohol concentration of between 70% and 95% (*e.g*. around 70%, 75%, 80%, 85%, 90% or 95%). The optimum final alcohol concentration may depend on the GBS serotype from which the polysaccharide is obtained. To achieve the high alcohol concentrations then it is preferred to add alcohol with a low water content *e.g*. 96% ethanol.

Re-solubilisation will typically occur at room temperature. Acidic conditions are preferably avoided, and re-solubilisation will typically take place at about pH 7.

The re-solubilised material is highly purified relative to the pre-precipitation suspension.

### Further treatment of the capsular polysaccharide

After re-solubilisation, the polysaccharide may be further treated to remove contaminants. This is particularly important in situations where even minor contamination is not acceptable (*e.g*. for human vaccine production).

One preferred further step is a further precipitation. Where an aqueous re-solubilisation was performed then this precipitation will typically use an alcohol, as described in the preceding section; conversely, where an alcoholic re-solubilisation was performed then this precipitation will typically use an aqueous cation solution, as described in the preceding section. The precipitated saccharide can then be separated from any remaining aqueous contaminants *e.g*. by centrifugation. The precipitated material is stable and can be stored for future use.

The precipitated material may be subjected to vacuum drying. This treatment will typically be used not to stabilise the saccharide for storage, but to dry the saccharide and remove any residual alcohol.

Further rounds of precipitation and filtration can also be performed. Depth filtration can also be used *e.g*. as an alternative to centrifugation. Depth filtration will typically be used after solubilisation in alcohol.

The polysaccharide may be depolymerised to form oligosaccharides. Oligosaccharides may be preferred to polysaccharides for use in vaccines, and chain length has been reported to affect immunogenicity of GBS saccharides in rabbits [4]. Depolymerisation from polysaccharide to oligosaccharide can occur before or after the detergent-mediated precipitation. If depolymerisation is performed, the products will generally be sized in order to remove short-length oligosaccharides. This can be achieved in various ways, such as ultrafiltration followed by ion-exchange chromatography. Where the composition of the invention includes a depolymerised saccharide, it is preferred that depolymerisation precedes any conjugation.

If sialic acid residues in the GBS capsular saccharides have been de-N-acetylated then the processes of the invention may include a step of re-N-acetylation. Controlled re-N-acetylation can conveniently be performed using a reagent such as acetic anhydride (CH₃CO)₂O *e.g*. in 5% ammonium bicarbonate [10].

These additional steps can generally be performed at room temperature.

### Conjugation

The final purified capsular polysaccharide of the invention can be used as an antigen without further modification *e.g*. for use in *in vitro* diagnostic assays, for use in immunisation, *etc.*

For immunisation purposes, however, it is preferred to conjugate the saccharide to a carrier molecule, such as a protein. In general, covalent conjugation of saccharides to carriers enhances the immunogenicity of saccharides as it converts them from T-independent antigens to T-dependent antigens, thus allowing priming for immunological memory. Conjugation is particularly useful for paediatric vaccines [*e.g*. ref. 19] and is a well known technique [*e.g*. reviewed in refs. 20 to 28]. Thus the processes of the invention may include the further step of conjugating the purified saccharide to a carrier molecule.

Conjugation of GBS saccharides has been widely reported *e.g*. see references 1 to 9. The typical prior art process for GBS saccharide conjugation typically involves reductive amination of a purified saccharide to a carrier protein such as tetanus toxoid (TT) or CRM197 [2]. The reductive amination involves an amine group on the side chain of an amino acid in the carrier and an aldehyde group in the saccharide. As GBS capsular saccharides do not include an aldehyde group in their natural form then this is generated before conjugation by periodate oxidation of a portion (*e.g*. between 5 and 15%, preferably about 10%) of the saccharide's sialic acid residues [2,29]. Conjugate vaccines prepared in this manner have been shown to be safe and immunogenic in humans for each of GBS serotypes Ia, Ib, II, III, and V [30]. An alternative conjugation process involves the use of -NH₂ groups in the saccharide (either from de-N-acetylation, or after introduction of amines) in conjunction with bifunctional linkers, as described in ref. 31.

Preferred carrier proteins are bacterial toxins or toxoids, such as diphtheria toxoid or tetanus toxoid. The CRM197 mutant of diphtheria toxin [32-34] is a particularly preferred carrier for, as is a diphtheria toxoid. Other suitable carrier proteins include the *N.meningitidis* outer membrane protein [35], synthetic peptides [36,37], heat shock proteins [38,39], pertussis proteins [40,41], cytokines [42], lymphokines [42], hormones [42], growth factors [42], human serum albumin (preferably recombinant), artificial proteins comprising multiple human CD4⁺ T cell epitopes from various pathogen-derived antigens [43] such as N19 [44], protein D from *H.influenzae* [45,46], pneumococcal surface protein PspA [47], pneumolysin [48], iron-uptake proteins [49], toxin A or B from *C.difficile* [50], a GBS protein [109], *etc.*

Attachment to the carrier is preferably via a -NH₂ group *e.g*. in the side chain of a lysine residue in a carrier protein, or of an arginine residue. Attachment may also be via a -SH group *e.g*. in the side chain of a cysteine residue.

It is possible to use more than one carrier protein *e.g*. to reduce the risk of carrier suppression. Thus different carrier proteins can be used for different GBS serotypes *e.g*. serotype Ia saccharides might be conjugated to CRM197 while serotype Ib saccharides might be conjugated to tetanus toxoid. It is also possible to use more than one carrier protein for a particular saccharide antigen *e.g*. serotype III saccharides might be in two groups, with some conjugated to CRM197 and others conjugated to tetanus toxoid. In general, however, it is preferred to use the same carrier protein for all saccharides.

A single carrier protein might carry more than one saccharide antigen [51,52]. For example, a single carrier protein might have conjugated to it saccharides from serotypes Ia and Ib. To achieve this goal, different saccharides can be mixed prior to the conjugation reaction. In general, however, it is preferred to have separate conjugates for each serogroup, with the different saccharides being mixed after conjugation. The separate conjugates may be based on the same carrier.

Conjugates with a saccharide:protein ratio (w/w) of between 1:5 (*i.e.* excess protein) and 5:1 (*i.e.* excess saccharide) are preferred. Ratios between 1:2 and 5:1 are preferred, as are ratios between 1:1.25 and 1:2.5.

Conjugates may be used in conjunction with free carrier [53]. When a given carrier protein is present in both free and conjugated form in a composition of the invention, the unconjugated form is preferably no more than 5% of the total amount of the carrier protein in the composition as a whole, and more preferably present at less than 2% by weight.

After conjugation, free and conjugated saccharides can be separated. There are many suitable methods, including hydrophobic chromatography, tangential ultrafiltration, diafiltration *etc.* [see also refs. 54 & 55, *etc.*].

In general, two types of conjugate can be made, as shown in Figure 5: (a) a conjugate where an individual saccharide is attached to a single carrier *e.g*. through its reducing terminus; and (b) a conjugate where an individual saccharide is attached to multiple carriers *e.g*. because several monosaccharide subunits are reactive. In both situations a single carrier protein can link to multiple saccharide molecules because it can have multiple exposed lysine side chains. Conjugates of type (b) are more typical in the present invention, because the modified sialic acid or galactose residues of the invention occur at multiple sites along a single saccharide [56]. In preferred conjugates, therefore, a single saccharide molecule is coupled on average to more than one carrier molecule.

### Combinations of conjugates and other antigens

Saccharides prepared by the methods of the invention (in particular after conjugation as described above) can be mixed *e.g*. with each other and/or with other antigens. Thus the processes of the invention may include the further step of mixing the saccharide with one or more further antigens. Where multiple different GBS conjugates are mixed then these may include different types of conjugate from the same GBS serotype and/or conjugates from different GBS serotypes. For example, the conjugates may be from two or three of serotypes Ia, Ib and III. The composition will be produced by preparing separate conjugates (*e.g*. a different conjugate for each serotype) and then combining the conjugates.

The further antigen(s) may comprise GBS amino acid sequences, as set out below.

The further antigen(s) may comprise antigens from non-GBS pathogens. Thus the compositions of the invention may further comprise one or more non-GBS antigens, including additional bacterial, viral or parasitic antigens. These may be selected from the following:
- a protein antigen from *N.meningitidis* serogroup B, such as those in refs. 57 to 63, with protein '287' (see below) and derivatives (*e.g*. 'ΔG287') being particularly preferred.
- an outer-membrane vesicle (OMV) preparation from *N.meningitidis* serogroup B, such as those disclosed in refs. 64, 65, 66, 67 *etc.*
- a saccharide antigen from *N.meningitidis* serogroup A, C, W135 and/or Y, such as the oligosaccharide disclosed in ref. 68 from serogroup C or the oligosaccharides of ref. 69.
- a saccharide antigen from *Streptococcus pneumoniae* [*e.g.* refs. 70-72; chapters 22 & 23 of ref. 79].
- an antigen from hepatitis A virus, such as inactivated virus [*e.g*. 73, 74; chapter 15 of ref. 79].
- an antigen from hepatitis B virus, such as the surface and/or core antigens [*e.g*. 74,75; chpater 16 ofref. 79].
- an antigen from hepatitis C virus [*e.g.* 76].
- an antigen from *Bordetella pertussis,* such as pertussis holotoxin (PT) and filamentous haemagglutinin (FHA) from *B.pertussis,* optionally also in combination with pertactin and/or agglutinogens 2 and 3 [*e.g*. refs. 77 & 78; chapter 21 of ref. 79].
- a diphtheria antigen, such as a diphtheria toxoid [*e.g*. chapter 13 of ref. 79].
- a tetanus antigen, such as a tetanus toxoid [*e.g*. chapter 27 of ref. 79].
- a saccharide antigen from *Haemophilus influenzae* B [*e.g.* chapter 14 of ref. 79]
- an antigen from *N.gonorrhoeae* [*e.g.* 57, 58, 59].
- an antigen from *Chlamydia pneumoniae* [*e.g.* 80, 81, 82, 83, 84, 85, 86].
- an antigen from *Chlamydia trachomatis* [*e.g.* 87]*.*
- an antigen from *Porphyromonas gingivalis* [*e.g.* 88].
- polio antigen(s) [*e.g*. 89, 90; chapter 24 of ref. 79] such as IPV.
- rabies antigen(s) [*e.g*. 91] such as lyophilised inactivated virus [*e.g*.92, RabAvert™].
- measles, mumps and/or rubella antigens [*e.g*. chapters 19, 20 and 26 of ref. 79].
- influenza antigen(s) [*e.g*. chapters 17 & 18 of ref. 79], such as the haemagglutinin and/or neuraminidase surface proteins.
- an antigen from *Moraxella catarrhalis* [*e.g.* 93]*.*
- an antigen from *Streptococcus pyogenes* (group A streptococcus) [*e.g*. 94, 95, 96].

Where a saccharide or carbohydrate antigen is used, it is preferably conjugated to a carrier in order to enhance immunogenicity. Conjugation of *H.influenzae* B, meningococcal and pneumococcal saccharide antigens is well known.

Toxic protein antigens may be detoxified where necessary (*e.g*. detoxification of pertussis toxin by chemical and/or genetic means [78]).

Where a diphtheria antigen is included in the composition it is preferred also to include tetanus antigen and pertussis antigens. Similarly, where a tetanus antigen is included it is preferred also to include diphtheria and pertussis antigens. Similarly, where a pertussis antigen is included it is preferred also to include diphtheria and tetanus antigens.

Antigens may be adsorbed to an aluminium salt.

Antigens in the composition will typically be present at a concentration of at least 1 µg/ml each. In general, the concentration of any given antigen will be sufficient to elicit an immune response against that antigen.

As an alternative to using proteins antigens in the composition of the invention, nucleic acid encoding the antigen may be used [*e.g*. refs. 97 to 105]. Protein components of the compositions of the invention may thus be replaced by nucleic acid (preferably DNA *e.g*. in the form of a plasmid) that encodes the protein.

In practical terms, there may be an upper limit to the number of antigens included in compositions of the invention. The number of antigens (including GBS antigens) in a composition of the invention may be less than 20, less than 19, less than 18, less than 17, less than 16, less than 15, less than 14, less than 13, less than 12, less than 11, less than 10, less than 9, less than 8, less than 7, less than 6, less than 5, less than 4, or less than 3. The number of GBS antigens in a composition of the invention may be less than 6, less than 5, or less than 4.

### Pharmaceutical compositions and methods

The invention provides processes for preparing pharmaceutical compositions, comprising the steps of mixing (a) a saccharide of the invention (optionally in the form of a conjugate) with (b) a pharmaceutically acceptable carrier. Typical 'pharmaceutically acceptable carriers' include any carrier that does not itself induce the production of antibodies harmful to the individual receiving the composition. Suitable carriers are typically large, slowly metabolised macromolecules such as proteins, polysaccharides, polylactic acids, polyglycolic acids, polymeric amino acids, amino acid copolymers, lactose, and lipid aggregates (such as oil droplets or liposomes). Such carriers are well known to those of ordinary skill in the art. The vaccines may also contain diluents, such as water, saline, glycerol, *etc.* Additionally, auxiliary substances, such as wetting or emulsifying agents, pH buffering substances, and the like, may be present. Sterile pyrogen-free, phosphate-buffered physiologic saline is a typical carrier. A thorough discussion of pharmaceutically acceptable excipients is available in reference 106.

The pharmaceutical compositions may be packaged into vials or into syringes. The syringes may be supplied with or without needles. A syringe will include a single dose of the composition, whereas a vial may include a single dose or multiple doses.

Aqueous compositions of saccharides of the invention are suitable for reconstituting other vaccines from a lyophilised form. Where a composition of the invention is to be used for such extemporaneous reconstitution, the invention provides a process for reconstituting such a lyophilised vaccine, comprising the step of mixing the lyophilised material with an aqueous composition of the invention. The reconstituted material can be used for injection.

### GBS protein antigens

As mentioned above, GBS proteins can be included in compositions of the invention. These may be used as carrier proteins for conjugates of the invention, carrier proteins for other conjugates, or as unconjugated protein antigens.

GBS protein antigens for use with the invention include those disclosed in references 94 and 107-109. Five preferred GBS protein antigens for use with the invention are known as: GBS67; GBS80; GBS104; GBS276; and GBS322 [see ref. 94]. Further details of these five antigens are given below.

The full-length sequences for these five GBS proteins are SEQ ID NOs 1 to 5 herein. Compositions of the invention may thus include (a) a polypeptide comprising an amino acid sequence selected from SEQ ID NOs 1 to 5, and/or (b) a polypeptide comprising (i) an amino acid sequence that has sequence identity to one or more of SEQ ID NOs 1 to 5 and/or (ii) a fragment of SEQ ID NOs 1 to 5.

Depending on the particular SEQ ID NO, the degree of sequence identity in (i) is preferably greater than 50% (*e.g*. 60%, 70%, 75%, 80%, 85%, 90%, 91%, 92%, 93%, 94%, 95%, 96%, 97%, 98%, 99% or more). These polypeptides include homologs, orthologs, allelic variants and functional mutants. Typically, 50% identity or more between two polypeptide sequences is considered to be an indication of functional equivalence. Identity between polypeptides is preferably determined by the Smith-Waterman homology search algorithm as implemented in the MPSRCH program (Oxford Molecular), using an affine gap search with parameters *gap open penalty*=*12* and *gap extension penalty*=*1.*

Depending on the particular SEQ ID NO, the fragments of (ii) should comprise at least n consecutive amino acids from the sequences and, depending on the particular sequence, n is 7 or more *(e.g.* 8, 10, 12, 14, 16, 18, 20, 22, 24, 26, 28, 30, 35, 40, 45, 50, 60, 70, 80, 90, 100 or more). The fragment may comprise at least one T-cell or, preferably, a B-cell epitope of the sequence. T- and B-cell epitopes can be identified empirically (*e.g*. using PEPSCAN [110,111] or similar methods), or they can be predicted (*e.g*. using the Jameson-Wolf antigenic index [112], matrix-based approaches [113], TEPITOPE [114], neural networks [115], OptiMer & EpiMer [116, 117], ADEPT [118], Tsites [119], hydrophilicity [120], antigenic index [121] or the methods disclosed in reference 122 *etc.).* Other preferred fragments are SEQ ID NOs 1 to 5 without their N-terminal amino acid residue or without their N-terminal signal peptide. Removal of one or more domains, such as a leader or signal sequence region, a transmembrane region, a cytoplasmic region or a cell wall anchoring motif can be used. Preferred fragments are given below (SEQ ID NOs 6 to 19).

These polypeptide may, compared to SEQ ID NOs 1 to 5, include one or more *(e.g.* 1, 2, 3, 4, 5, 6, 7, 8, 9, 10, *etc.)* conservative amino acid replacements *i.e*. replacements of one amino acid with another which has a related side chain. Genetically-encoded amino acids are generally divided into four families: (1) acidic *i.e*. aspartate, glutamate; (2) basic *i.e*. lysine, arginine, histidine; (3) non-polar *i.e*. alanine, valine, leucine, isoleucine, proline, phenylalanine, methionine, tryptophan; and (4) uncharged polar *i.e*. glycine, asparagine, glutamine, cystine, serine, threonine, tyrosine. Phenylalanine, tryptophan, and tyrosine are sometimes classified jointly as aromatic amino acids. In general, substitution of single amino acids within these families does not have a major effect on the biological activity. The polypeptides may also include one or more (*e.g*. 1, 2, 3, 4, 5, 6, 7, 8, 9, 10, *etc.)* single amino acid deletions relative to SEQ ID NOs 1 to 5. The polypeptides may also include one or more *(e.g.* 1, 2, 3, 4, 5, 6, 7, 8, 9, 10, *etc.)* insertions (*e.g*. each of 1, 2, 3, 4 or 5 amino acids) relative to the SEQ ID NOs 1 to 5.

Polypeptides of the invention can be prepared in many ways *e.g*. by chemical synthesis (in whole or in part), by digesting longer polypeptides using proteases, by translation from RNA, by purification from cell culture *(e.g.* from recombinant expression), from the organism itself *(e.g.* after bacterial culture, or direct from patients), *etc.* A preferred method for production of peptides <40 amino acids long involves *in vitro* chemical synthesis [123,124]. Solid-phase peptide synthesis is particularly preferred, such as methods based on tBoc or Fmoc [125] chemistry. Enzymatic synthesis [126] may also be used in part or in full. As an alternative to chemical synthesis, biological synthesis may be used *e.g.* the polypeptides may be produced by translation. This may be carried out *in vitro* or *in vivo.* Biological methods are in general restricted to the production of polypeptides based on L-amino acids, but manipulation of translation machinery (*e.g*. of aminoacyl tRNA molecules) can be used to allow the introduction of D-amino acids (or of other non natural amino acids, such as iodotyrosine or methylphenylalanine, azidohomoalanine, etc.) [127]. Where D-amino acids are included, however, it is preferred to use chemical synthesis. Polypeptides of the invention may have covalent modifications at the C-terminus and/or N-terminus.

If these GBS proteins are included in compositions of the invention then they can take various forms (*e.g*. native, fusions, glycosylated, non-glycosylated, lipidated, non-lipidated, phosphorylated, non-phosphorylated, myristoylated, non-myristoylated, monomeric, multimeric, particulate, denatured, *etc*.). They are preferably used in purified or substantially purified form *i.e*. substantially free from other polypeptides (*e.g*. free from naturally-occurring polypeptides), particularly from other GBS or host cell polypeptides).

### GBS67

Nucleotide and amino acid sequence of GBS67 sequenced from serotype V strain 2603 V/R are set forth in ref. 94 as SEQ ID NOs 3745 & 3746. The amino acid sequence is SEQ ID NO:1 herein:

MRKYQKFSKILTLSLFCLSQIPLNTNVLGESTVPENGAKGKLVVKKTDDQNKPLSKATFVLKTTAHPESKIEKVTAELT
GEATFDNLIPGDYTLSEETAPEGYKKTNQTWQVKVESNGKTTIQNSGDKNSTIGQNQEELDKQYPPTGIYEDTKESYKL
EHVKGSVPNGKSEAKAVNPYSSEGEHIREIPEGTLSKRISEVGDLAHNKYKIELTVSGKTIVKPVDKQKPLDVVFVLDN
SNSMNNDGPNFQRHNKAKKAAEALGTAVKDILGANSDNRVALVTYGSDIFDGRSVDVVKGFKEDDKYYGLQTKFTIQTE
NYSHKQLTNNAEEIIKRIPTEAPKAKWGSTTNGLTPEQQKEYYLSKVGETFTMKAFMEADDILSQVNRNSQKIIVHVTD
GVPTRSYAINNFKLGASYESQFEQMKKNGYLNKSNFLLTDKPEDIKGNGESYFLFPLDSYQTQIISGNLQKLHYLDLNL
NYPKGTIYRNGPVKEHGTPTKLYINSLKQKNYDIFNFGIDISGFRQVYNEEYKKNQDGTFQKLKEEAFKLSDGEITELM
RSFSSKPEYYTPIVTSADTSNNEILSKIQQQFETILTKENSIVNGTIEDPMGDKINLQLGNGQTLQPSDYTLQGNDGSV
MKDGIATGGPNNDGGILKGVKLEYIGNKLYVRGLNLGEGQKVTLTYDVKLDDSFISNKFYDTNGRTTLNPKSEDPNTLR
DFPIPKIRDVREYPTITIKNEKKLGEIEFIKVDKDNNKLLLKGATFELQEFNEDYKLYLPIKNNNSKVVTGENGKISYK
DLKDGKYQLIEAVSPEDYQKITNKPILTFEVVKGSIKNIIAVNKQISEYHEEGDKHLITNTHIPPKGIIPMTGGKGILS
FILIGGAMMSIAGGIYIWKRYKKSSDMSIKKD

GBS67 contains a C-terminus transmembrane region which is indicated by the underlined region closest to the C-terminus of SEQ ID NO: 1 above. One or more amino acids from the transmembrane region may be removed, or the amino acid may be truncated before the transmembrane region. An example of such a GBS67 fragment is set forth below as SEQ ID NO: 18.

MRKYQKFSKILTLSLFCLSQIPLNTNVLGESTVPENGAKGKLVVKKTDDQNKPLSKATFVLKTTAHPESKIEKVTAELT
GEATFDNLIPGDYTLSEETAPEGYKKTNQTWQVKVESNGKTTIQNSGDKNSTIGQNQEELDKQYPPTGIYEDTKESYKL
EHVKGSVPNGKSEAKAVNPYSSEGEHIREIPEGTLSKRISEVGDLAHNKYKIELTVSGKTIVKPVDKQKPLDVVFVLDN
SNSMNNDGPNFQRHNKAKKAAEALGTAVKDILGANSDNRVALVTYGSDIFDGRSVDVVKGFKEDDKYYGLQTKFTIQTE
NYSHKQLTNNAEEIIKRIPTEAPKAKWGSTTNGLTPEQQKEYYLSKVGETFTMKAFMEADDILSQVNRNSQKIIVHVTD
GVPTRSYAINNFKLGASYESQFEQMKKNGYLNKSNFLLTDKPEDIKGNGESYFLFPLDSYQTQIISGNLQKLHYLDLNL
NYPKGTIYRNGPVKEHGTPTKLYINSLKQKNYDIFNFGIDISGFRQVYNEEYKKNQDGTFQKLKEEAFKLSDGEITELM
RSFSSKPEYYTPIVTSADTSNNEILSKIQQQFETILTKENSIVNGTIEDPMGDKINLQLGNGQTLQPSDYTLQGNDGSV
MKDGIATGGPNNDGGILKGVKLEYIGNKLYVRGLNLGEGQKVTLTYDVKLDDSFISNKFYDTNGRTTLNPKSEDPNTLR
DFPIPKIRDVREYPTITIKNEKKLGEIEFIKVDKDNNKLLLKGATFELQEFNEDYKLYLPIKNNNSKVVTGENGKISYK
DLKDGKYQLIEAVSPEDYQKITNKPILTFEVVKGSIKNIIAVNKQISEYHEEGDKHLITNTHIPPKGIIPMTGGKGILS

GBS67 contains an amino acid motif indicative of a cell wall anchor, shown in italics in SEQ ID NO: 1 above. In some recombinant host cell systems, it may be preferable to remove this motif to facilitate secretion of a recombinant GBS67 protein from the host cell. Accordingly, in one preferred fragment of GBS67 for use in the invention, the transmembrane and the cell wall anchor motif are removed from GBS67. An example of such a GBS67 fragment is set forth below as SEQ ID NO: 19.

MRKYQKFSKILTLSLFCLSQIPLNTNVLGESTVPENGAKGKLVVKKTDDQNKPLSKATFVLKTTAHPESKIEKVTAELT
GEATFDNLIPGDYTLSEETAPEGYKKTNQTWQVKVESNGKTTIQNSGDKNSTIGQNQEELDKQYPPTGIYEDTKESYKL
EHVKGSVPNGKSEAKAVNPYSSEGEHIREIPEGTLSKRISEVGDLAHNKYKIELTVSGKTIVKPVDKQKPLDVVFVLDN
SNSMNNDGPNFQRHNKAKKAAEALGTAVKDILGANSDNRVALVTYGSDIFDGRSVDVVKGFKEDDKYYGLQTKFTIQTE
NYSHKQLTNNAEEIIKRIPTEAPKAKWGSTTNGLTPEQQKEYYLSKVGETFTMKAFMEADDILSQVNRNSQKIIVHVTD
GVPTRSYAINNFKLGASYESQFEQMKKNGYLNKSNFLLTDKPEDIKGNGESYFLFPLDSYQTQIISGNLQKLHYLDLNL
NYPKGTIYRNGPVKEHGTPTKLYINSLKQKNYDIFNFGIDISGFRQVYNEEYKKNQDGTFQKLKEEAFKLSDGEITELM
RSFSSKPEYYTPIVTSADTSNNEILSKIQQQFETILTKENSIVNGTIEDPMGDKINLQLGNGQTLQPSDYTLQGNDGSV

MKDGIATGGPNNDGGILKGVKLEYIGNKLYVRGLNLGEGQKVTLTYDVKLDDSFISNKFYDTNGRTTLNPKSEDPNTLR
DFPIPKIRDVREYPTITIKNEKKLGEIEFIKVDKDNNKLLLKGATFELQEFNEDYKLYLPIKNNNSKVVTGENGKISYK
DLKDGKYQLIEAVSPEDYQKITNKPILTFEVVKGSIKNIIAVNKQISEYHEEGDKHLITNTHIPPKGI

### GBS80

GBS80 refers to a putative cell wall surface anchor family protein. Nucleotide and amino acid sequence of GBS80 sequenced from serotype V isolated strain 2603 V/R are set forth in ref. 94 as SEQ ID NOs 8779 & 8780. The amino acid sequence is set forth below as SEQ ID NO: 2:

MKLSKKLLFSAAVLTMVAGSTVEPVAQFATGMSIVRAAEVSQERPAKTTVNIYKLQADSYKSEITSNGGIENKDGEVIS
NYAKLGDNVKGLQGVQFKRYKVKTDISVDELKKLTTVEAADAKVGTILEEGVSLPQKTNAQGLVVDALDSKSNVRYLYV
EDLKNSPSNITKAYAVPFVLELPVANSTGTGFLSEINIYPKNVVTDEPKTDKDVKKLGQDDAGYTIGEEFKWFLKSTIP
ANLGDYEKFEITDKFADGLTYKSVGKIKIGSKTLNRDEHYTIDEPTVDNQNTLKITFKPEKFKEIAELLKGMTLVKNQD
ALDKATANTDDAAFLEIPVASTINEKAVLGKAIENTFELQYDHTPDKADNPKPSNPPRKPEVHTGGKRFVKKDSTETQT
LGGAEFDLLASDGTAVKWTDALIKANTNKNYIAGEAVTGQPIKLKSHTDGTFEIKGLAYAVDANAEGTAVTYKLKETKA
PEGYVIPDKEIEFTVSQTSYNTKPTDITVDSADATPDTIKNNKRPSIPNTGGIGTAIFVAIGAAVMAFAVKGMKRRTKD
N

GBS80 contains a N-terminal leader or signal sequence region which is indicated by the underlined sequence above. One or more amino acids from the leader or signal sequence region of GBS80 can be removed. An example of such a GBS80 fragment is set forth below as SEQ ID NO: 6:

AEVSQERPAKTTVNIYKLQADSYKSEITSNGGIENKDGEVISNYAKLGDNVKGLQGVQFKRYKVKTDISVDELKKLTTV
EAADAKVGTILEEGVSLPQKTNAQGLVVDALDSKSNVRYLYVEDLKNSPSNITKAYAVPFVLELPVANSTGTGFLSEIN
IYPKNVVTDEPKTDKDVKKLGQDDAGYTIGEEFKWFLKSTIPANLGDYEKFEITDKFADGLTYKSVGKIKIGSKTLNRD
EHYTIDEPTVDNQNTLKITFKPEKFKEIAELLKGMTLVKNQDALDKATANTDDAAFLEIPVASTINEKAVLGKAIENTF
ELQYDHTPDKADNPKPSNPPRKPEVHTGGKRFVKKDSTETQTLGGAEFDLLASDGTAVKWTDALIKANTNKNYIAGEAV
TGQPIKLKSHTDGTFEIKGLAYAVDANAEGTAVTYKLKETKAPEGYVIPDKEIEFTVSQTSYNTKPTDITVDSADATPD
TIKNNKRPSIPNTGGIGTAIFVAIGAAVMAFAVKGMKRRTKDN

GBS80 contains a C-terminal transmembrane region which is indicated by the underlined sequence near the end of SEQ ID NO: 2 above. One or more amino acids from the transmembrane region and/or a cytoplasmic region may be removed. An example of such a fragment is set forth below as SEQ ID NO:7:

MKLSKKLLFSAAVLTMVAGSTVEPVAQFATGMSIVRAAEVSQERPAKTTVNIYKLQADSYKSEITSNGGIENKDGEVIS
NYAKLGDNVKGLQGVQFKRYKVKTDISVDELKKLTTVEAADAKVGTILEEGVSLPQKTNAQGLVVDALDSKSNVRYLYV
EDLKNSPSNITKAYAVPFVLELPVANSTGTGFLSEINIYPKNVVTDEPKTDKDVKKLGQDDAGYTIGEEFKWFLKSTIP
ANLGDYEKFEITDKFADGLTYKSVGKIKIGSKTLNRDEHYTIDEPTVDNQNTLKITFKPEKFKEIAELLKGMTLVKNQD
ALDKATANTDDAAFLEIPVASTINEKAVLGKAIENTFELQYDHTPDKADNPKPSNPPRKPEVHTGGKRFVKKDSTETQT
LGGAEFDLLASDGTAVKWTDALIKANTNKNYIAGEAVTGQPIKLKSHTDGTFEIKGLAYAVDANAEGTAVTYKLKETKA
PEGYVIPDKEIEFTVSQTSYNTKPTDITVDSADATPDTIKNNKRPSIPNTG

GBS80 contains an amino acid motif indicative of a cell wall anchor, shown in italics in SEQ ID NO: 2 above. In some recombinant host cell systems, it may be preferable to remove this motif to facilitate secretion of a recombinant GBS80 protein from the host cell. Thus the transmembrane and/or cytoplasmic regions and the cell wall anchor motif may be removed from GBS80. An example of such a fragment is set forth below as SEQ ID NO: 8.

MKLSKKLLFSAAVLTMVAGSTVEPVAQFATGMSIVRAAEVSQERPAKTTVNIYKLQADSYKSEITSNGGIENKDGEVIS
NYAKLGDNVKGLQGVQFKRYKVKTDISVDELKKLTTVEAADAKVGTILEEGVSLPQKTNAQGLVVDALDSKSNVRYLYV
EDLKNSPSNITKAYAVPFVLELPVANSTGTGFLSEINIYPKNVVTDEPKTDKDVKKLGQDDAGYTIGEEFKWFLKSTIP
ANLGDYEKFEITDKFADGLTYKSVGKIKIGSKTLNRDEHYTIDEPTVDNQNTLKITFKPEKFKEIAELLKGMTLVKNQD
ALDKATANTDDAAFLEIPVASTINEKAVLGKAIENTFELQYDHTPDKADNPKPSNPPRKPEVHTGGKRFVKKDSTETQT
LGGAEFDLLASDGTAVKWTDALIKANTNKNYIAGEAVTGQPIKLKSHTDGTFEIKGLAYAVDANAEGTAVTYKLKETKA
PEGYVIPDKEIEFTVSQTSYNTKPTDITVDSADATPDTIKNNKRPS

Alternatively, in some recombinant host cell systems, it may be preferable to use the cell wall anchor motif to anchor the recombinantly expressed protein to the cell wall. The extracellular domain of the expressed protein may be cleaved during purification or the recombinant protein may be left attached to either inactivated host cells or cell membranes in the final composition.

In one embodiment, the leader or signal sequence region, the transmembrane and cytoplasmic regions and the cell wall anchor motif are removed from the GBS80 sequence. An example of such a GBS80 fragment is set forth below as SEQ ID NO: 9:

AEVSQERPAKTTVNIYKLQADSYKSEITSNGGIENKDGEVISNYAKLGDNVKGLQGVQFKRYKVKTDISVDELKKLTTV
EAADAKVGTILEEGVSLPQKTNAQGLVVDALDSKSNVRYLYVEDLKNSPSNITKAYAVPFVLELPVANSTGTGFLSEIN
IYPKNVVTDEPKTDKDVKKLGQDDAGYTIGEEFKWFLKSTIPANLGDYEKFEITDKFADGLTYKSVGKIKIGSKTLNRD
EHYTIDEPTVDNQNTLKITFKPEKFKEIAELLKGMTLVKNQDALDKATANTDDAAFLEIPVASTINEKAVLGKAIENTF
ELQYDHTPDKADNPKPSNPPRKPEVHTGGKRFVKKDSTETQTLGGAEFDLLASDGTAVKWTDALIKANTNKNYIAGEAV
TGQPIKLKSHTDGTFEIKGLAYAVDANAEGTAVTYKLKETKAPEGYVIPDKEIEFTVSQTSYNTKPTDITVDSADATPD
TIKNNKRPS

A particularly immunogenic fragment of GBS80 is located towards the N-terminus of the protein, and is given herein as SEQ ID NO: 10:

AEVSQERPAKTTVNIYKLQADSYKSEITSNGGIENKDGEVISNYAKLGDNVKGLQGVQFKRYKVKTDISVDELKKLTTV
EAADAKVGTILEEGVSLPQKTNAQGLVVDALDSKSNVRYLYVEDLKNSPSNITKAYAVPFVLELPVANSTGTGFLSEIN
IYPKNVVTDEPKTDKDVKKLGQDDAGYTIGEEFKWFLKSTIPANLGDYEKFEITDKFADGLTYKSVGKIKIGSKTLNRD
EHYTIDEPTVDNQNTLKITFKPEKFKEIAELLKG

### GBS104

GBS104 refers to a putative cell wall surface anchor family protein. It has been referred to as *emaA.* Nucleotide and amino acid sequences of GBS104 sequenced from serotype V isolated strain 2603 V/R are set forth in Ref. 94 as SEQ ID 8777 and SEQ ID 8778. The amino acid sequence is SEQ ID NO: 3 herein:

MKKRQKIWRGLSVTLLILSQIPFGILVQGETQDTNQALGKVIVKKTGDNATPLGKATFVLKNDNDKSETSHETVEGSGE
ATFENIKPGDYTLREETAPIGYKKTDKTWKVKVADNGATIIEGMDADKAEKRKEVLNAQYPKSAIYEDTKENYPLVNVE
GSKVGEQYKALNPINGKDGRREIAEGWLSKKITGVNDLDKNKYKIELTVEGKTTVETKELNQPLDVVVLLDNSNSMNNE
RANNSQRALKAGEAVEKLIDKITSNKDNRVALVTYASTIFDGTEATVSKGVADQNGKALNDSVSWDYHKTTFTATTHNY
SYLNLTNDANEVNILKSRIPKEAEHINGDRTLYQFGATFTQKALMKANEILETQSSNARKKLIFHVTDGVPTMSYAINF
NPYISTSYQNQFNSFLNKIPDRSGILQEDFIINGDDYQIVKGDGESFKLFSDRKVPVTGGTTQAAYRVPQNQLSVMSNE
GYAINSGYIYLYWRDYNWVYPFDPKTKKVSATKQIKTHGEPTTLYFNGNIRPKGYDIFTVGIGVNGDPGATPLEAEKFM
QSISSKTENYTNVDDTNKIYDELNKYFKTIVEEKHSIVDGNVTDPMGEMIEFQLKNGQSFTHDDYVLVGNDGSQLKNGV
ALGGPNSDGGILKDVTVTYDKTSQTIKINHLNLGSGQKVVLTYDVRLKDNYISNKFYNTNNRTTLSPKSEKEPNTIRDF
PIPKIRDVREFPVLTISNQKKMGEVEFIKVNKDKHSESLLGAKFQLQIEKDFSGYKQFVPEGSDVTTKNDGKIYFKALQ
DGNYKLYEISSPDGYIEVKTKPVVTFTIQNGEVTNLKADPNANKNQIGYLEGNGKHLITNTPKRPPGVFPKTGGIGTIV
YILVGSTFMILTICSFRRKQL

GBS104 contains an N-terminal leader or signal sequence region which is indicated by the underlined sequence at the beginning of SEQ ID NO: 3 above. One or more amino acids from the leader or signal sequence region of GBS104 may be removed. An example of such a GBS104 fragment is set forth below as SEQ ID NO 11.

GETQDTNQALGKVIVKKTGDNATPLGKATFVLKNDNDKSETSHETVEGSGEATFENIKPGDYTLREETAPIGYKKTDKT
WKVKVADNGATIIEGMDADKAEKRKEVLNAQYPKSAIYEDTKENYPLVNVEGSKVGEQYKALNPINGKDGRREIAEGWL
SKKITGVNDLDKNKYKIELTVEGKTTVETKELNQPLDVVVLLDNSNSMNNERANNSQRALKAGEAVEKLIDKITSNKDN
RVALVTYASTIFDGTEATVSKGVADQNGKALNDSVSWDYHKTTFTATTHNYSYLNLTNDANEVNILKSRIPKEAEHING
DRTLYQFGATFTQKALMKANEILETQSSNARKKLIFHVTDGVPTMSYAINFNPYISTSYQNQFNSFLNKIPDRSGILQE
DFIINGDDYQIVKGDGESFKLFSDRKVPVTGGTTQAAYRVPQNQLSVMSNEGYAINSGYIYLYWRDYNWVYPFDPKTKK
VSATKQIKTHGEPTTLYFNGNIRPKGYDIFTVGIGVNGDPGATPLEAEKFMQSISSKTENYTNVDDTNKIYDELNKYFK
TIVEEKHSIVDGNVTDPMGEMIEFQLKNGQSFTHDDYVLVGNDGSQLKNGVALGGPNSDGGILKDVTVTYDKTSQTIKI
NHLNLGSGQKVVLTYDVRLKDNYISNKFYNTNNRTTLSPKSEKEPNTIRDFPIPKIRDVREFPVLTISNQKKMGEVEFI

KVNKDKHSESLLGAKFQLQIEKDFSGYKQFVPEGSDVTTKNDGKIYFKALQDGNYKLYEISSPDGYIEVKTKPVVTFTI
QNGEVTNLKADPNANKNQIGYLEGNGKHLITNTPKRPPGVFPKTGGIGTIVYILVGSTFMILTICSFRRKQL

GBS104 contains a C-terminal transmembrane and/or cytoplasmic region which is indicated by the underlined region near the end of SEQ ID NO:3 above. One or more amino acids from the transmembrane or cytoplasmic regions may be removed. An example of such a GBS104 fragment is set forth below as SEQ ID NO 12:

MKKRQKIWRGLSVTLLILSQIPFGILVQGETQDTNQALGKVIVKKTGDNATPLGKATFVLKNDNDKSETSHETVEGSGE
ATFENIKPGDYTLREETAPIGYKKTDKTWKVKVADNGATIIEGMDADKAEKRKEVLNAQYPKSAIYEDTKENYPLVNVE
GSKVGEQYKALNPINGKDGRREIAEGWLSKKITGVNDLDKNKYKIELTVEGKTTVETKELNQPLDVVVLLDNSNSMNNE
RANNSQRALKAGEAVEKLIDKITSNKDNRVALVTYASTIFDGTEATVSKGVADQNGKALNDSVSWDYHKTTFTATTHNY
SYLNLTNDANEVNILKSRIPKEAEHINGDRTLYQFGATFTQKALMKANEILETQSSNARKKLIFHVTDGVPTMSYAINF
NPYISTSYQNQFNSFLNKIPDRSGILQEDFIINGDDYQIVKGDGESFKLFSDRKVPVTGGTTQAAYRVPQNQLSVMSNE
GYAINSGYIYLYWRDYNWVYPFDPKTKKVSATKQIKTHGEPTTLYFNGNIRPKGYDIFTVGIGVNGDPGATPLEAEKFM
QSISSKTENYTNVDDTNKIYDELNKYFKTIVEEKHSIVDGNVTDPMGEMIEFQLKNGQSFTHDDYVLVGNDGSQLKNGV
ALGGPNSDGGILKDVTVTYDKTSQTIKINHLNLGSGQKVVLTYDVRLKDNYISNKFYNTNNRTTLSPKSEKEPNTIRDF
PIPKIRDVREFPVLTISNQKKMGEVEFIKVNKDKHSESLLGAKFQLQIEKDFSGYKQFVPEGSDVTTKNDGKIYFKALQ
DGNYKLYEISSPDGYIEVKTKPVVTFTIQNGEVTNLKADPNANKNQIGYLEGNGKHLITNT

One or more amino acids from the leader or signal sequence region and one or more amino acids from the transmembrane or cytoplasmic regions may be removed. An example of such a GBS104 fragment is set forth below as SEQ ID NO 13:

GETQDTNQALGKVIVKKTGDNATPLGKATFVLKNDNDKSETSHETVEGSGEATFENIKPGDYTLREETAPIGYKKTDKT
WKVKVADNGATIIEGMDADKAEKRKEVLNAQYPKSAIYEDTKENYPLVNVEGSKVGEQYKALNPINGKDGRREIAEGWL
SKKITGVNDLDKNKYKIELTVEGKTTVETKELNQPLDVVVLLDNSNSMNNERANNSQRALKAGEAVEKLIDKITSNKDN
RVALVTYASTIFDGTEATVSKGVADQNGKALNDSVSWDYHKTTFTATTHNYSYLNLTNDANEVNILKSRIPKEAEHING
DRTLYQFGATFTQKALMKANEILETQSSNARKKLIFHVTDGVPTMSYAINFNPYISTSYQNQFNSFLNKIPDRSGILQE
DFIINGDDYQIVKGDGESFKLFSDRKVPVTGGTTQAAYRVPQNQLSVMSNEGYAINSGYIYLYWRDYNWVYPFDPKTKK
VSATKQIKTHGEPTTLYFNGNIRPKGYDIFTVGIGVNGDPGATPLEAEKFMQSISSKTENYTNVDDTNKIYDELNKYFK
TIVEEKHSIVDGNVTDPMGEMIEFQLKNGQSFTHDDYVLVGNDGSQLKNGVALGGPNSDGGILKDVTVTYDKTSQTIKI
NHLNLGSGQKVVLTYDVRLKDNYISNKFYNTNNRTTLSPKSEKEPNTIRDFPIPKIRDVREFPVLTISNQKKMGEVEFI
KVNKDKHSESLLGAKFQLQIEKDFSGYKQFVPEGSDVTTKNDGKIYFKALQDGNYKLYEISSPDGYIEVKTKPVVTFTI
QNGEVTNLKADPNANKNQIGYLEGNGKHLITNT

Further fragments of GBS104 include an 830 amino acid fragment of GBS104 of amino acids 28-858 (numbered by SEQ ID NO: 3), a 359 amino acid fragment of GBS104 of amino acids 28-387, a 581 amino acid fragment of GBS104 of amino acids 28-609, or a 740 amino acid fragment of GBS104 of amino acids 28-768.

### GBS276

GBS276 refers to a C5a peptidase. Further description of GBS276 can be found in references 128-131. Nucleotide and amino acid sequences of GBS276 sequenced from serotype V isolated strain 2603 V/R are set forth in Ref. 94 as SEQ ID NOs 8941 & 8942. The amino acid sequence is SEQ ID NO: 4 herein: 
MRKKQKLPFDKLAIALISTSILLNAQSDIKANTVTEDTPATEQAVEPPQPIAVSEESRSSKETKTSQTPSDVGETVADD
ANDLAPQAPAKTADTPATSKATIRDLNDPSHVKTLQEKAGKGAGTVVAVIDAGFDKNHEAWRLTDKTKARYQSKENLEK
AKKEHGITYGEWVNDKVAYYHDYSKDGKNAVDQEHGTHVSGILSGNAPSEMKEPYRLEGAMPEAQLLLMRVEIVNGLAD
YARNYAQAIRDAVNLGAKVINMSFGNAALAYANLPDETKKAFDYAKSKGVSIVTSAGNDSSFGGKPRLPLADHPDYGVV
GTPAAADSTLTVASYSPDKQLTETATVKTDDHQDKEMPVISTNRFEPNKAYDYAYANRGTKEDDFKDVEGKIALIERGD
IDFKDKIANAKKAGAVGVLIYDNQDKGFPIELPNVDQMPAAFISRRDGLLLKDNPPKTITFNATPKVLPTASGTKLSRF
SSWGLTADGNIKPDIAAPGQDILSSVANNKYAKLSGTSMSAPLVAGIMGLLQKQYETQYPDMTPSERLDLAKKVLMSSA
TALYDEDEKAYFSPRQQGAGAVDAKKASAATMYVTDKDNTSSKVHLNNVSDKFEVTVTVHNKSDKPQELYYQVTVQTDK
VDGKHFALAPKALYETSWQKITIPANSSKQVTVPIDASRFSKDLLAQMKNGYFLEGFVRFKQDPTKEELMSIPYIGFRG
DFGNLSALEKPIYDSKDGSSYYHEANSDAKDQLDGDGLQFYALKNNFTALTTESNPWTIIKAVKEGVENIEDIESSEIT
ETIFAGTFAKQDDDSHYYIHRHANGKPYAAISPNGDGNRDYVQFQGTFLRNAKNLVAEVLDKEGNVVWTSEVTEQVVKN
YNNDLASTLGSTRFEKTRWDGKDKDGKVVANGTYTYRVRYTPISSGAKEQHTDFDVIVDNTTPEVATSATFSTEDSRLT
 
LASKPKTSQPVYRERIAYTYMDEDLPTTEYISPNEDGTFTLPEEAETMEGATVPLKMSDFTYVVEDMAGNITYTPVTKL
LEGHSNKPEQDGSDQAPDKKPEAKPEQDGSGQTPDKKKETKPEKDSSGQTPGKTPQKGQSSRTLEKRSSKRALATKAST
RDQLPTTNDKDTNRLHLLKLVMTTFFLG
 GBS276 contains an N-terminal leader or signal sequence region which is indicated by the underlined sequence at the beginning of SEQ ID NO: 4 above. One or more amino acids from the leader or signal sequence region of GBS276 may be removed. An example of such a GBS276 fragment is set forth below as SEQ ID NO: 14:

QSDIKANTVTEDTPATEQAVEPPQPIAVSEESRSSKETKTSQTPSDVGETVADDANDLAPQAPAKTADTPATSKATIRD
LNDPSHVKTLQEKAGKGAGTVVAVIDAGFDKNHEAWRLTDKTKARYQSKENLEKAKKEHGITYGEWVNDKVAYYHDYSK
DGKNAVDQEHGTHVSGILSGNAPSEMKEPYRLEGAMPEAQLLLMRVEIVNGLADYARNYAQAIRDAVNLGAKVINMSFG
NAALAYANLPDETKKAFDYAKSKGVSIVTSAGNDSSFGGKPRLPLADHPDYGVVGTPAAADSTLTVASYSPDKQLTETA
TVKTDDHQDKEMPVISTNRFEPNKAYDYAYANRGTKEDDFKDVEGKIALIERGDIDFKDKIANAKKAGAVGVLIYDNQD
KGFPIELPNVDQMPAAFISRRDGLLLKDNPPKTITFNATPKVLPTASGTKLSRFSSWGLTADGNIKPDIAAPGQDILSS
VANNKYAKLSGTSMSAPLVAGIMGLLQKQYETQYPDMTPSERLDLAKKVLMSSATALYDEDEKAYFSPRQQGAGAVDAK
KASAATMYVTDKDNTSSKVHLNNVSDKFEVTVTVHNKSDKPQELYYQVTVQTDKVDGKHFALAPKALYETSWQKITIPA
NSSKQVTVPIDASRFSKDLLAQMKNGYFLEGFVRFKQDPTKEELMSIPYIGFRGDFGNLSALEKPIYDSKDGSSYYHEA
NSDAKDQLDGDGLQFYALKNNFTALTTESNPWTIIKAVKEGVENIEDIESSEITETIFAGTFAKQDDDSHYYIHRHANG
KPYAAISPNGDGNRDYVQFQGTFLRNAKNLVAEVLDKEGNVVWTSEVTEQVVKNYNNDLASTLGSTRFEKTRWDGKDKD
GKVVANGTYTYRVRYTPISSGAKEQHTDFDVIVDNTTPEVATSATFSTEDSRLTLASKPKTSQPVYRERIAYTYMDEDL
PTTEYISPNEDGTFTLPEEAETMEGATVPLKMSDFTYVVEDMAGNITYTPVTKLLEGHSNKPEQDGSDQAPDKKPEAKP
EQDGSGQTPDKKKETKPEKDSSGQTPGKTPQKGQSSRTLEKRSSKRALATKASTRDQLPTTNDKDTNRLHLLKLVMTTF
FLG

GBS276 contains a C-terminal transmembrane and/or cytoplasmic region which is indicated by the underlined sequence near the end of SEQ ID NO: 4 above. One or more amino acids from the transmembrane or cytoplasmic regions of GBS276 may be removed. An example of such a GBS276 fragment is set forth below as SEQ ID NO: 15:

MRKKQKLPFDKLAIALISTSILLNAQSDIKANTVTEDTPATEQAVEPPQPIAVSEESRSSKETKTSQTPSDVGETVADD
ANDLAPQAPAKTADTPATSKATIRDLNDPSHVKTLQEKAGKGAGTVVAVIDAGFDKNHEAWRLTDKTKARYQSKENLEK
AKKEHGITYGEWVNDKVAYYHDYSKDGKNAVDQEHGTHVSGILSGNAPSEMKEPYRLEGAMPEAQLLLMRVEIVNGLAD
YARNYAQAIRDAVNLGAKVINMSFGNAALAYANLPDETKKAFDYAKSKGVSIVTSAGNDSSFGGKPRLPLADHPDYGVV
GTPAAADSTLTVASYSPDKQLTETATVKTDDHQDKEMPVISTNRFEPNKAYDYAYANRGTKEDDFKDVEGKIALIERGD
IDFKDKIANAKKAGAVGVLIYDNQDKGFPIELPNVDQMPAAFISRRDGLLLKDNPPKTITFNATPKVLPTASGTKLSRF
SSWGLTADGNIKPDIAAPGQDILSSVANNKYAKLSGTSMSAPLVAGIMGLLQKQYETQYPDMTPSERLDLAKKVLMSSA
TALYDEDEKAYFSPRQQGAGAVDAKKASAATMYVTDKDNTSSKVHLNNVSDKFEVTVTVHNKSDKPQELYYQVTVQTDK
VDGKHFALAPKALYETSWQKITIPANSSKQVTVPIDASRFSKDLLAQMKNGYFLEGFVRFKQDPTKEELMSIPYIGFRG
DFGNLSALEKPIYDSKDGSSYYHEANSDAKDQLDGDGLQFYALKNNFTALTTESNPWTIIKAVKEGVENIEDIESSEIT
ETIFAGTFAKQDDDSHYYIHRHANGKPYAAISPNGDGNRDYVQFQGTFLRNAKNLVAEVLDKEGNVVWTSEVTEQVVKN
YNNDLASTLGSTRFEKTRWDGKDKDGKVVANGTYTYRVRYTPISSGAKEQHTDFDVIVDNTTPEVATSATFSTEDSRLT
LASKPKTSQPVYRERIAYTYMDEDLPTTEYISPNEDGTFTLPEEAETMEGATVPLKMSDFTYVVEDMAGNITYTPVTKL
LEGHSNKPEQDGSDQAPDKKPEAKPEQDGSGQTPDKKKETKPEKDSSGQTPGKTPQKGQSSRTLEKRSSKRALATK

One or more amino acids from the leader or signal sequence region and one or more amino acids from the transmembrane or cytoplasmic regions of GBS276 may be removed. An example of such a GBS276 fragment is set forth below as SEQ ID NO: 16:

QSDIKANTVTEDTPATEQAVEPPQPIAVSEESRSSKETKTSQTPSDVGETVADDANDLAPQAPAKTADTPATSKATIRD
LNDPSHVKTLQEKAGKGAGTVVAVIDAGFDKNHEAWRLTDKTKARYQSKENLEKAKKEHGITYGEWVNDKVAYYHDYSK
DGKNAVDQEHGTHVSGILSGNAPSEMKEPYRLEGAMPEAQLLLMRVEIVNGLADYARNYAQAIRDAVNLGAKVINMSFG
NAALAYANLPDETKKAFDYAKSKGVSIVTSAGNDSSFGGKPRLPLADHPDYGVVGTPAAADSTLTVASYSPDKQLTETA
TVKTDDHQDKEMPVISTNRFEPNKAYDYAYANRGTKEDDFKDVEGKIALIERGDIDFKDKIANAKKAGAVGVLIYDNQD
KGFPIELPNVDQMPAAFISRRDGLLLKDNPPKTITFNATPKVLPTASGTKLSRFSSWGLTADGNIKPDIAAPGQDILSS
VANNKYAKLSGTSMSAPLVAGIMGLLQKQYETQYPDMTPSERLDLAKKVLMSSATALYDEDEKAYFSPRQQGAGAVDAK
KASAATMYVTDKDNTSSKVHLNNVSDKFEVTVTVHNKSDKPQELYYQVTVQTDKVDGKHFALAPKALYETSWQKITIPA
NSSKQVTVPIDASRFSKDLLAQMKNGYFLEGFVRFKQDPTKEELMSIPYIGFRGDFGNLSALEKPIYDSKDGSSYYHEA
NSDAKDQLDGDGLQFYALKNNFTALTTESNPWTIIKAVKEGVENIEDIESSEITETIFAGTFAKQDDDSHYYIHRHANG
KPYAAISPNGDGNRDYVQFQGTFLRNAKNLVAEVLDKEGNVVWTSEVTEQVVKNYNNDLASTLGSTRFEKTRWDGKDKD

GKVVANGTYTYRVRYTPISSGAKEQHTDFDVIVDNTTPEVATSATFSTEDSRLTLASKPKTSQPVYRERIAYTYMDEDL
PTTEYISPNEDGTFTLPEEAETMEGATVPLKMSDFTYVVEDMAGNITYTPVTKLLEGHSNKPEQDGSDQAPDKKPEAKP
EQDGSGQTPDKKKETKPEKDSSGQTPGKTPQKGQSSRTLEKRSSKRALATK

### GBS322.

GBS322 refers to a surface immunogenic protein, also referred to as 'sip'. Nucleotide and amino acid sequences of GBS322 sequenced from serotype V isolated strain 2603 V/R are set forth in Ref. 94 as SEQ ID NOs 8539 & 8540. The amino acid sequence is SEQ ID NO: 5 herein:

MNKKVLLTSTMAASLLSVASVQAQETDTTWTARTVSEVKADLVKQDNKSSYTVKYGDTLSVISEAMSIDMNVLAKINNI
ADINLIYPETTLTVTYDQKSHTATSMKIETPATNAAGQTTATVDLKTNQVSVADQKVSLNTISEGMTPEAATTIVSPMK
TYSSAPALKSKEVLAQEQAVSQAAANEQVSPAPVKSITSEVPAAKEEVKPTQTSVSQSTTVSPASVAAETPAPVAKVAP
VRTVAAPRVASVKVVTPKVETGASPEHVSAPAVPVTTTSPATDSKLQATEVKSVPVAQKAPTATPVAQPASTTNAVAAH
PENAGLQPHVAAYKEKVASTYGVNEFSTYRAGDPGDHGKGLAVDFIVGTNQALGNKVAQYSTQNMAANNISYVIWQQKF
YSNTNSIYGPANTWNAMPDRGGVTANHYDHVHVSFNK

GBS322 contains a N-terminal leader or signal sequence region which is indicated by the underlined sequence near the beginning of SEQ ID NO: 5. One or more amino acids from the leader or signal sequence region of GBS322 may be removed. An example of such a GBS322 fragment is set forth below as SEQ ID NO: 17:

DLVKQDNKSSYTVKYGDTLSVISEAMSIDMNVLAKINNIADINLIYPETTLTVTYDQKSHTATSMKIETPATNAAGQTT
ATVDLKTNQVSVADQKVSLNTISEGMTPEAATTIVSPMKTYSSAPALKSKEVLAQEQAVSQAAANEQVSPAPVKSITSE
VPAAKEEVKPTQTSVSQSTTVSPASVAAETPAPVAKVAPVRTVAAPRVASVKVVTPKVETGASPEHVSAPAVPVTTTSP
ATDSKLQATEVKSVPVAQKAPTATPVAQPASTTNAVAAHPENAGLQPHVAAYKEKVASTYGVNEFSTYRAGDPGDHGKG
LAVDFIVGTNQALGNKVAQYSTQNMAANNISYVIWQQKFYSNTNSIYGPANTWNAMPDRGGVTANHYDHVHVSFNK

### General

The term "comprising" encompasses "including" as well as "consisting" *e.g*. a composition "comprising" X may consist exclusively of X or may include something additional *e.g*. X + Y.

The term "about" in relation to a numerical value *x* means, for example, *x*±10%.

The word "substantially" does not exclude "completely" *e.g*. a composition which is "substantially free" from Y may be completely free from Y. Where necessary, the word "substantially" may be omitted from the definition of the invention.

Where the invention provides a process involving multiple sequential steps, the invention can also provide a process involving less than the total number of steps. For example, if a saccharide has already been partially purified by removing contaminating nucleic acids and/or proteins then this step can be omitted from the processes of the invention. Similarly, a step of removing contaminants can be performed to give material ready for detergent-mediated precipitation, but the precipitation need not be performed. The precipitation step need not be performed in order to fall within the scope of the invention, as the pre-precipitation material has utility as an intermediate in saccharide preparation, and may be used, stored, exported, *etc.* for later use *e.g.* for later precipitation. These different steps can be performed at very different times by different people in different places (*e.g*. in different countries).

It will be appreciated that sugar rings can exist in open and closed form and that, whilst closed forms are shown in structural formulae herein, open forms are also encompassed by the invention. Similarly, it will be appreciated that sugars can exist in pyranose and furanose forms and that, whilst pyranose forms are shown in structural formulae herein, furanose forms are also encompassed. Different anomeric forms of sugars are also encompassed.

### BRIEF DESCRIPTION OF DRAWINGS

Figure 1 illustrates the capsular polysaccharide (left) and the group-specific polysaccharide (right) attached to the peptidoglycan of GBS.
Figure 2 shows the repeating structures of capsular saccharides in GBS serotypes Ia, Ib, II, III & V.
Figure 3 shows the difference between the repeating structures in GBS serotypes Ia and III.
Figure 4 shows the tetraantennary structure of the group B antigen. A-D represent the major component oligosaccharides, and P represents phosphate [12].
Figure 5 shows two types of conjugate that can be prepared.
Figure 6 is a flowchart showing an overall process of the invention.
Figure 7 is a UV absorbance spectrum of six GBS capsular polysaccharide presentations. A peak or shoulder is visible at around 275nm. At that point, the top three spectra are for material purified by prior art methods, and the bottom three spectra are for material purified according to the invention. In order from top to bottom at the ∼275nm point: Ib; Ia; III; Ia; Ib; III.
Figures 8 to 10 show NMR spectra for different serotypes: (8) Ia; (9) Ib; (10) III.

### MODES FOR CARRYING OUT THE INVENTION

### A. Purification of capsular saccharide from GBS serotypes Ia, Ib and III

The supernatant of a group B streptococcus culture was collected after centrifugation and was treated with sodium hydroxide (final concentration 0.8M) at 37°C for 36 hours. The resulting suspension was neutralised by addition of HCl. A mixture of aqueous ethanol (30%) and CaCl₂ (0.1M) was added to the neutralised mixture. A precipitate rapidly formed, which was removed by centrifugation. Sialic acid assays showed that the capsular saccharide remained in the supernatant. The supernatant was subjected to dead-end microfiltration in regnerated cellulose filters (0.22µm cut-off), and was then subjected to tangential flow diafiltration using a 30kDa cut-off cellulose membrane for around 2 hours. The capsular saccharide was in the ultrafiltration retentate. The retentate was treated by adding 10% CTAB detergent until a precipitate formed (within minutes). The precipitated material (including the capsular polysaccharide) was separated by centrifugation. The pellet was re-solubilised by addition of 0.1M aqueous CaCl₂. A further precipitation step was performed by adding 96% ethanol to give a final ethanol concentration of 80%. The precipitate was again removed by centrifugation, and the pellet was dried by vacuum drying.

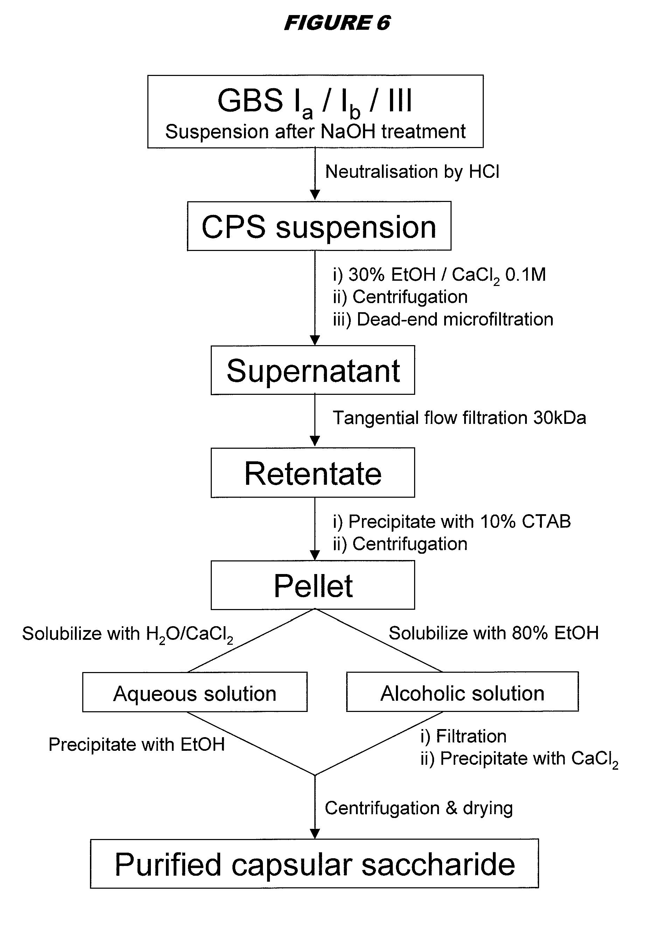

The overall process (illustrated in Figure 6) took 2-3 days and had a yield of about 60%. For the final dried material the following parameters were tested: total weight; capsular saccharide weight; and sialic acid content. Results for the three serotypes were as follows:

| | **Total w/v (mg/ml)*** | **Sialic acid content (mg/ml)** | **Saccharide content (mg/ml)** | **Purity (%)** |
|---|---|---|---|---|
| **Ia** | 18.33 | 4.57 | 14.74 | 80.4 |
| **Ib** | 19.67 | 5.03 | 16.23 | 82.5 |
| **III** | 16.33 | 4.08 | 13.16 | 80.6 |

| | | | | |
|---|---|---|---|---|
| * Standard solution obtained by solubilising dried powder in water | | | | |

The purity in each case was better than could be achieved by the prior art methods, particularly for the serotype III material (89% *vs*. 74%). In contrast to the prior art, however, the process took 2-3 days (compared to 15-20 days) and had a yield of about 60% (as compared to about 20%).

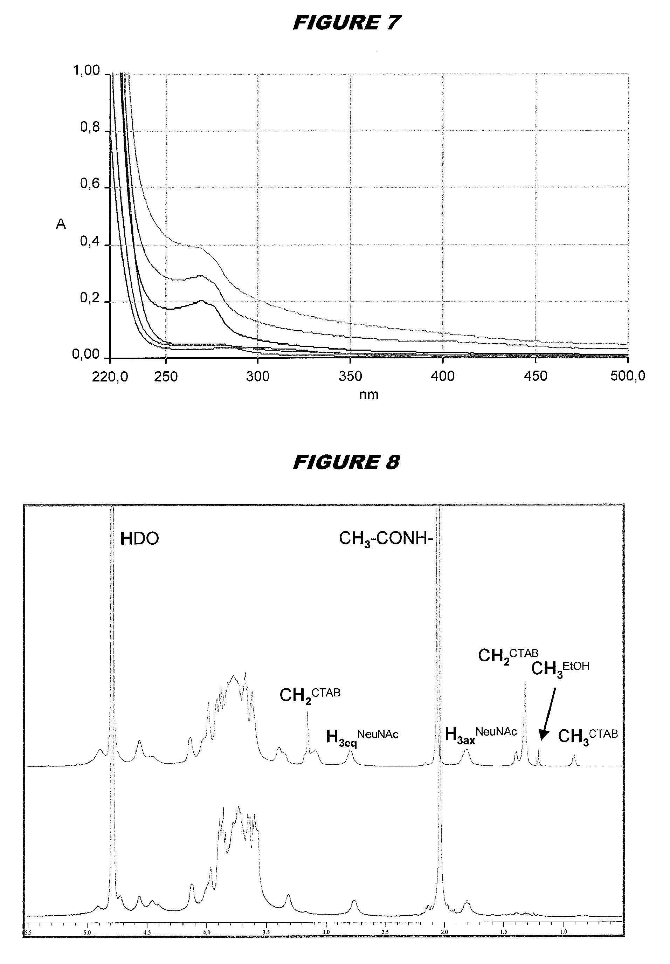

Contamination with proteins and nucleic acids was assessed by UV absorption. Saccharides prepared by the prior art processes were also tested for comparison. The spectra are shown in Figure 7, and the prior art material has a clear peak at around 270nm for each of the three serogroups. In contrast, material purified by the process of the invention has a flat spectrum in this region. The ratio of absorbances at 280nm and 260nm were as follows:

| **Serotype** | **Prior art ^{280nm}/₂₆₀ₙₘ** | **Invention ^{280nm}/₂₆₀ₙₘ** |
|---|---|---|
| **Ia** | 0.81 | 1.11 |
| **Ib** | 0.79 | 1.03 |
| **III** | 0.80 | 1.04 |

These ratios show that the material prepared by the methods of the invention is less contaminated than material prepared by the methods of the prior art.

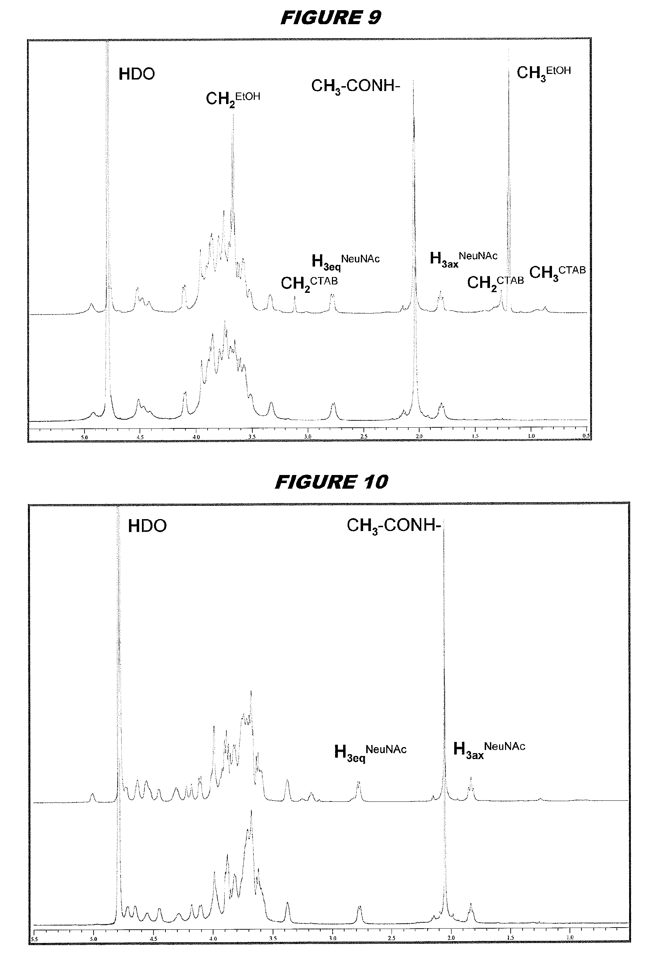

NMR was used to study the saccharides and, in particular, to assess the degree of N-acetylation. The NMR spectra are shown in Figures 8 to 10. The spectra for the prior art process and the invention's process are overlaid, with the lower spectrum being the prior art material. The calculated %s N-acetylation were as follows:

| **Serotype** | **Prior art ^{%}** | **Invention ^{%}** |
|---|---|---|
| **Ia** | 95.5 | 52.0 |
| **Ib** | 76.4 | 85.4 |
| **III** | 77.9 | 66.0 |

### B. Conjugation of purified capsular saccharides

Capsular saccharides from each of GBS serotypes Ia, Ib and III were purified and re-acetylated. The saccharides were then covalently conjugated either to monomeric tetanus toxoid (TT) or to CRM197 carrier proteins by direct reductive amination. Results were as follows:

| Conjugate | % Oxidation | Saccharide (mg/ml) | Carrier (mg/ml) | Saccharide/Protein ratio (w/w) |
|---|---|---|---|---|
| | | | | |
| Ia-TT | 10.8 | 2.033 | 0.865 | 2.35 |
| Ia-CRM | 9.1 | 1.156 | 0.401 | 2.88 |
| | | | | |
| Ib-TT | 15.2 | 1.740 | 1.271 | 1.37 |
| Ib-CRM | 8.2 | 0.898 | 0.448 | 2.00 |
| | | | | |
| III-TT | 14.3 | 0.964 | 0.631 | 1.53 |
| III-CRM | 6.5 | 1.105 | 0.626 | 1.77 |

### C. Challenge studies with conjugates

Capsular saccharide from a serotype Ia strain was purified either using the prior art processes or by the methods of the invention. For conjugation, a fraction of the sialic acid residues in the saccharide was oxidised, with a target of between 5 and 15%.

Two lots of material purified by the prior art methods had oxidation percentages of 54.5% and 17.6%. Material purified according to the invention was 6.6% oxidised.

The saccharides were conjugated to tetanus toxoid by reductive amination. Immunizations of mice were performed in parallel with the three conjugates at 0 and 21 days with 1µg saccharide per dose. Groups of CD-1 outbred female mice 6-7 weeks old (Charles River Laboratories) received the conjugate suspended in 250/µl PBS and an equal volume of PBS containing an aluminium hydroxide adjuvant (2 mg/ml final concentration) by intra-peritoneal injection. Mice were then challenged with three different serotypes. Survival rates were as follows:

| | **Challenge strain** | | |
|---|---|---|---|
| **Saccharide** | **A909** | **515** | **090** |
| Prior art, 54.5% | 7 | 24 | 18 |
| Prior art, 17.6% | 100 | 47 | 50 |
| Invention, 5.5% | 100 | 97 | 93 |
| PBS control | 12 | 17 | 0 |

Thus saccharides purified by the process of the invention are immunologically superior to those purified by prior art methods.

### Opsonophagocytosis studies

TT-conjugates saccharides from serotypes Ia, Ib and III were prepared, either by the prior art methods [1-9] or by the methods of the invention. Groups of four CD-1 outbred female mice 6-7 weeks old (Charles River Laboratories) were immunized with the conjugates (dose: 1/µg saccharide) suspended in 250 µl PBS and an equal volume of PBS containing an aluminium hydroxide adjuvant (2mg/ml final concentration). Each group received two doses at days 0 and 21 by intra-peritoneal injection. In each immunization scheme negative and positive control groups were also used.

Immune responses were determined from serum samples taken on days 0 and 36. The sera were analyzed as pools from each group of mice, against 7 different GBS strains, including '515' (type Ia; MLST type ST23), 'COH1' (type III; MLST type ST17) and 'H36B' (type Ib; MLST type ST6). Both protection and the opsonic titres were measured, and results were as follows:

| **Serotype Conjugation** | **Challenge strain Protection (%)** | | | **Opsonic titre** |
|---|---|---|---|---|
| **Ia** | **COH1** | **M781** | - | |
| Invention | 100 | 92 | - | 1400 |
| Prior art | 100 | - | - | 380 |
| PBS control | 15 | 17 | - | - |
| **Ib** | **7357b** | **H36B** | - | |
| Invention | 95 | 87 | - | 6500 |
| Prior art | 90 | - | - | 500 |
| PBS control | 25 | 0 | - | - |
| **III** | **515** | **090** | **A909** | |
| Invention | 93 | 95 | 100 | 2150 |
| Prior art | 47 | 50 | 100 | 300 |
| PBS control | 5 | 0 | 17 | - |

The data show that the saccharides purified by the methods of the invention give equivalent or improved protective efficacy compared to the material purified by prior art methods. Moreover, where protective efficacy is comparable, material purified by the methods of the invention gives improved opsonic titres.

It will be understood that the invention has been described by way of example only and modifications may be made whilst remaining within the scope and spirit of the invention.

### REFERENCES (the contents of which are hereby incorporated in full)

[1] Paoletti et al. (1990) J Biol Chem 265:18278-83.
[2] Wessels et al. (1990) J Clin Invest 86:1428-33.
[3] Paoletti et al. (1992) Infect Immun 60:4009-14.
[4] Paoletti et al. (1992) J Clin Invest 89:203-9.
[5] Wessels et al. (1987) Proc Natl Acad Sci USA 84:9170-4.
[6] Wang et al. (2003) Vaccine 21:1112-7.
[7] Wessels et al. (1993) Infect Immun 61:4760-6
[8] Wessels et al. (1995) J Infect Dis 171:879-84.
[9] Baker et al. (2004) J Infect Dis 189:1103-12.
[10] Wessels et al. (1989) Infect Immun 57:1089-94.
[11] WO02/00249.
[12] Deng et al. (2000) J Biol Chem 275:7497-04.
[13] US patents 6027733 & 6274144.
[14] www.polymer.de
[15] Lewis et al. (2004) PNAS USA 101:11123-8*.*
[16] US patent 6248570.
[17] Inzana (1987) Infect. Immun. 55:1573-79.
[18] Wessels et al. (1998) Infect Immun 66:2186-92.
[19] Ramsay et al. (2001) Lancet 357(9251):195-196.
[20] Lindberg (1999) Vaccine 17 Suppl 2:528-36.
[21] Buttery & Moxon (2000) J R Coll Physicians Lond 34:163-68.
[22] Ahmad & Chapnick (1999) Infect Dis Clin North Am 13:113-33, vii.
[23] Goldblatt (1998) J. Med. Microbiol. 47:563-7.
[24] European patent 0477508.
[25] US patent 5,306,492.
[26] WO98/42721.
[27] Dick et al. in Conjugate Vaccines (eds. Cruse et al.) Karger, Basel, 1989, 10:48-114.
[28] Hermanson Bioconjugae Techniques, Academic Press, San Diego (1996) ISBN: 0123423368.
[29] US patent 4356170.
[30] Paoletti & Kasper (2003) Expert Opin Biol Ther 3:975-84.
[31] International patent application PCT/IB05/_, 'CONJUGATION OF STREPTOCOCCAL CAPSULAR SACCHARIDES', claiming priority from GB-0502095.3 (in CHIRON SRL).
[32] Anonymous (Jan 2002) Research Disclosure, 453077.
[33] Anderson (1983) Infect Immun 39(1):233-8.
[34] Anderson et al. (1985) J Clin Invest 76(1):52-9.
[35] EP-A-0372501.
[36] EP-A-0378881.
[37] EP-A-0427347.
[38] WO93/17712
[39] WO94/03208.
[40] WO98/58668.
[41] EP-A-0471177.
[42] WO91/01146
[43] Falugi et al. (2001) Eur J Immunol 31:3816-24.
[44] Baraldo et al. (2004) Infect Immun 72(8):4884-7.
[45] EP-A-0594610.
[46] WO00/56360.
[47] WO02/091998.
[48] Kuo et al. (1995) Infect Immun 63:2706-13.
[49] WO01/72337
[50] WO00/61761.
[51] WO99/42130.
[52] W02004/011027.
[53] WO96/40242.
[54] Lei et al. (2000) Dev Biol (Basel) 103:259-264.
[55] WO00/38711; US patent 6,146,902.
[56] WO94/06467.
[57] WO99/24578.
[58] WO99/36544.
[59] WO99/57280.
[60] WO00/22430.
[61] Tettelin et al. (2000) Science 287:1809-1815.
[62] WO96/29412.
[63] Pizza et al. (2000) Science 287:1816-1820.
[64] WO01/52885.
[65] Bjune et al. (1991) Lancet 338(8775):1093-1096.
[66] Fukasawa et al. (1999) Vaccine 17:2951-2958.
[67] Rosenqvist et al. (1998) Dev. Biol. Stand. 92:323-333.
[68] Costantino et al. (1992) Vaccine 10:691-698.
[69] WO03/007985.
[70] Watson (2000) Pediatr Infect Dis J 19:331-332.
[71] Rubin (2000) Pediatr Clin North Am 47:269-285, v.
[72] Jedrzejas (2001) Microbiol Mol Biol Rev 65:187-207.
[73] Bell (2000) Pediatr Infect Dis J 19:1187-1188.
[74] Iwarson (1995) APMIS 103:321-326.
[75] Gerlich et al. (1990) Vaccine 8 Suppl:S63-68 & 79-80.
[76] Hsu et al. (1999) Clin Liver Dis 3:901-915.
[77] Gustafsson et al. (1996) N. Engl. J. Med. 334:349-355.
[78] Rappuoli etal. (1991) TIBTECH 9:232-238.
[79] Vaccines (2004) eds. Plotkin & Orenstein. ISBN 0-7216-9688-0.
[80] WO02/02606.
[81] Kalman et al. (1999) Nature Genetics 21:385-389.
[82] Read et al. (2000) Nucleic Acids Res 28:1397-406.
[83] Shirai et al. (2000) J. Infect. Dis. 181(Suppl 3):S524-S527.
[84] WO99/27105.
[85] WO00/27994.
[86] WO00/37494.
[87] WO99/28475.
[88] Ross et al. (2001) Vaccine 19:4135-4142.
[89] Sutter et al. (2000) Pediatr Clin North Am 47:287-308.
[90] Zimmerman & Spann (1999) Am Fam Physician 59:113-118, 125-126.
[91] Dreesen (1997) Vaccine 15 Suppl:S2-6.
*[92]* MMWR Morb Mortal Wkly Rep 1998 Jan 16;47(1):12, 19.
[93] McMichael (2000) Vaccine 19 Suppl 1:5101-107.
[94] WO02/34771.
[95] Dale (1999) Infect Dis Clin North Am 13:227-43, viii.
[96] Ferretti et al. (2001) PNAS USA 98: 4658-4663.
[97] Robinson & Torres (1997) Seminars in Immunology 9:271-283.
[98] Donnelly et al. (1997) Annu Rev Immunol 15:617-648.
[99] Scott-Taylor & Dalgleish (2000) Expert Opin Investig Drugs 9:471-480.
[100] Apostolopoulos & Plebanski (2000) Curr Opin Mol Ther 2:441-447.
[101] Ilan (1999) Curr Opin Mol Ther 1:116-120.
[102] Dubensky et al. (2000) Mol Med 6:723-732.
[103] Robinson & Pertmer (2000) Adv Virus Res 55:1-74.
[104] Donnelly et al. (2000) Am J Respir Crit Care Med 162(4 Pt 2):S190-193.
[105] Davis (1999) Mt. Sinai J. Med. 66:84-90.
[106] Gennaro (2000) Remington: The Science and Practice of Pharmacy. 20th edition, ISBN: 0683306472.
[107] WO03/093306.
[108] WO2004/018646.
[109] W02004/041157.
[110] Geysen et al. (1984) PNAS USA 81:3998-4002.
[111] Carter (1994) Methods Mol Biol 36:207-23.
[112] Jameson, BA et al. 1988, CABIOS 4(1):181-186.
[113] Raddrizzani & Hammer (2000) Brief Bioinform 1(2):179-89.
[114] De Lalla et al. (1999) J. Immunol. 163:1725-29.
[115] Brusic et al. (1998) Bioinformatics 14(2):121-30
[116] Meister et al. (1995) Vaccine 13(6):581-91.
[117] Roberts et al. (1996) AIDS Res Hum Retroviruses 12(7):593-610.
[118] Maksyutov & Zagrebelnaya (1993) Comput Appl Biosci 9(3):291-7.
[119] Feller & de la Cruz (1991) Nature 349(6311):720-1.
[120] Hopp (1993) Peptide Research 6:183-190.
[121] Welling et al. (1985) FEBS Lett. 188:215-218.
[122] Davenport et al. (1995) Immunogenetics 42:392-297.
[123] Bodanszky (1993) Principles of Peptide Synthesis (ISBN: 0387564314).
[124] Fields et al. (1997) Meth Enzymol 289: Solid-Phase Peptide Synthesis. ISBN: 0121821900.
[125] Chan & White (2000) Fmoc Solid Phase Peptide Synthesis. ISBN: 0199637245.
[126] Kullmann (1987) Enzymatic Peptide Synthesis. ISBN: 0849368413.
[127] Ibba (1996) Biotechnol Genet Eng Rev 13:197-216.
[128] Qi Chen et al. (2002) Infect Immun 70:6409-15.
[129] Beckmann et al. (2002) Infect Immun 70:2869-76.
[130] Cheng et al. (2002) Infect Immun 70:2408-13.
[131] Cheng et al. (2001) Infect Immun 69:2302-8.

The invention also provides the following numbered embodiments:
1. A process for purifying a *Streptococcus agalactiae* capsular polysaccharide, comprising the steps of: (a) treating a suspension comprising streptococcal proteins, nucleic acids and capsular polysaccharide with an aqueous metal cation and an alcohol in order to precipitate nucleic acids and proteins; (b) separating the precipitated material from the aqueous material; and (c) treating the aqueous material with a cationic detergent in order to precipitate the capsular polysaccharide.
2. The process of embodiment 1, wherein the polysaccharide is from a *S.agalactiae* serotype selected from Ia, Ib, II, III, IV, V, VI, VII or VIII.
3. The process of embodiment 2, wherein the serotype is selected from Ia, Ib, II, III or V.
4. The process of any preceding embodiment, wherein the polysaccharide is a substantially full-length capsular polysaccharide.
5. The process of any preceding embodiment, wherein the polysaccharide has a molecular weight >30kDa.
6. The process of any preceding embodiment, wherein the saccharide is partially or fully de-O-acetylated.
7. The process of any preceding embodiment, wherein the saccharide is partially or fully de-N-acetylated.
8. The process of any preceding embodiment, wherein the suspension is the supernatant from a centrifuged *S.agalactiae* culture.
9. The process of any one of embodiments 1 to 7, wherein the suspension is prepared by treating *S.agalactiae* such that the capsular saccharide is released.
10. The process of embodiment 9, wherein the capsular saccharide is released by chemical or enzymatic treatment.
11. The process of embodiment 10, wherein the capsular saccharide is released by base extraction.
12. The process of embodiment 10, wherein the capsular saccharide is released by treatment with both mutanolysin and β-N-acetylglucosaminidase.
13. The process of embodiment 10, wherein the capsular saccharide is released by treatment with a type II phosphodiesterase.
14. The process of any preceding embodiment, wherein the alcohol is a lower alcohol.
15. The process of embodiment 14, wherein the alcohol is ethanol or isopropanol.
16. The process of any preceding embodiment, wherein the alcohol is added to the suspension to give a final alcohol concentration of between 10% and 50%.
17. The process of any preceding embodiment, wherein the aqueous metal cation is monovalent or divalent.
18. The process of embodiment 17, wherein the cation is Mg⁺⁺, Mn⁺⁺ or Ca⁺⁺.
19. The process of embodiment 18, where Ca⁺⁺ ions are used and are present at a final concentration of between 10 and 500 mM.
20. The process of any preceding embodiment, wherein step (b) includes centrifugation.
21. The process of embodiment 20, wherein the supernatant after centrifugation is subjected to microfiltration.
22. The process of any preceding embodiment, wherein a step of diafiltration is performed after step (a) and before step (c).
23. The process of any preceding embodiment, wherein the cationic step in step (c) is a tetrabutylammonium or cetyltrimethylammonium salt, such as CTAB.
24. The process of any preceding embodiment, wherein the process further comprises re-solubilising the saccharide into aqueous medium or into alcoholic medium.
25. The process of embodiment 24, wherein an aqueous medium is used for re-solubilising the saccharide, and wherein the aqueous medium includes Mg⁺⁺, Mn⁺⁺ or Ca⁺⁺.
26. The process of embodiment 24, wherein an alcoholic medium is used for re-solubilising the saccharide, and wherein the alcohol has a final concentration of between 70% and 95%.
27. A composition comprising a *Streptococcus agalactiae* capsular polysaccharide, obtainable by the process of any preceding embodiment.
28. The composition of embodiment 27, wherein the composition has UV absorbance at 280nm of less than 0.20.
29. The composition of embodiment 27, wherein the ratio of UV absorbance of the composition at 280nm to the UV absorbance at 260nm is greater than 0.85.
30. The composition of embodiment 27, wherein the UV absorbance spectrum of the composition between 220nm and 300nm does exhibit either a shoulder or peak at around 270nm.
31. The composition of embodiment 27, wherein the UV spectrum of the composition between 250nm and 275nm has neither a maximum point nor a point of inflexion.
32. The composition of embodiment 27, wherein the purity of the saccharide is at least 89% relative to the total weight of saccharide, protein and nucleic acid in the composition.

## Claims

1. A process for purifying a *Streptococcus agalactiae* capsular polysaccharide, comprising the step of removing contaminating nucleic acids and/or proteins by the use of precipitation.

2. The process of claim 1, wherein the process further comprises a step of diafiltration after the precipitation of nucleic acids and/or proteins.

3. The process of claim 2, wherein the process comprises the steps of: (a) removing contaminating nucleic acids and/or proteins from the capsular polysaccharide in aqueous form by the use of alcoholic precipitation, wherein an alcohol and an aqueous metal cation are used to precipitate the nucleic acids and/or proteins leaving the polysaccharide in solution; (b) separating the precipitated material from the polysaccharide; and (c) diafiltration to remove fragments of group specific saccharide from the polysaccharide.

4. The process of claim 1, wherein the process comprises a step of precipitating the saccharide using a cationic detergent.

5. The process of claim 4, wherein the process comprises the steps of: (a) treating a suspension comprising streptococcal proteins, nucleic acids and capsular polysaccharide with an aqueous metal cation and an alcohol in order to precipitate nucleic acids and proteins; (b) separating the precipitated material from the aqueous material; and (c) treating the aqueous material with a cationic detergent in order to precipitate the capsular polysaccharide.

6. The process of claim 2 or claim 3, wherein the diafiltration is tangential flow diafiltration.

7. The process of claim 6, wherein the tangential flow diafiltration is carried out using a filtration membrane with a cut-off in the range 10kDa-30kDa.

8. The process of claim 6 or claim 7, wherein at least five cycles of tangential flow diafiltration are performed.

9. The process of any preceding claim, wherein the polysaccharide is from a *S.agalactiae* serotype selected from Ia, Ib, II, III and V.

10. A composition comprising a *Streptococcus agalactiae* capsular polysaccharide, obtainable by the process of any preceding claim.

11. The composition of claim 10, wherein the composition has UV absorbance at 280nm of less than 0.20.

12. The composition of claim 10, wherein the ratio of UV absorbance of the composition at 280nm to the UV absorbance at 260nm is greater than 0.85.

13. The composition of claim 10, wherein the UV absorbance spectrum of the composition between 220nm and 300nm does exhibit either a shoulder or peak at around 270nm.

14. The composition of claim 10, wherein the UV spectrum of the composition between 250nm and 275nm has neither a maximum point nor a point of inflexion.

15. The composition of claim 10, wherein the purity of the saccharide is at least 89% relative to the total weight of saccharide, protein and nucleic acid in the composition.


SEQUENCE LISTING

<110>   NOVARTIS VACCINES AND DIAGNOSTICS SRL

<120>   PURIFICATION OF STREPTOCOCCAL CAPSULAR SACCHARIDE

<130>   P055516EP

<150>   GB-0502096.1
<151>   2005-02-01

<160>   19

<170>   SeqWin99, version 1.02

<210>   1
<211>   901
<212>   PRT
<213>   Streptococcus agalactiae

<400>   1
Met Arg Lys Tyr Gln Lys Phe Ser Lys Ile Leu Thr Leu Ser Leu Phe
1               5                   10                  15

Cys Leu Ser Gln Ile Pro Leu Asn Thr Asn Val Leu Gly Glu Ser Thr
            20                  25                  30

Val Pro Glu Asn Gly Ala Lys Gly Lys Leu Val Val Lys Lys Thr Asp
        35                  40                  45

Asp Gln Asn Lys Pro Leu Ser Lys Ala Thr Phe Val Leu Lys Thr Thr
    50                  55                  60

Ala His Pro Glu Ser Lys Ile Glu Lys Val Thr Ala Glu Leu Thr Gly
65                  70                  75                  80

Glu Ala Thr Phe Asp Asn Leu Ile Pro Gly Asp Tyr Thr Leu Ser Glu
                85                  90                  95

Glu Thr Ala Pro Glu Gly Tyr Lys Lys Thr Asn Gln Thr Trp Gln Val
            100                 105                 110

Lys Val Glu Ser Asn Gly Lys Thr Thr Ile Gln Asn Ser Gly Asp Lys
        115                 120                 125

Asn Ser Thr Ile Gly Gln Asn Gln Glu Glu Leu Asp Lys Gln Tyr Pro
    130                 135                 140

Pro Thr Gly Ile Tyr Glu Asp Thr Lys Glu Ser Tyr Lys Leu Glu His
145                 150                 155                 160

Val Lys Gly Ser Val Pro Asn Gly Lys Ser Glu Ala Lys Ala Val Asn
                165                 170                 175

Pro Tyr Ser Ser Glu Gly Glu His Ile Arg Glu Ile Pro Glu Gly Thr
            180                 185                 190

Leu Ser Lys Arg Ile Ser Glu Val Gly Asp Leu Ala His Asn Lys Tyr
        195                 200                 205

Lys Ile Glu Leu Thr Val Ser Gly Lys Thr Ile Val Lys Pro Val Asp

210                 215                 220

Lys Gln Lys Pro Leu Asp Val Val Phe Val Leu Asp Asn Ser Asn Ser
225                 230                 235                 240

Met Asn Asn Asp Gly Pro Asn Phe Gln Arg His Asn Lys Ala Lys Lys
                245                 250                 255

Ala Ala Glu Ala Leu Gly Thr Ala Val Lys Asp Ile Leu Gly Ala Asn
            260                 265                 270

Ser Asp Asn Arg Val Ala Leu Val Thr Tyr Gly Ser Asp Ile Phe Asp
        275                 280                 285

Gly Arg Ser Val Asp Val Val Lys Gly Phe Lys Glu Asp Asp Lys Tyr
    290                 295                 300

Tyr Gly Leu Gln Thr Lys Phe Thr Ile Gln Thr Glu Asn Tyr Ser His
305                 310                 315                 320

Lys Gln Leu Thr Asn Asn Ala Glu Glu Ile Ile Lys Arg Ile Pro Thr
                325                 330                 335

Glu Ala Pro Lys Ala Lys Trp Gly Ser Thr Thr Asn Gly Leu Thr Pro
            340                 345                 350

Glu Gln Gln Lys Glu Tyr Tyr Leu Ser Lys Val Gly Glu Thr Phe Thr
        355                 360                 365

Met Lys Ala Phe Met Glu Ala Asp Asp Ile Leu Ser Gln Val Asn Arg
    370                 375                 380

Asn Ser Gln Lys Ile Ile Val His Val Thr Asp Gly Val Pro Thr Arg
385                 390                 395                 400

Ser Tyr Ala Ile Asn Asn Phe Lys Leu Gly Ala Ser Tyr Glu Ser Gln
                405                 410                 415

Phe Glu Gln Met Lys Lys Asn Gly Tyr Leu Asn Lys Ser Asn Phe Leu
            420                 425                 430

Leu Thr Asp Lys Pro Glu Asp Ile Lys Gly Asn Gly Glu Ser Tyr Phe
        435                 440                 445

Leu Phe Pro Leu Asp Ser Tyr Gln Thr Gln Ile Ile Ser Gly Asn Leu
    450                 455                 460

Gln Lys Leu His Tyr Leu Asp Leu Asn Leu Asn Tyr Pro Lys Gly Thr
465                 470                 475                 480

Ile Tyr Arg Asn Gly Pro Val Lys Glu His Gly Thr Pro Thr Lys Leu
                485                 490                 495

Tyr Ile Asn Ser Leu Lys Gln Lys Asn Tyr Asp Ile Phe Asn Phe Gly
            500                 505                 510

Ile Asp Ile Ser Gly Phe Arg Gln Val Tyr Asn Glu Glu Tyr Lys Lys
        515                 520                 525

Asn Gln Asp Gly Thr Phe Gln Lys Leu Lys Glu Glu Ala Phe Lys Leu
    530                 535                 540

Ser Asp Gly Glu Ile Thr Glu Leu Met Arg Ser Phe Ser Ser Lys Pro
545                 550                 555                 560

Glu Tyr Tyr Thr Pro Ile Val Thr Ser Ala Asp Thr Ser Asn Asn Glu
                565                 570                 575

Ile Leu Ser Lys Ile Gln Gln Gln Phe Glu Thr Ile Leu Thr Lys Glu
            580                 585                 590

Asn Ser Ile Val Asn Gly Thr Ile Glu Asp Pro Met Gly Asp Lys Ile
        595                 600                 605

Asn Leu Gln Leu Gly Asn Gly Gln Thr Leu Gln Pro Ser Asp Tyr Thr
    610                 615                 620

Leu Gln Gly Asn Asp Gly Ser Val Met Lys Asp Gly Ile Ala Thr Gly
625                 630                 635                 640

Gly Pro Asn Asn Asp Gly Gly Ile Leu Lys Gly Val Lys Leu Glu Tyr
                645                 650                 655

Ile Gly Asn Lys Leu Tyr Val Arg Gly Leu Asn Leu Gly Glu Gly Gln
            660                 665                 670

Lys Val Thr Leu Thr Tyr Asp Val Lys Leu Asp Asp Ser Phe Ile Ser
        675                 680                 685

Asn Lys Phe Tyr Asp Thr Asn Gly Arg Thr Thr Leu Asn Pro Lys Ser
    690                 695                 700

Glu Asp Pro Asn Thr Leu Arg Asp Phe Pro Ile Pro Lys Ile Arg Asp
705                 710                 715                 720

Val Arg Glu Tyr Pro Thr Ile Thr Ile Lys Asn Glu Lys Lys Leu Gly
                725                 730                 735

Glu Ile Glu Phe Ile Lys Val Asp Lys Asp Asn Asn Lys Leu Leu Leu
            740                 745                 750

Lys Gly Ala Thr Phe Glu Leu Gln Glu Phe Asn Glu Asp Tyr Lys Leu
        755                 760                 765

Tyr Leu Pro Ile Lys Asn Asn Asn Ser Lys Val Val Thr Gly Glu Asn
    770                 775                 780

Gly Lys Ile Ser Tyr Lys Asp Leu Lys Asp Gly Lys Tyr Gln Leu Ile
785                 790                 795                 800

Glu Ala Val Ser Pro Glu Asp Tyr Gln Lys Ile Thr Asn Lys Pro Ile
                805                 810                 815

Leu Thr Phe Glu Val Val Lys Gly Ser Ile Lys Asn Ile Ile Ala Val
            820                 825                 830

Asn Lys Gln Ile Ser Glu Tyr His Glu Glu Gly Asp Lys His Leu Ile
        835                 840                 845

Thr Asn Thr His Ile Pro Pro Lys Gly Ile Ile Pro Met Thr Gly Gly
    850                 855                 860

Lys Gly Ile Leu Ser Phe Ile Leu Ile Gly Gly Ala Met Met Ser Ile
865                 870                 875                 880

Ala Gly Gly Ile Tyr Ile Trp Lys Arg Tyr Lys Lys Ser Ser Asp Met
                885                 890                 895

Ser Ile Lys Lys Asp
            900

<210>  2
<211>  554
<212>  PRT
<213>  Streptococcus agalactiae

<400>  2
Met Lys Leu Ser Lys Lys Leu Leu Phe Ser Ala Ala Val Leu Thr Met
1               5                   10                  15

Val Ala Gly Ser Thr Val Glu Pro Val Ala Gln Phe Ala Thr Gly Met
            20                  25                  30

Ser Ile Val Arg Ala Ala Glu Val Ser Gln Glu Arg Pro Ala Lys Thr
        35                  40                  45

Thr Val Asn Ile Tyr Lys Leu Gln Ala Asp Ser Tyr Lys Ser Glu Ile
    50                  55                  60

Thr Ser Asn Gly Gly Ile Glu Asn Lys Asp Gly Glu Val Ile Ser Asn
65                  70                  75                  80

Tyr Ala Lys Leu Gly Asp Asn Val Lys Gly Leu Gln Gly Val Gln Phe
                85                  90                  95

Lys Arg Tyr Lys Val Lys Thr Asp Ile Ser Val Asp Glu Leu Lys Lys
            100                 105                 110

Leu Thr Thr Val Glu Ala Ala Asp Ala Lys Val Gly Thr Ile Leu Glu
        115                 120                 125

Glu Gly Val Ser Leu Pro Gln Lys Thr Asn Ala Gln Gly Leu Val Val
    130                 135                 140

Asp Ala Leu Asp Ser Lys Ser Asn Val Arg Tyr Leu Tyr Val Glu Asp
145                 150                 155                 160

Leu Lys Asn Ser Pro Ser Asn Ile Thr Lys Ala Tyr Ala Val Pro Phe
                165                 170                 175

Val Leu Glu Leu Pro Val Ala Asn Ser Thr Gly Thr Gly Phe Leu Ser
            180                 185                 190

Glu Ile Asn Ile Tyr Pro Lys Asn Val Val Thr Asp Glu Pro Lys Thr
        195                 200                 205

Asp Lys Asp Val Lys Lys Leu Gly Gln Asp Asp Ala Gly Tyr Thr Ile
    210                 215                 220

Gly Glu Glu Phe Lys Trp Phe Leu Lys Ser Thr Ile Pro Ala Asn Leu
225                 230                 235                 240

Gly Asp Tyr Glu Lys Phe Glu Ile Thr Asp Lys Phe Ala Asp Gly Leu

245                 250                 255

Thr Tyr Lys Ser Val Gly Lys Ile Lys Ile Gly Ser Lys Thr Leu Asn
            260                 265                 270

Arg Asp Glu His Tyr Thr Ile Asp Glu Pro Thr Val Asp Asn Gln Asn
        275                 280                 285

Thr Leu Lys Ile Thr Phe Lys Pro Glu Lys Phe Lys Glu Ile Ala Glu
    290                 295                 300

Leu Leu Lys Gly Met Thr Leu Val Lys Asn Gln Asp Ala Leu Asp Lys
305                 310                 315                 320

Ala Thr Ala Asn Thr Asp Asp Ala Ala Phe Leu Glu Ile Pro Val Ala
                325                 330                 335

Ser Thr Ile Asn Glu Lys Ala Val Leu Gly Lys Ala Ile Glu Asn Thr
            340                 345                 350

Phe Glu Leu Gln Tyr Asp His Thr Pro Asp Lys Ala Asp Asn Pro Lys
        355                 360                 365

Pro Ser Asn Pro Pro Arg Lys Pro Glu Val His Thr Gly Gly Lys Arg
    370                 375                 380

Phe Val Lys Lys Asp Ser Thr Glu Thr Gln Thr Leu Gly Gly Ala Glu
385                 390                 395                 400

Phe Asp Leu Leu Ala Ser Asp Gly Thr Ala Val Lys Trp Thr Asp Ala
                405                 410                 415

Leu Ile Lys Ala Asn Thr Asn Lys Asn Tyr Ile Ala Gly Glu Ala Val
            420                 425                 430

Thr Gly Gln Pro Ile Lys Leu Lys Ser His Thr Asp Gly Thr Phe Glu
        435                 440                 445

Ile Lys Gly Leu Ala Tyr Ala Val Asp Ala Asn Ala Glu Gly Thr Ala
    450                 455                 460

Val Thr Tyr Lys Leu Lys Glu Thr Lys Ala Pro Glu Gly Tyr Val Ile
465                 470                 475                 480

Pro Asp Lys Glu Ile Glu Phe Thr Val Ser Gln Thr Ser Tyr Asn Thr
                485                 490                 495

Lys Pro Thr Asp Ile Thr Val Asp Ser Ala Asp Ala Thr Pro Asp Thr
            500                 505                 510

Ile Lys Asn Asn Lys Arg Pro Ser Ile Pro Asn Thr Gly Gly Ile Gly
        515                 520                 525

Thr Ala Ile Phe Val Ala Ile Gly Ala Ala Val Met Ala Phe Ala Val
    530                 535                 540

Lys Gly Met Lys Arg Arg Thr Lys Asp Asn
545                 550

<210>  3
<211>  890

<212>   PRT
<213>   Streptococcus agalactiae

<400>   3
Met Lys Lys Arg Gln Lys Ile Trp Arg Gly Leu Ser Val Thr Leu Leu
1               5                   10                  15

Ile Leu Ser Gln Ile Pro Phe Gly Ile Leu Val Gln Gly Glu Thr Gln
            20                  25                  30

Asp Thr Asn Gln Ala Leu Gly Lys Val Ile Val Lys Lys Thr Gly Asp
        35                  40                  45

Asn Ala Thr Pro Leu Gly Lys Ala Thr Phe Val Leu Lys Asn Asp Asn
    50                  55                  60

Asp Lys Ser Glu Thr Ser His Glu Thr Val Glu Gly Ser Gly Glu Ala
65                  70                  75                  80

Thr Phe Glu Asn Ile Lys Pro Gly Asp Tyr Thr Leu Arg Glu Glu Thr
                85                  90                  95

Ala Pro Ile Gly Tyr Lys Lys Thr Asp Lys Thr Trp Lys Val Lys Val
            100                 105                 110

Ala Asp Asn Gly Ala Thr Ile Ile Glu Gly Met Asp Ala Asp Lys Ala
        115                 120                 125

Glu Lys Arg Lys Glu Val Leu Asn Ala Gln Tyr Pro Lys Ser Ala Ile
    130                 135                 140

Tyr Glu Asp Thr Lys Glu Asn Tyr Pro Leu Val Asn Val Glu Gly Ser
145                 150                 155                 160

Lys Val Gly Glu Gln Tyr Lys Ala Leu Asn Pro Ile Asn Gly Lys Asp
                165                 170                 175

Gly Arg Arg Glu Ile Ala Glu Gly Trp Leu Ser Lys Lys Ile Thr Gly
            180                 185                 190

Val Asn Asp Leu Asp Lys Asn Lys Tyr Lys Ile Glu Leu Thr Val Glu
        195                 200                 205

Gly Lys Thr Thr Val Glu Thr Lys Glu Leu Asn Gln Pro Leu Asp Val
    210                 215                 220

Val Val Leu Leu Asp Asn Ser Asn Ser Met Asn Asn Glu Arg Ala Asn
225                 230                 235                 240

Asn Ser Gln Arg Ala Leu Lys Ala Gly Glu Ala Val Glu Lys Leu Ile
                245                 250                 255

Asp Lys Ile Thr Ser Asn Lys Asp Asn Arg Val Ala Leu Val Thr Tyr
            260                 265                 270

Ala Ser Thr Ile Phe Asp Gly Thr Glu Ala Thr Val Ser Lys Gly Val
        275                 280                 285

Ala Asp Gln Asn Gly Lys Ala Leu Asn Asp Ser Val Ser Trp Asp Tyr
    290                 295                 300

His Lys Thr Thr Phe Thr Ala Thr Thr His Asn Tyr Ser Tyr Leu Asn
305                 310                 315                 320

Leu Thr Asn Asp Ala Asn Glu Val Asn Ile Leu Lys Ser Arg Ile Pro
                325                 330                 335

Lys Glu Ala Glu His Ile Asn Gly Asp Arg Thr Leu Tyr Gln Phe Gly
            340                 345                 350

Ala Thr Phe Thr Gln Lys Ala Leu Met Lys Ala Asn Glu Ile Leu Glu
        355                 360                 365

Thr Gln Ser Ser Asn Ala Arg Lys Lys Leu Ile Phe His Val Thr Asp
    370                 375                 380

Gly Val Pro Thr Met Ser Tyr Ala Ile Asn Phe Asn Pro Tyr Ile Ser
385                 390                 395                 400

Thr Ser Tyr Gln Asn Gln Phe Asn Ser Phe Leu Asn Lys Ile Pro Asp
                405                 410                 415

Arg Ser Gly Ile Leu Gln Glu Asp Phe Ile Ile Asn Gly Asp Asp Tyr
            420                 425                 430

Gln Ile Val Lys Gly Asp Gly Glu Ser Phe Lys Leu Phe Ser Asp Arg
        435                 440                 445

Lys Val Pro Val Thr Gly Gly Thr Thr Gln Ala Ala Tyr Arg Val Pro
    450                 455                 460

Gln Asn Gln Leu Ser Val Met Ser Asn Glu Gly Tyr Ala Ile Asn Ser
465                 470                 475                 480

Gly Tyr Ile Tyr Leu Tyr Trp Arg Asp Tyr Asn Trp Val Tyr Pro Phe
                485                 490                 495

Asp Pro Lys Thr Lys Lys Val Ser Ala Thr Lys Gln Ile Lys Thr His
            500                 505                 510

Gly Glu Pro Thr Thr Leu Tyr Phe Asn Gly Asn Ile Arg Pro Lys Gly
        515                 520                 525

Tyr Asp Ile Phe Thr Val Gly Ile Gly Val Asn Gly Asp Pro Gly Ala
    530                 535                 540

Thr Pro Leu Glu Ala Glu Lys Phe Met Gln Ser Ile Ser Ser Lys Thr
545                 550                 555                 560

Glu Asn Tyr Thr Asn Val Asp Asp Thr Asn Lys Ile Tyr Asp Glu Leu
                565                 570                 575

Asn Lys Tyr Phe Lys Thr Ile Val Glu Glu Lys His Ser Ile Val Asp
            580                 585                 590

Gly Asn Val Thr Asp Pro Met Gly Glu Met Ile Glu Phe Gln Leu Lys
        595                 600                 605

Asn Gly Gln Ser Phe Thr His Asp Asp Tyr Val Leu Val Gly Asn Asp
    610                 615                 620

Gly Ser Gln Leu Lys Asn Gly Val Ala Leu Gly Gly Pro Asn Ser Asp

625                 630                 635                 640

Gly Gly Ile Leu Lys Asp Val Thr Val Thr Tyr Asp Lys Thr Ser Gln
                645                 650                 655

Thr Ile Lys Ile Asn His Leu Asn Leu Gly Ser Gly Gln Lys Val Val
            660                 665                 670

Leu Thr Tyr Asp Val Arg Leu Lys Asp Asn Tyr Ile Ser Asn Lys Phe
        675                 680                 685

Tyr Asn Thr Asn Asn Arg Thr Thr Leu Ser Pro Lys Ser Glu Lys Glu
    690                 695                 700

Pro Asn Thr Ile Arg Asp Phe Pro Ile Pro Lys Ile Arg Asp Val Arg
705                 710                 715                 720

Glu Phe Pro Val Leu Thr Ile Ser Asn Gln Lys Lys Met Gly Glu Val
                725                 730                 735

Glu Phe Ile Lys Val Asn Lys Asp Lys His Ser Glu Ser Leu Leu Gly
            740                 745                 750

Ala Lys Phe Gln Leu Gln Ile Glu Lys Asp Phe Ser Gly Tyr Lys Gln
        755                 760                 765

Phe Val Pro Glu Gly Ser Asp Val Thr Thr Lys Asn Asp Gly Lys Ile
    770                 775                 780

Tyr Phe Lys Ala Leu Gln Asp Gly Asn Tyr Lys Leu Tyr Glu Ile Ser
785                 790                 795                 800

Ser Pro Asp Gly Tyr Ile Glu Val Lys Thr Lys Pro Val Val Thr Phe
                805                 810                 815

Thr Ile Gln Asn Gly Glu Val Thr Asn Leu Lys Ala Asp Pro Asn Ala
            820                 825                 830

Asn Lys Asn Gln Ile Gly Tyr Leu Glu Gly Asn Gly Lys His Leu Ile
        835                 840                 845

Thr Asn Thr Pro Lys Arg Pro Pro Gly Val Phe Pro Lys Thr Gly Gly
    850                 855                 860

Ile Gly Thr Ile Val Tyr Ile Leu Val Gly Ser Thr Phe Met Ile Leu
865                 870                 875                 880

Thr Ile Cys Ser Phe Arg Arg Lys Gln Leu
                885                 890

<210>  4
<211>  1134
<212>  PRT
<213>  Streptococcus agalactiae

<400>  4
Met Arg Lys Lys Gln Lys Leu Pro Phe Asp Lys Leu Ala Ile Ala Leu
1               5                   10                  15

Ile Ser Thr Ser Ile Leu Leu Asn Ala Gln Ser Asp Ile Lys Ala Asn
            20                  25                  30

Thr Val Thr Glu Asp Thr Pro Ala Thr Glu Gln Ala Val Glu Pro Pro
        35                  40                  45

Gln Pro Ile Ala Val Ser Glu Glu Ser Arg Ser Ser Lys Glu Thr Lys
    50                  55                  60

Thr Ser Gln Thr Pro Ser Asp Val Gly Glu Thr Val Ala Asp Asp Ala
65                  70                  75                  80

Asn Asp Leu Ala Pro Gln Ala Pro Ala Lys Thr Ala Asp Thr Pro Ala
                85                  90                  95

Thr Ser Lys Ala Thr Ile Arg Asp Leu Asn Asp Pro Ser His Val Lys
            100                 105                 110

Thr Leu Gln Glu Lys Ala Gly Lys Gly Ala Gly Thr Val Val Ala Val
        115                 120                 125

Ile Asp Ala Gly Phe Asp Lys Asn His Glu Ala Trp Arg Leu Thr Asp
    130                 135                 140

Lys Thr Lys Ala Arg Tyr Gln Ser Lys Glu Asn Leu Glu Lys Ala Lys
145                 150                 155                 160

Lys Glu His Gly Ile Thr Tyr Gly Glu Trp Val Asn Asp Lys Val Ala
                165                 170                 175

Tyr Tyr His Asp Tyr Ser Lys Asp Gly Lys Asn Ala Val Asp Gln Glu
            180                 185                 190

His Gly Thr His Val Ser Gly Ile Leu Ser Gly Asn Ala Pro Ser Glu
        195                 200                 205

Met Lys Glu Pro Tyr Arg Leu Glu Gly Ala Met Pro Glu Ala Gln Leu
    210                 215                 220

Leu Leu Met Arg Val Glu Ile Val Asn Gly Leu Ala Asp Tyr Ala Arg
225                 230                 235                 240

Asn Tyr Ala Gln Ala Ile Arg Asp Ala Val Asn Leu Gly Ala Lys Val
                245                 250                 255

Ile Asn Met Ser Phe Gly Asn Ala Ala Leu Ala Tyr Ala Asn Leu Pro
            260                 265                 270

Asp Glu Thr Lys Lys Ala Phe Asp Tyr Ala Lys Ser Lys Gly Val Ser
        275                 280                 285

Ile Val Thr Ser Ala Gly Asn Asp Ser Ser Phe Gly Gly Lys Pro Arg
    290                 295                 300

Leu Pro Leu Ala Asp His Pro Asp Tyr Gly Val Val Gly Thr Pro Ala
305                 310                 315                 320

Ala Ala Asp Ser Thr Leu Thr Val Ala Ser Tyr Ser Pro Asp Lys Gln
                325                 330                 335

Leu Thr Glu Thr Ala Thr Val Lys Thr Asp Asp His Gln Asp Lys Glu
            340                 345                 350

Met Pro Val Ile Ser Thr Asn Arg Phe Glu Pro Asn Lys Ala Tyr Asp
        355                 360                 365

Tyr Ala Tyr Ala Asn Arg Gly Thr Lys Glu Asp Asp Phe Lys Asp Val
    370                 375                 380

Glu Gly Lys Ile Ala Leu Ile Glu Arg Gly Asp Ile Asp Phe Lys Asp
385                 390                 395                 400

Lys Ile Ala Asn Ala Lys Lys Ala Gly Ala Val Gly Val Leu Ile Tyr
                405                 410                 415

Asp Asn Gln Asp Lys Gly Phe Pro Ile Glu Leu Pro Asn Val Asp Gln
            420                 425                 430

Met Pro Ala Ala Phe Ile Ser Arg Arg Asp Gly Leu Leu Leu Lys Asp
        435                 440                 445

Asn Pro Pro Lys Thr Ile Thr Phe Asn Ala Thr Pro Lys Val Leu Pro
    450                 455                 460

Thr Ala Ser Gly Thr Lys Leu Ser Arg Phe Ser Ser Trp Gly Leu Thr
465                 470                 475                 480

Ala Asp Gly Asn Ile Lys Pro Asp Ile Ala Ala Pro Gly Gln Asp Ile
                485                 490                 495

Leu Ser Ser Val Ala Asn Asn Lys Tyr Ala Lys Leu Ser Gly Thr Ser
            500                 505                 510

Met Ser Ala Pro Leu Val Ala Gly Ile Met Gly Leu Leu Gln Lys Gln
        515                 520                 525

Tyr Glu Thr Gln Tyr Pro Asp Met Thr Pro Ser Glu Arg Leu Asp Leu
    530                 535                 540

Ala Lys Lys Val Leu Met Ser Ser Ala Thr Ala Leu Tyr Asp Glu Asp
545                 550                 555                 560

Glu Lys Ala Tyr Phe Ser Pro Arg Gln Gln Gly Ala Gly Ala Val Asp
                565                 570                 575

Ala Lys Lys Ala Ser Ala Ala Thr Met Tyr Val Thr Asp Lys Asp Asn
            580                 585                 590

Thr Ser Ser Lys Val His Leu Asn Asn Val Ser Asp Lys Phe Glu Val
        595                 600                 605

Thr Val Thr Val His Asn Lys Ser Asp Lys Pro Gln Glu Leu Tyr Tyr
    610                 615                 620

Gln Val Thr Val Gln Thr Asp Lys Val Asp Gly Lys His Phe Ala Leu
625                 630                 635                 640

Ala Pro Lys Ala Leu Tyr Glu Thr Ser Trp Gln Lys Ile Thr Ile Pro
                645                 650                 655

Ala Asn Ser Ser Lys Gln Val Thr Val Pro Ile Asp Ala Ser Arg Phe
            660                 665                 670

Ser Lys Asp Leu Leu Ala Gln Met Lys Asn Gly Tyr Phe Leu Glu Gly

675                 680                 685

Phe Val Arg Phe Lys Gln Asp Pro Thr Lys Glu Glu Leu Met Ser Ile
    690                 695                 700

Pro Tyr Ile Gly Phe Arg Gly Asp Phe Gly Asn Leu Ser Ala Leu Glu
705                 710                 715                 720

Lys Pro Ile Tyr Asp Ser Lys Asp Gly Ser Ser Tyr Tyr His Glu Ala
                725                 730                 735

Asn Ser Asp Ala Lys Asp Gln Leu Asp Gly Asp Gly Leu Gln Phe Tyr
            740                 745                 750

Ala Leu Lys Asn Asn Phe Thr Ala Leu Thr Thr Glu Ser Asn Pro Trp
        755                 760                 765

Thr Ile Ile Lys Ala Val Lys Glu Gly Val Glu Asn Ile Glu Asp Ile
    770                 775                 780

Glu Ser Ser Glu Ile Thr Glu Thr Ile Phe Ala Gly Thr Phe Ala Lys
785                 790                 795                 800

Gln Asp Asp Asp Ser His Tyr Tyr Ile His Arg His Ala Asn Gly Lys
                805                 810                 815

Pro Tyr Ala Ala Ile Ser Pro Asn Gly Asp Gly Asn Arg Asp Tyr Val
            820                 825                 830

Gln Phe Gln Gly Thr Phe Leu Arg Asn Ala Lys Asn Leu Val Ala Glu
        835                 840                 845

Val Leu Asp Lys Glu Gly Asn Val Val Trp Thr Ser Glu Val Thr Glu
    850                 855                 860

Gln Val Val Lys Asn Tyr Asn Asn Asp Leu Ala Ser Thr Leu Gly Ser
865                 870                 875                 880

Thr Arg Phe Glu Lys Thr Arg Trp Asp Gly Lys Asp Lys Asp Gly Lys
                885                 890                 895

Val Val Ala Asn Gly Thr Tyr Thr Tyr Arg Val Arg Tyr Thr Pro Ile
            900                 905                 910

Ser Ser Gly Ala Lys Glu Gln His Thr Asp Phe Asp Val Ile Val Asp
        915                 920                 925

Asn Thr Thr Pro Glu Val Ala Thr Ser Ala Thr Phe Ser Thr Glu Asp
    930                 935                 940

Ser Arg Leu Thr Leu Ala Ser Lys Pro Lys Thr Ser Gln Pro Val Tyr
945                 950                 955                 960

Arg Glu Arg Ile Ala Tyr Thr Tyr Met Asp Glu Asp Leu Pro Thr Thr
                965                 970                 975

Glu Tyr Ile Ser Pro Asn Glu Asp Gly Thr Phe Thr Leu Pro Glu Glu
            980                 985                 990

Ala Glu Thr Met Glu Gly Ala Thr Val Pro Leu Lys Met Ser Asp Phe
        995                 1000                1005

Thr Tyr Val Val Glu Asp Met Ala Gly Asn Ile Thr Tyr Thr Pro Val
    1010                1015                1020

Thr Lys Leu Leu Glu Gly His Ser Asn Lys Pro Glu Gln Asp Gly Ser
1025                1030                1035                1040

Asp Gln Ala Pro Asp Lys Lys Pro Glu Ala Lys Pro Glu Gln Asp Gly
                1045                1050                1055

Ser Gly Gln Thr Pro Asp Lys Lys Lys Glu Thr Lys Pro Glu Lys Asp
            1060                1065                1070

Ser Ser Gly Gln Thr Pro Gly Lys Thr Pro Gln Lys Gly Gln Ser Ser
        1075                1080                1085

Arg Thr Leu Glu Lys Arg Ser Ser Lys Arg Ala Leu Ala Thr Lys Ala
    1090                1095                1100

Ser Thr Arg Asp Gln Leu Pro Thr Thr Asn Asp Lys Asp Thr Asn Arg
1105                1110                1115                1120

Leu His Leu Leu Lys Leu Val Met Thr Thr Phe Phe Leu Gly
                1125                1130

<210>   5
<211>   432
<212>   PRT
<213>   Streptococcus agalactiae

<400>   5
Met Asn Lys Lys Val Leu Leu Thr Ser Thr Met Ala Ala Ser Leu Leu
1               5                   10                  15

Ser Val Ala Ser Val Gln Ala Gln Glu Thr Asp Thr Thr Trp Thr Ala
            20                  25                  30

Arg Thr Val Ser Glu Val Lys Ala Asp Leu Val Lys Gln Asp Asn Lys
        35                  40                  45

Ser Ser Tyr Thr Val Lys Tyr Gly Asp Thr Leu Ser Val Ile Ser Glu
    50                  55                  60

Ala Met Ser Ile Asp Met Asn Val Leu Ala Lys Ile Asn Asn Ile Ala
65                  70                  75                  80

Asp Ile Asn Leu Ile Tyr Pro Glu Thr Thr Leu Thr Val Thr Tyr Asp
                85                  90                  95

Gln Lys Ser His Thr Ala Thr Ser Met Lys Ile Glu Thr Pro Ala Thr
            100                 105                 110

Asn Ala Ala Gly Gln Thr Thr Ala Thr Val Asp Leu Lys Thr Asn Gln
        115                 120                 125

Val Ser Val Ala Asp Gln Lys Val Ser Leu Asn Thr Ile Ser Glu Gly
    130                 135                 140

Met Thr Pro Glu Ala Ala Thr Thr Ile Val Ser Pro Met Lys Thr Tyr
145                 150                 155                 160

Ser Ser Ala Pro Ala Leu Lys Ser Lys Glu Val Leu Ala Gln Glu Gln
                165                 170                 175

Ala Val Ser Gln Ala Ala Ala Asn Glu Gln Val Ser Pro Ala Pro Val
            180                 185                 190

Lys Ser Ile Thr Ser Glu Val Pro Ala Ala Lys Glu Glu Val Lys Pro
        195                 200                 205

Thr Gln Thr Ser Val Ser Gln Ser Thr Thr Val Ser Pro Ala Ser Val
    210                 215                 220

Ala Ala Glu Thr Pro Ala Pro Val Ala Lys Val Ala Pro Val Arg Thr
225                 230                 235                 240

Val Ala Ala Pro Arg Val Ala Ser Val Lys Val Val Thr Pro Lys Val
                245                 250                 255

Glu Thr Gly Ala Ser Pro Glu His Val Ser Ala Pro Ala Val Pro Val
            260                 265                 270

Thr Thr Thr Ser Pro Ala Thr Asp Ser Lys Leu Gln Ala Thr Glu Val
        275                 280                 285

Lys Ser Val Pro Val Ala Gln Lys Ala Pro Thr Ala Thr Pro Val Ala
    290                 295                 300

Gln Pro Ala Ser Thr Thr Asn Ala Val Ala Ala His Pro Glu Asn Ala
305                 310                 315                 320

Gly Leu Gln Pro His Val Ala Ala Tyr Lys Glu Lys Val Ala Ser Thr
                325                 330                 335

Tyr Gly Val Asn Glu Phe Ser Thr Tyr Arg Ala Gly Asp Pro Gly Asp
            340                 345                 350

His Gly Lys Gly Leu Ala Val Asp Phe Ile Val Gly Thr Asn Gln Ala
        355                 360                 365

Leu Gly Asn Lys Val Ala Gln Tyr Ser Thr Gln Asn Met Ala Ala Asn
    370                 375                 380

Asn Ile Ser Tyr Val Ile Trp Gln Gln Lys Phe Tyr Ser Asn Thr Asn
385                 390                 395                 400

Ser Ile Tyr Gly Pro Ala Asn Thr Trp Asn Ala Met Pro Asp Arg Gly
                405                 410                 415

Gly Val Thr Ala Asn His Tyr Asp His Val His Val Ser Phe Asn Lys
            420                 425                 430


<210>   6
<211>   517
<212>   PRT
<213>   Streptococcus agalactiae

<400>   6
Ala Glu Val Ser Gln Glu Arg Pro Ala Lys Thr Thr Val Asn Ile Tyr
1               5                   10                  15

Lys Leu Gln Ala Asp Ser Tyr Lys Ser Glu Ile Thr Ser Asn Gly Gly
            20                  25                  30

Ile Glu Asn Lys Asp Gly Glu Val Ile Ser Asn Tyr Ala Lys Leu Gly
        35                  40                  45

Asp Asn Val Lys Gly Leu Gln Gly Val Gln Phe Lys Arg Tyr Lys Val
    50                  55                  60

Lys Thr Asp Ile Ser Val Asp Glu Leu Lys Lys Leu Thr Thr Val Glu
65                  70                  75                  80

Ala Ala Asp Ala Lys Val Gly Thr Ile Leu Glu Glu Gly Val Ser Leu
                85                  90                  95

Pro Gln Lys Thr Asn Ala Gln Gly Leu Val Val Asp Ala Leu Asp Ser
            100                 105                 110

Lys Ser Asn Val Arg Tyr Leu Tyr Val Glu Asp Leu Lys Asn Ser Pro
        115                 120                 125

Ser Asn Ile Thr Lys Ala Tyr Ala Val Pro Phe Val Leu Glu Leu Pro
    130                 135                 140

Val Ala Asn Ser Thr Gly Thr Gly Phe Leu Ser Glu Ile Asn Ile Tyr
145                 150                 155                 160

Pro Lys Asn Val Val Thr Asp Glu Pro Lys Thr Asp Lys Asp Val Lys
                165                 170                 175

Lys Leu Gly Gln Asp Asp Ala Gly Tyr Thr Ile Gly Glu Glu Phe Lys
            180                 185                 190

Trp Phe Leu Lys Ser Thr Ile Pro Ala Asn Leu Gly Asp Tyr Glu Lys
        195                 200                 205

Phe Glu Ile Thr Asp Lys Phe Ala Asp Gly Leu Thr Tyr Lys Ser Val
    210                 215                 220

Gly Lys Ile Lys Ile Gly Ser Lys Thr Leu Asn Arg Asp Glu His Tyr
225                 230                 235                 240

Thr Ile Asp Glu Pro Thr Val Asp Asn Gln Asn Thr Leu Lys Ile Thr
                245                 250                 255

Phe Lys Pro Glu Lys Phe Lys Glu Ile Ala Glu Leu Leu Lys Gly Met
            260                 265                 270

Thr Leu Val Lys Asn Gln Asp Ala Leu Asp Lys Ala Thr Ala Asn Thr
        275                 280                 285

Asp Asp Ala Ala Phe Leu Glu Ile Pro Val Ala Ser Thr Ile Asn Glu
    290                 295                 300

Lys Ala Val Leu Gly Lys Ala Ile Glu Asn Thr Phe Glu Leu Gln Tyr
305                 310                 315                 320

Asp His Thr Pro Asp Lys Ala Asp Asn Pro Lys Pro Ser Asn Pro Pro
                325                 330                 335

Arg Lys Pro Glu Val His Thr Gly Gly Lys Arg Phe Val Lys Lys Asp

            340                 345                 350

Ser Thr Glu Thr Gln Thr Leu Gly Gly Ala Glu Phe Asp Leu Leu Ala
        355                 360                 365

Ser Asp Gly Thr Ala Val Lys Trp Thr Asp Ala Leu Ile Lys Ala Asn
    370                 375                 380

Thr Asn Lys Asn Tyr Ile Ala Gly Glu Ala Val Thr Gly Gln Pro Ile
385                 390                 395                 400

Lys Leu Lys Ser His Thr Asp Gly Thr Phe Glu Ile Lys Gly Leu Ala
                405                 410                 415

Tyr Ala Val Asp Ala Asn Ala Glu Gly Thr Ala Val Thr Tyr Lys Leu
            420                 425                 430

Lys Glu Thr Lys Ala Pro Glu Gly Tyr Val Ile Pro Asp Lys Glu Ile
        435                 440                 445

Glu Phe Thr Val Ser Gln Thr Ser Tyr Asn Thr Lys Pro Thr Asp Ile
    450                 455                 460

Thr Val Asp Ser Ala Asp Ala Thr Pro Asp Thr Ile Lys Asn Asn Lys
465                 470                 475                 480

Arg Pro Ser Ile Pro Asn Thr Gly Gly Ile Gly Thr Ala Ile Phe Val
                485                 490                 495

Ala Ile Gly Ala Ala Val Met Ala Phe Ala Val Lys Gly Met Lys Arg
            500                 505                 510

Arg Thr Lys Asp Asn
        515

<210> 7
<211> 525
<212> PRT
<213> Streptococcus agalactiae

<400> 7
Met Lys Leu Ser Lys Lys Leu Leu Phe Ser Ala Ala Val Leu Thr Met
1               5                   10                  15

Val Ala Gly Ser Thr Val Glu Pro Val Ala Gln Phe Ala Thr Gly Met
            20                  25                  30

Ser Ile Val Arg Ala Ala Glu Val Ser Gln Glu Arg Pro Ala Lys Thr
        35                  40                  45

Thr Val Asn Ile Tyr Lys Leu Gln Ala Asp Ser Tyr Lys Ser Glu Ile
    50                  55                  60

Thr Ser Asn Gly Gly Ile Glu Asn Lys Asp Gly Glu Val Ile Ser Asn
65                  70                  75                  80

Tyr Ala Lys Leu Gly Asp Asn Val Lys Gly Leu Gln Gly Val Gln Phe
                85                  90                  95

Lys Arg Tyr Lys Val Lys Thr Asp Ile Ser Val Asp Glu Leu Lys Lys
            100                 105                 110

Leu Thr Thr Val Glu Ala Ala Asp Ala Lys Val Gly Thr Ile Leu Glu
        115                 120                 125

Glu Gly Val Ser Leu Pro Gln Lys Thr Asn Ala Gln Gly Leu Val Val
    130                 135                 140

Asp Ala Leu Asp Ser Lys Ser Asn Val Arg Tyr Leu Tyr Val Glu Asp
145                 150                 155                 160

Leu Lys Asn Ser Pro Ser Asn Ile Thr Lys Ala Tyr Ala Val Pro Phe
                165                 170                 175

Val Leu Glu Leu Pro Val Ala Asn Ser Thr Gly Thr Gly Phe Leu Ser
            180                 185                 190

Glu Ile Asn Ile Tyr Pro Lys Asn Val Val Thr Asp Glu Pro Lys Thr
        195                 200                 205

Asp Lys Asp Val Lys Lys Leu Gly Gln Asp Asp Ala Gly Tyr Thr Ile
    210                 215                 220

Gly Glu Glu Phe Lys Trp Phe Leu Lys Ser Thr Ile Pro Ala Asn Leu
225                 230                 235                 240

Gly Asp Tyr Glu Lys Phe Glu Ile Thr Asp Lys Phe Ala Asp Gly Leu
                245                 250                 255

Thr Tyr Lys Ser Val Gly Lys Ile Lys Ile Gly Ser Lys Thr Leu Asn
            260                 265                 270

Arg Asp Glu His Tyr Thr Ile Asp Glu Pro Thr Val Asp Asn Gln Asn
        275                 280                 285

Thr Leu Lys Ile Thr Phe Lys Pro Glu Lys Phe Lys Glu Ile Ala Glu
    290                 295                 300

Leu Leu Lys Gly Met Thr Leu Val Lys Asn Gln Asp Ala Leu Asp Lys
305                 310                 315                 320

Ala Thr Ala Asn Thr Asp Asp Ala Ala Phe Leu Glu Ile Pro Val Ala
                325                 330                 335

Ser Thr Ile Asn Glu Lys Ala Val Leu Gly Lys Ala Ile Glu Asn Thr
            340                 345                 350

Phe Glu Leu Gln Tyr Asp His Thr Pro Asp Lys Ala Asp Asn Pro Lys
        355                 360                 365

Pro Ser Asn Pro Pro Arg Lys Pro Glu Val His Thr Gly Gly Lys Arg
    370                 375                 380

Phe Val Lys Lys Asp Ser Thr Glu Thr Gln Thr Leu Gly Gly Ala Glu
385                 390                 395                 400

Phe Asp Leu Leu Ala Ser Asp Gly Thr Ala Val Lys Trp Thr Asp Ala
                405                 410                 415

Leu Ile Lys Ala Asn Thr Asn Lys Asn Tyr Ile Ala Gly Glu Ala Val
            420                 425                 430

Thr Gly Gln Pro Ile Lys Leu Lys Ser His Thr Asp Gly Thr Phe Glu
        435                 440                 445

Ile Lys Gly Leu Ala Tyr Ala Val Asp Ala Asn Ala Glu Gly Thr Ala
    450                 455                 460

Val Thr Tyr Lys Leu Lys Glu Thr Lys Ala Pro Glu Gly Tyr Val Ile
465                 470                 475                 480

Pro Asp Lys Glu Ile Glu Phe Thr Val Ser Gln Thr Ser Tyr Asn Thr
                485                 490                 495

Lys Pro Thr Asp Ile Thr Val Asp Ser Ala Asp Ala Thr Pro Asp Thr
            500                 505                 510

Ile Lys Asn Asn Lys Arg Pro Ser Ile Pro Asn Thr Gly
        515                 520                 525

<210>   8
<211>   520
<212>   PRT
<213>   Streptococcus agalactiae

<400>   8
Met Lys Leu Ser Lys Lys Leu Leu Phe Ser Ala Ala Val Leu Thr Met
1               5                   10                  15

Val Ala Gly Ser Thr Val Glu Pro Val Ala Gln Phe Ala Thr Gly Met
            20                  25                  30

Ser Ile Val Arg Ala Ala Glu Val Ser Gln Glu Arg Pro Ala Lys Thr
        35                  40                  45

Thr Val Asn Ile Tyr Lys Leu Gln Ala Asp Ser Tyr Lys Ser Glu Ile
    50                  55                  60

Thr Ser Asn Gly Gly Ile Glu Asn Lys Asp Gly Glu Val Ile Ser Asn
65                  70                  75                  80

Tyr Ala Lys Leu Gly Asp Asn Val Lys Gly Leu Gln Gly Val Gln Phe
                85                  90                  95

Lys Arg Tyr Lys Val Lys Thr Asp Ile Ser Val Asp Glu Leu Lys Lys
            100                 105                 110

Leu Thr Thr Val Glu Ala Ala Asp Ala Lys Val Gly Thr Ile Leu Glu
        115                 120                 125

Glu Gly Val Ser Leu Pro Gln Lys Thr Asn Ala Gln Gly Leu Val Val
    130                 135                 140

Asp Ala Leu Asp Ser Lys Ser Asn Val Arg Tyr Leu Tyr Val Glu Asp
145                 150                 155                 160

Leu Lys Asn Ser Pro Ser Asn Ile Thr Lys Ala Tyr Ala Val Pro Phe
                165                 170                 175

Val Leu Glu Leu Pro Val Ala Asn Ser Thr Gly Thr Gly Phe Leu Ser
            180                 185                 190

Glu Ile Asn Ile Tyr Pro Lys Asn Val Val Thr Asp Glu Pro Lys Thr

195                 200                 205

Asp Lys Asp Val Lys Lys Leu Gly Gln Asp Asp Ala Gly Tyr Thr Ile
    210                 215                 220

Gly Glu Glu Phe Lys Trp Phe Leu Lys Ser Thr Ile Pro Ala Asn Leu
225                 230                 235                 240

Gly Asp Tyr Glu Lys Phe Glu Ile Thr Asp Lys Phe Ala Asp Gly Leu
                245                 250                 255

Thr Tyr Lys Ser Val Gly Lys Ile Lys Ile Gly Ser Lys Thr Leu Asn
            260                 265                 270

Arg Asp Glu His Tyr Thr Ile Asp Glu Pro Thr Val Asp Asn Gln Asn
        275                 280                 285

Thr Leu Lys Ile Thr Phe Lys Pro Glu Lys Phe Lys Glu Ile Ala Glu
    290                 295                 300

Leu Leu Lys Gly Met Thr Leu Val Lys Asn Gln Asp Ala Leu Asp Lys
305                 310                 315                 320

Ala Thr Ala Asn Thr Asp Asp Ala Ala Phe Leu Glu Ile Pro Val Ala
                325                 330                 335

Ser Thr Ile Asn Glu Lys Ala Val Leu Gly Lys Ala Ile Glu Asn Thr
            340                 345                 350

Phe Glu Leu Gln Tyr Asp His Thr Pro Asp Lys Ala Asp Asn Pro Lys
        355                 360                 365

Pro Ser Asn Pro Pro Arg Lys Pro Glu Val His Thr Gly Gly Lys Arg
    370                 375                 380

Phe Val Lys Lys Asp Ser Thr Glu Thr Gln Thr Leu Gly Gly Ala Glu
385                 390                 395                 400

Phe Asp Leu Leu Ala Ser Asp Gly Thr Ala Val Lys Trp Thr Asp Ala
                405                 410                 415

Leu Ile Lys Ala Asn Thr Asn Lys Asn Tyr Ile Ala Gly Glu Ala Val
            420                 425                 430

Thr Gly Gln Pro Ile Lys Leu Lys Ser His Thr Asp Gly Thr Phe Glu
        435                 440                 445

Ile Lys Gly Leu Ala Tyr Ala Val Asp Ala Asn Ala Glu Gly Thr Ala
    450                 455                 460

Val Thr Tyr Lys Leu Lys Glu Thr Lys Ala Pro Glu Gly Tyr Val Ile
465                 470                 475                 480

Pro Asp Lys Glu Ile Glu Phe Thr Val Ser Gln Thr Ser Tyr Asn Thr
                485                 490                 495

Lys Pro Thr Asp Ile Thr Val Asp Ser Ala Asp Ala Thr Pro Asp Thr
            500                 505                 510

Ile Lys Asn Asn Lys Arg Pro Ser
        515                 520

<210>   9
<211>   483
<212>   PRT
<213>   Streptococcus agalactiae

<400>   9
Ala Glu Val Ser Gln Glu Arg Pro Ala Lys Thr Thr Val Asn Ile Tyr
1               5                   10                  15

Lys Leu Gln Ala Asp Ser Tyr Lys Ser Glu Ile Thr Ser Asn Gly Gly
            20                  25                  30

Ile Glu Asn Lys Asp Gly Glu Val Ile Ser Asn Tyr Ala Lys Leu Gly
        35                  40                  45

Asp Asn Val Lys Gly Leu Gln Gly Val Gln Phe Lys Arg Tyr Lys Val
    50                  55                  60

Lys Thr Asp Ile Ser Val Asp Glu Leu Lys Lys Leu Thr Thr Val Glu
65                  70                  75                  80

Ala Ala Asp Ala Lys Val Gly Thr Ile Leu Glu Glu Gly Val Ser Leu
                85                  90                  95

Pro Gln Lys Thr Asn Ala Gln Gly Leu Val Val Asp Ala Leu Asp Ser
            100                 105                 110

Lys Ser Asn Val Arg Tyr Leu Tyr Val Glu Asp Leu Lys Asn Ser Pro
        115                 120                 125

Ser Asn Ile Thr Lys Ala Tyr Ala Val Pro Phe Val Leu Glu Leu Pro
    130                 135                 140

Val Ala Asn Ser Thr Gly Thr Gly Phe Leu Ser Glu Ile Asn Ile Tyr
145                 150                 155                 160

Pro Lys Asn Val Val Thr Asp Glu Pro Lys Thr Asp Lys Asp Val Lys
                165                 170                 175

Lys Leu Gly Gln Asp Asp Ala Gly Tyr Thr Ile Gly Glu Glu Phe Lys
            180                 185                 190

Trp Phe Leu Lys Ser Thr Ile Pro Ala Asn Leu Gly Asp Tyr Glu Lys
        195                 200                 205

Phe Glu Ile Thr Asp Lys Phe Ala Asp Gly Leu Thr Tyr Lys Ser Val
    210                 215                 220

Gly Lys Ile Lys Ile Gly Ser Lys Thr Leu Asn Arg Asp Glu His Tyr
225                 230                 235                 240

Thr Ile Asp Glu Pro Thr Val Asp Asn Gln Asn Thr Leu Lys Ile Thr
                245                 250                 255

Phe Lys Pro Glu Lys Phe Lys Glu Ile Ala Glu Leu Leu Lys Gly Met
            260                 265                 270

Thr Leu Val Lys Asn Gln Asp Ala Leu Asp Lys Ala Thr Ala Asn Thr
        275                 280                 285

Asp Asp Ala Ala Phe Leu Glu Ile Pro Val Ala Ser Thr Ile Asn Glu
    290                 295                 300

Lys Ala Val Leu Gly Lys Ala Ile Glu Asn Thr Phe Glu Leu Gln Tyr
305                 310                 315                 320

Asp His Thr Pro Asp Lys Ala Asp Asn Pro Lys Pro Ser Asn Pro Pro
                325                 330                 335

Arg Lys Pro Glu Val His Thr Gly Gly Lys Arg Phe Val Lys Lys Asp
            340                 345                 350

Ser Thr Glu Thr Gln Thr Leu Gly Gly Ala Glu Phe Asp Leu Leu Ala
        355                 360                 365

Ser Asp Gly Thr Ala Val Lys Trp Thr Asp Ala Leu Ile Lys Ala Asn
    370                 375                 380

Thr Asn Lys Asn Tyr Ile Ala Gly Glu Ala Val Thr Gly Gln Pro Ile
385                 390                 395                 400

Lys Leu Lys Ser His Thr Asp Gly Thr Phe Glu Ile Lys Gly Leu Ala
                405                 410                 415

Tyr Ala Val Asp Ala Asn Ala Glu Gly Thr Ala Val Thr Tyr Lys Leu
            420                 425                 430

Lys Glu Thr Lys Ala Pro Glu Gly Tyr Val Ile Pro Asp Lys Glu Ile
        435                 440                 445

Glu Phe Thr Val Ser Gln Thr Ser Tyr Asn Thr Lys Pro Thr Asp Ile
    450                 455                 460

Thr Val Asp Ser Ala Asp Ala Thr Pro Asp Thr Ile Lys Asn Asn Lys
465                 470                 475                 480

Arg Pro Ser


<210>   10
<211>   271
<212>   PRT
<213>   Streptococcus agalactiae

<400>   10
Ala Glu Val Ser Gln Glu Arg Pro Ala Lys Thr Thr Val Asn Ile Tyr
1               5                   10                  15

Lys Leu Gln Ala Asp Ser Tyr Lys Ser Glu Ile Thr Ser Asn Gly Gly
            20                  25                  30

Ile Glu Asn Lys Asp Gly Glu Val Ile Ser Asn Tyr Ala Lys Leu Gly
        35                  40                  45

Asp Asn Val Lys Gly Leu Gln Gly Val Gln Phe Lys Arg Tyr Lys Val
    50                  55                  60

Lys Thr Asp Ile Ser Val Asp Glu Leu Lys Lys Leu Thr Thr Val Glu
65                  70                  75                  80

Ala Ala Asp Ala Lys Val Gly Thr Ile Leu Glu Glu Gly Val Ser Leu

85                  90                  95

Pro Gln Lys Thr Asn Ala Gln Gly Leu Val Val Asp Ala Leu Asp Ser
            100                 105                 110

Lys Ser Asn Val Arg Tyr Leu Tyr Val Glu Asp Leu Lys Asn Ser Pro
        115                 120                 125

Ser Asn Ile Thr Lys Ala Tyr Ala Val Pro Phe Val Leu Glu Leu Pro
    130                 135                 140

Val Ala Asn Ser Thr Gly Thr Gly Phe Leu Ser Glu Ile Asn Ile Tyr
145                 150                 155                 160

Pro Lys Asn Val Val Thr Asp Glu Pro Lys Thr Asp Lys Asp Val Lys
                165                 170                 175

Lys Leu Gly Gln Asp Asp Ala Gly Tyr Thr Ile Gly Glu Glu Phe Lys
            180                 185                 190

Trp Phe Leu Lys Ser Thr Ile Pro Ala Asn Leu Gly Asp Tyr Glu Lys
        195                 200                 205

Phe Glu Ile Thr Asp Lys Phe Ala Asp Gly Leu Thr Tyr Lys Ser Val
    210                 215                 220

Gly Lys Ile Lys Ile Gly Ser Lys Thr Leu Asn Arg Asp Glu His Tyr
225                 230                 235                 240

Thr Ile Asp Glu Pro Thr Val Asp Asn Gln Asn Thr Leu Lys Ile Thr
                245                 250                 255

Phe Lys Pro Glu Lys Phe Lys Glu Ile Ala Glu Leu Leu Lys Gly
            260                 265                 270

<210>   11
<211>   862
<212>   PRT
<213>   Streptococcus agalactiae

<400>   11
Gly Glu Thr Gln Asp Thr Asn Gln Ala Leu Gly Lys Val Ile Val Lys
1               5                   10                  15

Lys Thr Gly Asp Asn Ala Thr Pro Leu Gly Lys Ala Thr Phe Val Leu
            20                  25                  30

Lys Asn Asp Asn Asp Lys Ser Glu Thr Ser His Glu Thr Val Glu Gly
        35                  40                  45

Ser Gly Glu Ala Thr Phe Glu Asn Ile Lys Pro Gly Asp Tyr Thr Leu
    50                  55                  60

Arg Glu Glu Thr Ala Pro Ile Gly Tyr Lys Lys Thr Asp Lys Thr Trp
65                  70                  75                  80

Lys Val Lys Val Ala Asp Asn Gly Ala Thr Ile Ile Glu Gly Met Asp
                85                  90                  95

Ala Asp Lys Ala Glu Lys Arg Lys Glu Val Leu Asn Ala Gln Tyr Pro
            100                 105                 110

Lys Ser Ala Ile Tyr Glu Asp Thr Lys Glu Asn Tyr Pro Leu Val Asn
        115                 120                 125

Val Glu Gly Ser Lys Val Gly Glu Gln Tyr Lys Ala Leu Asn Pro Ile
    130                 135                 140

Asn Gly Lys Asp Gly Arg Arg Glu Ile Ala Glu Gly Trp Leu Ser Lys
145                 150                 155                 160

Lys Ile Thr Gly Val Asn Asp Leu Asp Lys Asn Lys Tyr Lys Ile Glu
                165                 170                 175

Leu Thr Val Glu Gly Lys Thr Thr Val Glu Thr Lys Glu Leu Asn Gln
            180                 185                 190

Pro Leu Asp Val Val Val Leu Leu Asp Asn Ser Asn Ser Met Asn Asn
        195                 200                 205

Glu Arg Ala Asn Asn Ser Gln Arg Ala Leu Lys Ala Gly Glu Ala Val
    210                 215                 220

Glu Lys Leu Ile Asp Lys Ile Thr Ser Asn Lys Asp Asn Arg Val Ala
225                 230                 235                 240

Leu Val Thr Tyr Ala Ser Thr Ile Phe Asp Gly Thr Glu Ala Thr Val
                245                 250                 255

Ser Lys Gly Val Ala Asp Gln Asn Gly Lys Ala Leu Asn Asp Ser Val
            260                 265                 270

Ser Trp Asp Tyr His Lys Thr Thr Phe Thr Ala Thr Thr His Asn Tyr
        275                 280                 285

Ser Tyr Leu Asn Leu Thr Asn Asp Ala Asn Glu Val Asn Ile Leu Lys
    290                 295                 300

Ser Arg Ile Pro Lys Glu Ala Glu His Ile Asn Gly Asp Arg Thr Leu
305                 310                 315                 320

Tyr Gln Phe Gly Ala Thr Phe Thr Gln Lys Ala Leu Met Lys Ala Asn
                325                 330                 335

Glu Ile Leu Glu Thr Gln Ser Ser Asn Ala Arg Lys Lys Leu Ile Phe
            340                 345                 350

His Val Thr Asp Gly Val Pro Thr Met Ser Tyr Ala Ile Asn Phe Asn
        355                 360                 365

Pro Tyr Ile Ser Thr Ser Tyr Gln Asn Gln Phe Asn Ser Phe Leu Asn
    370                 375                 380

Lys Ile Pro Asp Arg Ser Gly Ile Leu Gln Glu Asp Phe Ile Ile Asn
385                 390                 395                 400

Gly Asp Asp Tyr Gln Ile Val Lys Gly Asp Gly Glu Ser Phe Lys Leu
                405                 410                 415

Phe Ser Asp Arg Lys Val Pro Val Thr Gly Gly Thr Thr Gln Ala Ala
            420                 425                 430

Tyr Arg Val Pro Gln Asn Gln Leu Ser Val Met Ser Asn Glu Gly Tyr
        435                 440                 445

Ala Ile Asn Ser Gly Tyr Ile Tyr Leu Tyr Trp Arg Asp Tyr Asn Trp
    450                 455                 460

Val Tyr Pro Phe Asp Pro Lys Thr Lys Lys Val Ser Ala Thr Lys Gln
465                 470                 475                 480

Ile Lys Thr His Gly Glu Pro Thr Thr Leu Tyr Phe Asn Gly Asn Ile
                485                 490                 495

Arg Pro Lys Gly Tyr Asp Ile Phe Thr Val Gly Ile Gly Val Asn Gly
            500                 505                 510

Asp Pro Gly Ala Thr Pro Leu Glu Ala Glu Lys Phe Met Gln Ser Ile
        515                 520                 525

Ser Ser Lys Thr Glu Asn Tyr Thr Asn Val Asp Asp Thr Asn Lys Ile
    530                 535                 540

Tyr Asp Glu Leu Asn Lys Tyr Phe Lys Thr Ile Val Glu Glu Lys His
545                 550                 555                 560

Ser Ile Val Asp Gly Asn Val Thr Asp Pro Met Gly Glu Met Ile Glu
                565                 570                 575

Phe Gln Leu Lys Asn Gly Gln Ser Phe Thr His Asp Asp Tyr Val Leu
            580                 585                 590

Val Gly Asn Asp Gly Ser Gln Leu Lys Asn Gly Val Ala Leu Gly Gly
        595                 600                 605

Pro Asn Ser Asp Gly Gly Ile Leu Lys Asp Val Thr Val Thr Tyr Asp
    610                 615                 620

Lys Thr Ser Gln Thr Ile Lys Ile Asn His Leu Asn Leu Gly Ser Gly
625                 630                 635                 640

Gln Lys Val Val Leu Thr Tyr Asp Val Arg Leu Lys Asp Asn Tyr Ile
                645                 650                 655

Ser Asn Lys Phe Tyr Asn Thr Asn Asn Arg Thr Thr Leu Ser Pro Lys
            660                 665                 670

Ser Glu Lys Glu Pro Asn Thr Ile Arg Asp Phe Pro Ile Pro Lys Ile
        675                 680                 685

Arg Asp Val Arg Glu Phe Pro Val Leu Thr Ile Ser Asn Gln Lys Lys
    690                 695                 700

Met Gly Glu Val Glu Phe Ile Lys Val Asn Lys Asp Lys His Ser Glu
705                 710                 715                 720

Ser Leu Leu Gly Ala Lys Phe Gln Leu Gln Ile Glu Lys Asp Phe Ser
                725                 730                 735

Gly Tyr Lys Gln Phe Val Pro Glu Gly Ser Asp Val Thr Thr Lys Asn
            740                 745                 750

Asp Gly Lys Ile Tyr Phe Lys Ala Leu Gln Asp Gly Asn Tyr Lys Leu

755                 760                 765

Tyr Glu Ile Ser Ser Pro Asp Gly Tyr Ile Glu Val Lys Thr Lys Pro
    770                 775                 780

Val Val Thr Phe Thr Ile Gln Asn Gly Glu Val Thr Asn Leu Lys Ala
785                 790                 795                 800

Asp Pro Asn Ala Asn Lys Asn Gln Ile Gly Tyr Leu Glu Gly Asn Gly
                805                 810                 815

Lys His Leu Ile Thr Asn Thr Pro Lys Arg Pro Pro Gly Val Phe Pro
            820                 825                 830

Lys Thr Gly Gly Ile Gly Thr Ile Val Tyr Ile Leu Val Gly Ser Thr
        835                 840                 845

Phe Met Ile Leu Thr Ile Cys Ser Phe Arg Arg Lys Gln Leu
    850                 855                 860

<210>   12
<211>   851
<212>   PRT
<213>   Streptococcus agalactiae

<400>   12
Met Lys Lys Arg Gln Lys Ile Trp Arg Gly Leu Ser Val Thr Leu Leu
1               5                   10                  15

Ile Leu Ser Gln Ile Pro Phe Gly Ile Leu Val Gln Gly Glu Thr Gln
            20                  25                  30

Asp Thr Asn Gln Ala Leu Gly Lys Val Ile Val Lys Lys Thr Gly Asp
        35                  40                  45

Asn Ala Thr Pro Leu Gly Lys Ala Thr Phe Val Leu Lys Asn Asp Asn
    50                  55                  60

Asp Lys Ser Glu Thr Ser His Glu Thr Val Glu Gly Ser Gly Glu Ala
65                  70                  75                  80

Thr Phe Glu Asn Ile Lys Pro Gly Asp Tyr Thr Leu Arg Glu Glu Thr
                85                  90                  95

Ala Pro Ile Gly Tyr Lys Lys Thr Asp Lys Thr Trp Lys Val Lys Val
            100                 105                 110

Ala Asp Asn Gly Ala Thr Ile Ile Glu Gly Met Asp Ala Asp Lys Ala
        115                 120                 125

Glu Lys Arg Lys Glu Val Leu Asn Ala Gln Tyr Pro Lys Ser Ala Ile
    130                 135                 140

Tyr Glu Asp Thr Lys Glu Asn Tyr Pro Leu Val Asn Val Glu Gly Ser
145                 150                 155                 160

Lys Val Gly Glu Gln Tyr Lys Ala Leu Asn Pro Ile Asn Gly Lys Asp
                165                 170                 175

Gly Arg Arg Glu Ile Ala Glu Gly Trp Leu Ser Lys Lys Ile Thr Gly
            180                 185                 190

Val Asn Asp Leu Asp Lys Asn Lys Tyr Lys Ile Glu Leu Thr Val Glu
        195                 200                 205

Gly Lys Thr Thr Val Glu Thr Lys Glu Leu Asn Gln Pro Leu Asp Val
    210                 215                 220

Val Val Leu Leu Asp Asn Ser Asn Ser Met Asn Asn Glu Arg Ala Asn
225                 230                 235                 240

Asn Ser Gln Arg Ala Leu Lys Ala Gly Glu Ala Val Glu Lys Leu Ile
                245                 250                 255

Asp Lys Ile Thr Ser Asn Lys Asp Asn Arg Val Ala Leu Val Thr Tyr
            260                 265                 270

Ala Ser Thr Ile Phe Asp Gly Thr Glu Ala Thr Val Ser Lys Gly Val
        275                 280                 285

Ala Asp Gln Asn Gly Lys Ala Leu Asn Asp Ser Val Ser Trp Asp Tyr
    290                 295                 300

His Lys Thr Thr Phe Thr Ala Thr Thr His Asn Tyr Ser Tyr Leu Asn
305                 310                 315                 320

Leu Thr Asn Asp Ala Asn Glu Val Asn Ile Leu Lys Ser Arg Ile Pro
                325                 330                 335

Lys Glu Ala Glu His Ile Asn Gly Asp Arg Thr Leu Tyr Gln Phe Gly
            340                 345                 350

Ala Thr Phe Thr Gln Lys Ala Leu Met Lys Ala Asn Glu Ile Leu Glu
        355                 360                 365

Thr Gln Ser Ser Asn Ala Arg Lys Lys Leu Ile Phe His Val Thr Asp
    370                 375                 380

Gly Val Pro Thr Met Ser Tyr Ala Ile Asn Phe Asn Pro Tyr Ile Ser
385                 390                 395                 400

Thr Ser Tyr Gln Asn Gln Phe Asn Ser Phe Leu Asn Lys Ile Pro Asp
                405                 410                 415

Arg Ser Gly Ile Leu Gln Glu Asp Phe Ile Ile Asn Gly Asp Asp Tyr
            420                 425                 430

Gln Ile Val Lys Gly Asp Gly Glu Ser Phe Lys Leu Phe Ser Asp Arg
        435                 440                 445

Lys Val Pro Val Thr Gly Gly Thr Thr Gln Ala Ala Tyr Arg Val Pro
    450                 455                 460

Gln Asn Gln Leu Ser Val Met Ser Asn Glu Gly Tyr Ala Ile Asn Ser
465                 470                 475                 480

Gly Tyr Ile Tyr Leu Tyr Trp Arg Asp Tyr Asn Trp Val Tyr Pro Phe
                485                 490                 495

Asp Pro Lys Thr Lys Lys Val Ser Ala Thr Lys Gln Ile Lys Thr His
            500                 505                 510

Gly Glu Pro Thr Thr Leu Tyr Phe Asn Gly Asn Ile Arg Pro Lys Gly
        515                 520                 525

Tyr Asp Ile Phe Thr Val Gly Ile Gly Val Asn Gly Asp Pro Gly Ala
    530                 535                 540

Thr Pro Leu Glu Ala Glu Lys Phe Met Gln Ser Ile Ser Ser Lys Thr
545                 550                 555                 560

Glu Asn Tyr Thr Asn Val Asp Asp Thr Asn Lys Ile Tyr Asp Glu Leu
                565                 570                 575

Asn Lys Tyr Phe Lys Thr Ile Val Glu Glu Lys His Ser Ile Val Asp
            580                 585                 590

Gly Asn Val Thr Asp Pro Met Gly Glu Met Ile Glu Phe Gln Leu Lys
        595                 600                 605

Asn Gly Gln Ser Phe Thr His Asp Asp Tyr Val Leu Val Gly Asn Asp
    610                 615                 620

Gly Ser Gln Leu Lys Asn Gly Val Ala Leu Gly Gly Pro Asn Ser Asp
625                 630                 635                 640

Gly Gly Ile Leu Lys Asp Val Thr Val Thr Tyr Asp Lys Thr Ser Gln
                645                 650                 655

Thr Ile Lys Ile Asn His Leu Asn Leu Gly Ser Gly Gln Lys Val Val
            660                 665                 670

Leu Thr Tyr Asp Val Arg Leu Lys Asp Asn Tyr Ile Ser Asn Lys Phe
        675                 680                 685

Tyr Asn Thr Asn Asn Arg Thr Thr Leu Ser Pro Lys Ser Glu Lys Glu
    690                 695                 700

Pro Asn Thr Ile Arg Asp Phe Pro Ile Pro Lys Ile Arg Asp Val Arg
705                 710                 715                 720

Glu Phe Pro Val Leu Thr Ile Ser Asn Gln Lys Lys Met Gly Glu Val
                725                 730                 735

Glu Phe Ile Lys Val Asn Lys Asp Lys His Ser Glu Ser Leu Leu Gly
            740                 745                 750

Ala Lys Phe Gln Leu Gln Ile Glu Lys Asp Phe Ser Gly Tyr Lys Gln
        755                 760                 765

Phe Val Pro Glu Gly Ser Asp Val Thr Thr Lys Asn Asp Gly Lys Ile
    770                 775                 780

Tyr Phe Lys Ala Leu Gln Asp Gly Asn Tyr Lys Leu Tyr Glu Ile Ser
785                 790                 795                 800

Ser Pro Asp Gly Tyr Ile Glu Val Lys Thr Lys Pro Val Val Thr Phe
                805                 810                 815

Thr Ile Gln Asn Gly Glu Val Thr Asn Leu Lys Ala Asp Pro Asn Ala
            820                 825                 830

Asn Lys Asn Gln Ile Gly Tyr Leu Glu Gly Asn Gly Lys His Leu Ile

835                 840                 845

Thr Asn Thr
    850

<210>   13
<211>   823
<212>   PRT
<213>   Streptococcus agalactiae

<400>   13
Gly Glu Thr Gln Asp Thr Asn Gln Ala Leu Gly Lys Val Ile Val Lys
1               5                   10                  15

Lys Thr Gly Asp Asn Ala Thr Pro Leu Gly Lys Ala Thr Phe Val Leu
            20                  25                  30

Lys Asn Asp Asn Asp Lys Ser Glu Thr Ser His Glu Thr Val Glu Gly
        35                  40                  45

Ser Gly Glu Ala Thr Phe Glu Asn Ile Lys Pro Gly Asp Tyr Thr Leu
    50                  55                  60

Arg Glu Glu Thr Ala Pro Ile Gly Tyr Lys Lys Thr Asp Lys Thr Trp
65                  70                  75                  80

Lys Val Lys Val Ala Asp Asn Gly Ala Thr Ile Ile Glu Gly Met Asp
                85                  90                  95

Ala Asp Lys Ala Glu Lys Arg Lys Glu Val Leu Asn Ala Gln Tyr Pro
            100                 105                 110

Lys Ser Ala Ile Tyr Glu Asp Thr Lys Glu Asn Tyr Pro Leu Val Asn
        115                 120                 125

Val Glu Gly Ser Lys Val Gly Glu Gln Tyr Lys Ala Leu Asn Pro Ile
    130                 135                 140

Asn Gly Lys Asp Gly Arg Arg Glu Ile Ala Glu Gly Trp Leu Ser Lys
145                 150                 155                 160

Lys Ile Thr Gly Val Asn Asp Leu Asp Lys Asn Lys Tyr Lys Ile Glu
                165                 170                 175

Leu Thr Val Glu Gly Lys Thr Thr Val Glu Thr Lys Glu Leu Asn Gln
            180                 185                 190

Pro Leu Asp Val Val Val Leu Leu Asp Asn Ser Asn Ser Met Asn Asn
        195                 200                 205

Glu Arg Ala Asn Asn Ser Gln Arg Ala Leu Lys Ala Gly Glu Ala Val
    210                 215                 220

Glu Lys Leu Ile Asp Lys Ile Thr Ser Asn Lys Asp Asn Arg Val Ala
225                 230                 235                 240

Leu Val Thr Tyr Ala Ser Thr Ile Phe Asp Gly Thr Glu Ala Thr Val
                245                 250                 255

Ser Lys Gly Val Ala Asp Gln Asn Gly Lys Ala Leu Asn Asp Ser Val
            260                 265                 270

Ser Trp Asp Tyr His Lys Thr Thr Phe Thr Ala Thr Thr His Asn Tyr
        275                 280                 285

Ser Tyr Leu Asn Leu Thr Asn Asp Ala Asn Glu Val Asn Ile Leu Lys
    290                 295                 300

Ser Arg Ile Pro Lys Glu Ala Glu His Ile Asn Gly Asp Arg Thr Leu
305                 310                 315                 320

Tyr Gln Phe Gly Ala Thr Phe Thr Gln Lys Ala Leu Met Lys Ala Asn
                325                 330                 335

Glu Ile Leu Glu Thr Gln Ser Ser Asn Ala Arg Lys Lys Leu Ile Phe
            340                 345                 350

His Val Thr Asp Gly Val Pro Thr Met Ser Tyr Ala Ile Asn Phe Asn
        355                 360                 365

Pro Tyr Ile Ser Thr Ser Tyr Gln Asn Gln Phe Asn Ser Phe Leu Asn
    370                 375                 380

Lys Ile Pro Asp Arg Ser Gly Ile Leu Gln Glu Asp Phe Ile Ile Asn
385                 390                 395                 400

Gly Asp Asp Tyr Gln Ile Val Lys Gly Asp Gly Glu Ser Phe Lys Leu
                405                 410                 415

Phe Ser Asp Arg Lys Val Pro Val Thr Gly Gly Thr Thr Gln Ala Ala
            420                 425                 430

Tyr Arg Val Pro Gln Asn Gln Leu Ser Val Met Ser Asn Glu Gly Tyr
        435                 440                 445

Ala Ile Asn Ser Gly Tyr Ile Tyr Leu Tyr Trp Arg Asp Tyr Asn Trp
    450                 455                 460

Val Tyr Pro Phe Asp Pro Lys Thr Lys Lys Val Ser Ala Thr Lys Gln
465                 470                 475                 480

Ile Lys Thr His Gly Glu Pro Thr Thr Leu Tyr Phe Asn Gly Asn Ile
                485                 490                 495

Arg Pro Lys Gly Tyr Asp Ile Phe Thr Val Gly Ile Gly Val Asn Gly
            500                 505                 510

Asp Pro Gly Ala Thr Pro Leu Glu Ala Glu Lys Phe Met Gln Ser Ile
        515                 520                 525

Ser Ser Lys Thr Glu Asn Tyr Thr Asn Val Asp Asp Thr Asn Lys Ile
    530                 535                 540

Tyr Asp Glu Leu Asn Lys Tyr Phe Lys Thr Ile Val Glu Glu Lys His
545                 550                 555                 560

Ser Ile Val Asp Gly Asn Val Thr Asp Pro Met Gly Glu Met Ile Glu
                565                 570                 575

Phe Gln Leu Lys Asn Gly Gln Ser Phe Thr His Asp Asp Tyr Val Leu
            580                 585                 590

Val Gly Asn Asp Gly Ser Gln Leu Lys Asn Gly Val Ala Leu Gly Gly
        595                 600                 605

Pro Asn Ser Asp Gly Gly Ile Leu Lys Asp Val Thr Val Thr Tyr Asp
    610                 615                 620

Lys Thr Ser Gln Thr Ile Lys Ile Asn His Leu Asn Leu Gly Ser Gly
625                 630                 635                 640

Gln Lys Val Val Leu Thr Tyr Asp Val Arg Leu Lys Asp Asn Tyr Ile
                645                 650                 655

Ser Asn Lys Phe Tyr Asn Thr Asn Asn Arg Thr Thr Leu Ser Pro Lys
            660                 665                 670

Ser Glu Lys Glu Pro Asn Thr Ile Arg Asp Phe Pro Ile Pro Lys Ile
        675                 680                 685

Arg Asp Val Arg Glu Phe Pro Val Leu Thr Ile Ser Asn Gln Lys Lys
    690                 695                 700

Met Gly Glu Val Glu Phe Ile Lys Val Asn Lys Asp Lys His Ser Glu
705                 710                 715                 720

Ser Leu Leu Gly Ala Lys Phe Gln Leu Gln Ile Glu Lys Asp Phe Ser
                725                 730                 735

Gly Tyr Lys Gln Phe Val Pro Glu Gly Ser Asp Val Thr Thr Lys Asn
            740                 745                 750

Asp Gly Lys Ile Tyr Phe Lys Ala Leu Gln Asp Gly Asn Tyr Lys Leu
        755                 760                 765

Tyr Glu Ile Ser Ser Pro Asp Gly Tyr Ile Glu Val Lys Thr Lys Pro
    770                 775                 780

Val Val Thr Phe Thr Ile Gln Asn Gly Glu Val Thr Asn Leu Lys Ala
785                 790                 795                 800

Asp Pro Asn Ala Asn Lys Asn Gln Ile Gly Tyr Leu Glu Gly Asn Gly
                805                 810                 815

Lys His Leu Ile Thr Asn Thr
            820

<210>   14
<211>   1109
<212>   PRT
<213>   Streptococcus agalactiae

<400>   14
Gln Ser Asp Ile Lys Ala Asn Thr Val Thr Glu Asp Thr Pro Ala Thr
1               5                   10                  15

Glu Gln Ala Val Glu Pro Pro Gln Pro Ile Ala Val Ser Glu Glu Ser
            20                  25                  30

Arg Ser Ser Lys Glu Thr Lys Thr Ser Gln Thr Pro Ser Asp Val Gly
        35                  40                  45

Glu Thr Val Ala Asp Asp Ala Asn Asp Leu Ala Pro Gln Ala Pro Ala

50                  55                  60

Lys Thr Ala Asp Thr Pro Ala Thr Ser Lys Ala Thr Ile Arg Asp Leu
65                  70                  75                  80

Asn Asp Pro Ser His Val Lys Thr Leu Gln Glu Lys Ala Gly Lys Gly
                85                  90                  95

Ala Gly Thr Val Val Ala Val Ile Asp Ala Gly Phe Asp Lys Asn His
            100                 105                 110

Glu Ala Trp Arg Leu Thr Asp Lys Thr Lys Ala Arg Tyr Gln Ser Lys
        115                 120                 125

Glu Asn Leu Glu Lys Ala Lys Lys Glu His Gly Ile Thr Tyr Gly Glu
    130                 135                 140

Trp Val Asn Asp Lys Val Ala Tyr Tyr His Asp Tyr Ser Lys Asp Gly
145                 150                 155                 160

Lys Asn Ala Val Asp Gln Glu His Gly Thr His Val Ser Gly Ile Leu
                165                 170                 175

Ser Gly Asn Ala Pro Ser Glu Met Lys Glu Pro Tyr Arg Leu Glu Gly
            180                 185                 190

Ala Met Pro Glu Ala Gln Leu Leu Leu Met Arg Val Glu Ile Val Asn
        195                 200                 205

Gly Leu Ala Asp Tyr Ala Arg Asn Tyr Ala Gln Ala Ile Arg Asp Ala
    210                 215                 220

Val Asn Leu Gly Ala Lys Val Ile Asn Met Ser Phe Gly Asn Ala Ala
225                 230                 235                 240

Leu Ala Tyr Ala Asn Leu Pro Asp Glu Thr Lys Lys Ala Phe Asp Tyr
                245                 250                 255

Ala Lys Ser Lys Gly Val Ser Ile Val Thr Ser Ala Gly Asn Asp Ser
            260                 265                 270

Ser Phe Gly Gly Lys Pro Arg Leu Pro Leu Ala Asp His Pro Asp Tyr
        275                 280                 285

Gly Val Val Gly Thr Pro Ala Ala Ala Asp Ser Thr Leu Thr Val Ala
    290                 295                 300

Ser Tyr Ser Pro Asp Lys Gln Leu Thr Glu Thr Ala Thr Val Lys Thr
305                 310                 315                 320

Asp Asp His Gln Asp Lys Glu Met Pro Val Ile Ser Thr Asn Arg Phe
                325                 330                 335

Glu Pro Asn Lys Ala Tyr Asp Tyr Ala Tyr Ala Asn Arg Gly Thr Lys
            340                 345                 350

Glu Asp Asp Phe Lys Asp Val Glu Gly Lys Ile Ala Leu Ile Glu Arg
        355                 360                 365

Gly Asp Ile Asp Phe Lys Asp Lys Ile Ala Asn Ala Lys Lys Ala Gly
    370                 375                 380

Ala Val Gly Val Leu Ile Tyr Asp Asn Gln Asp Lys Gly Phe Pro Ile
385                 390                 395                 400

Glu Leu Pro Asn Val Asp Gln Met Pro Ala Ala Phe Ile Ser Arg Arg
                405                 410                 415

Asp Gly Leu Leu Leu Lys Asp Asn Pro Pro Lys Thr Ile Thr Phe Asn
            420                 425                 430

Ala Thr Pro Lys Val Leu Pro Thr Ala Ser Gly Thr Lys Leu Ser Arg
        435                 440                 445

Phe Ser Ser Trp Gly Leu Thr Ala Asp Gly Asn Ile Lys Pro Asp Ile
    450                 455                 460

Ala Ala Pro Gly Gln Asp Ile Leu Ser Ser Val Ala Asn Asn Lys Tyr
465                 470                 475                 480

Ala Lys Leu Ser Gly Thr Ser Met Ser Ala Pro Leu Val Ala Gly Ile
                485                 490                 495

Met Gly Leu Leu Gln Lys Gln Tyr Glu Thr Gln Tyr Pro Asp Met Thr
            500                 505                 510

Pro Ser Glu Arg Leu Asp Leu Ala Lys Lys Val Leu Met Ser Ser Ala
        515                 520                 525

Thr Ala Leu Tyr Asp Glu Asp Glu Lys Ala Tyr Phe Ser Pro Arg Gln
    530                 535                 540

Gln Gly Ala Gly Ala Val Asp Ala Lys Lys Ala Ser Ala Ala Thr Met
545                 550                 555                 560

Tyr Val Thr Asp Lys Asp Asn Thr Ser Ser Lys Val His Leu Asn Asn
                565                 570                 575

Val Ser Asp Lys Phe Glu Val Thr Val Thr Val His Asn Lys Ser Asp
            580                 585                 590

Lys Pro Gln Glu Leu Tyr Tyr Gln Val Thr Val Gln Thr Asp Lys Val
        595                 600                 605

Asp Gly Lys His Phe Ala Leu Ala Pro Lys Ala Leu Tyr Glu Thr Ser
    610                 615                 620

Trp Gln Lys Ile Thr Ile Pro Ala Asn Ser Ser Lys Gln Val Thr Val
625                 630                 635                 640

Pro Ile Asp Ala Ser Arg Phe Ser Lys Asp Leu Leu Ala Gln Met Lys
                645                 650                 655

Asn Gly Tyr Phe Leu Glu Gly Phe Val Arg Phe Lys Gln Asp Pro Thr
            660                 665                 670

Lys Glu Glu Leu Met Ser Ile Pro Tyr Ile Gly Phe Arg Gly Asp Phe
        675                 680                 685

Gly Asn Leu Ser Ala Leu Glu Lys Pro Ile Tyr Asp Ser Lys Asp Gly
    690                 695                 700

Ser Ser Tyr Tyr His Glu Ala Asn Ser Asp Ala Lys Asp Gln Leu Asp
705                 710                 715                 720

Gly Asp Gly Leu Gln Phe Tyr Ala Leu Lys Asn Asn Phe Thr Ala Leu
                725                 730                 735

Thr Thr Glu Ser Asn Pro Trp Thr Ile Ile Lys Ala Val Lys Glu Gly
            740                 745                 750

Val Glu Asn Ile Glu Asp Ile Glu Ser Ser Glu Ile Thr Glu Thr Ile
        755                 760                 765

Phe Ala Gly Thr Phe Ala Lys Gln Asp Asp Asp Ser His Tyr Tyr Ile
    770                 775                 780

His Arg His Ala Asn Gly Lys Pro Tyr Ala Ala Ile Ser Pro Asn Gly
785                 790                 795                 800

Asp Gly Asn Arg Asp Tyr Val Gln Phe Gln Gly Thr Phe Leu Arg Asn
                805                 810                 815

Ala Lys Asn Leu Val Ala Glu Val Leu Asp Lys Glu Gly Asn Val Val
            820                 825                 830

Trp Thr Ser Glu Val Thr Glu Gln Val Val Lys Asn Tyr Asn Asn Asp
        835                 840                 845

Leu Ala Ser Thr Leu Gly Ser Thr Arg Phe Glu Lys Thr Arg Trp Asp
    850                 855                 860

Gly Lys Asp Lys Asp Gly Lys Val Val Ala Asn Gly Thr Tyr Thr Tyr
865                 870                 875                 880

Arg Val Arg Tyr Thr Pro Ile Ser Ser Gly Ala Lys Glu Gln His Thr
                885                 890                 895

Asp Phe Asp Val Ile Val Asp Asn Thr Thr Pro Glu Val Ala Thr Ser
            900                 905                 910

Ala Thr Phe Ser Thr Glu Asp Ser Arg Leu Thr Leu Ala Ser Lys Pro
        915                 920                 925

Lys Thr Ser Gln Pro Val Tyr Arg Glu Arg Ile Ala Tyr Thr Tyr Met
    930                 935                 940

Asp Glu Asp Leu Pro Thr Thr Glu Tyr Ile Ser Pro Asn Glu Asp Gly
945                 950                 955                 960

Thr Phe Thr Leu Pro Glu Glu Ala Glu Thr Met Glu Gly Ala Thr Val
                965                 970                 975

Pro Leu Lys Met Ser Asp Phe Thr Tyr Val Val Glu Asp Met Ala Gly
            980                 985                 990

Asn Ile Thr Tyr Thr Pro Val Thr Lys Leu Leu Glu Gly His Ser Asn
        995                 1000                1005

Lys Pro Glu Gln Asp Gly Ser Asp Gln Ala Pro Asp Lys Lys Pro Glu
    1010                1015                1020

Ala Lys Pro Glu Gln Asp Gly Ser Gly Gln Thr Pro Asp Lys Lys Lys

1025                1030                1035                1040

Glu Thr Lys Pro Glu Lys Asp Ser Ser Gly Gln Thr Pro Gly Lys Thr
            1045                1050                1055

Pro Gln Lys Gly Gln Ser Ser Arg Thr Leu Glu Lys Arg Ser Ser Lys
        1060                1065                1070

Arg Ala Leu Ala Thr Lys Ala Ser Thr Arg Asp Gln Leu Pro Thr Thr
    1075                1080                1085

Asn Asp Lys Asp Thr Asn Arg Leu His Leu Leu Lys Leu Val Met Thr
    1090                1095                1100

Thr Phe Phe Leu Gly
1105

<210>  15
<211>  1103
<212>  PRT
<213>  Streptococcus agalactiae

<400>  15
Met Arg Lys Lys Gln Lys Leu Pro Phe Asp Lys Leu Ala Ile Ala Leu
1               5                   10                  15

Ile Ser Thr Ser Ile Leu Leu Asn Ala Gln Ser Asp Ile Lys Ala Asn
            20                  25                  30

Thr Val Thr Glu Asp Thr Pro Ala Thr Glu Gln Ala Val Glu Pro Pro
        35                  40                  45

Gln Pro Ile Ala Val Ser Glu Glu Ser Arg Ser Ser Lys Glu Thr Lys
    50                  55                  60

Thr Ser Gln Thr Pro Ser Asp Val Gly Glu Thr Val Ala Asp Asp Ala
65                  70                  75                  80

Asn Asp Leu Ala Pro Gln Ala Pro Ala Lys Thr Ala Asp Thr Pro Ala
                85                  90                  95

Thr Ser Lys Ala Thr Ile Arg Asp Leu Asn Asp Pro Ser His Val Lys
            100                 105                 110

Thr Leu Gln Glu Lys Ala Gly Lys Gly Ala Gly Thr Val Val Ala Val
        115                 120                 125

Ile Asp Ala Gly Phe Asp Lys Asn His Glu Ala Trp Arg Leu Thr Asp
    130                 135                 140

Lys Thr Lys Ala Arg Tyr Gln Ser Lys Glu Asn Leu Glu Lys Ala Lys
145                 150                 155                 160

Lys Glu His Gly Ile Thr Tyr Gly Glu Trp Val Asn Asp Lys Val Ala
                165                 170                 175

Tyr Tyr His Asp Tyr Ser Lys Asp Gly Lys Asn Ala Val Asp Gln Glu
            180                 185                 190

His Gly Thr His Val Ser Gly Ile Leu Ser Gly Asn Ala Pro Ser Glu
        195                 200                 205

Met Lys Glu Pro Tyr Arg Leu Glu Gly Ala Met Pro Glu Ala Gln Leu
    210                 215                 220

Leu Leu Met Arg Val Glu Ile Val Asn Gly Leu Ala Asp Tyr Ala Arg
225                 230                 235                 240

Asn Tyr Ala Gln Ala Ile Arg Asp Ala Val Asn Leu Gly Ala Lys Val
                245                 250                 255

Ile Asn Met Ser Phe Gly Asn Ala Ala Leu Ala Tyr Ala Asn Leu Pro
            260                 265                 270

Asp Glu Thr Lys Lys Ala Phe Asp Tyr Ala Lys Ser Lys Gly Val Ser
        275                 280                 285

Ile Val Thr Ser Ala Gly Asn Asp Ser Ser Phe Gly Gly Lys Pro Arg
    290                 295                 300

Leu Pro Leu Ala Asp His Pro Asp Tyr Gly Val Val Gly Thr Pro Ala
305                 310                 315                 320

Ala Ala Asp Ser Thr Leu Thr Val Ala Ser Tyr Ser Pro Asp Lys Gln
                325                 330                 335

Leu Thr Glu Thr Ala Thr Val Lys Thr Asp Asp His Gln Asp Lys Glu
            340                 345                 350

Met Pro Val Ile Ser Thr Asn Arg Phe Glu Pro Asn Lys Ala Tyr Asp
        355                 360                 365

Tyr Ala Tyr Ala Asn Arg Gly Thr Lys Glu Asp Asp Phe Lys Asp Val
    370                 375                 380

Glu Gly Lys Ile Ala Leu Ile Glu Arg Gly Asp Ile Asp Phe Lys Asp
385                 390                 395                 400

Lys Ile Ala Asn Ala Lys Lys Ala Gly Ala Val Gly Val Leu Ile Tyr
                405                 410                 415

Asp Asn Gln Asp Lys Gly Phe Pro Ile Glu Leu Pro Asn Val Asp Gln
            420                 425                 430

Met Pro Ala Ala Phe Ile Ser Arg Arg Asp Gly Leu Leu Leu Lys Asp
        435                 440                 445

Asn Pro Pro Lys Thr Ile Thr Phe Asn Ala Thr Pro Lys Val Leu Pro
    450                 455                 460

Thr Ala Ser Gly Thr Lys Leu Ser Arg Phe Ser Ser Trp Gly Leu Thr
465                 470                 475                 480

Ala Asp Gly Asn Ile Lys Pro Asp Ile Ala Ala Pro Gly Gln Asp Ile
                485                 490                 495

Leu Ser Ser Val Ala Asn Asn Lys Tyr Ala Lys Leu Ser Gly Thr Ser
            500                 505                 510

Met Ser Ala Pro Leu Val Ala Gly Ile Met Gly Leu Leu Gln Lys Gln
        515                 520                 525

Tyr Glu Thr Gln Tyr Pro Asp Met Thr Pro Ser Glu Arg Leu Asp Leu
    530                 535                 540

Ala Lys Lys Val Leu Met Ser Ser Ala Thr Ala Leu Tyr Asp Glu Asp
545                 550                 555                 560

Glu Lys Ala Tyr Phe Ser Pro Arg Gln Gln Gly Ala Gly Ala Val Asp
                565                 570                 575

Ala Lys Lys Ala Ser Ala Ala Thr Met Tyr Val Thr Asp Lys Asp Asn
            580                 585                 590

Thr Ser Ser Lys Val His Leu Asn Asn Val Ser Asp Lys Phe Glu Val
        595                 600                 605

Thr Val Thr Val His Asn Lys Ser Asp Lys Pro Gln Glu Leu Tyr Tyr
    610                 615                 620

Gln Val Thr Val Gln Thr Asp Lys Val Asp Gly Lys His Phe Ala Leu
625                 630                 635                 640

Ala Pro Lys Ala Leu Tyr Glu Thr Ser Trp Gln Lys Ile Thr Ile Pro
                645                 650                 655

Ala Asn Ser Ser Lys Gln Val Thr Val Pro Ile Asp Ala Ser Arg Phe
            660                 665                 670

Ser Lys Asp Leu Leu Ala Gln Met Lys Asn Gly Tyr Phe Leu Glu Gly
        675                 680                 685

Phe Val Arg Phe Lys Gln Asp Pro Thr Lys Glu Glu Leu Met Ser Ile
    690                 695                 700

Pro Tyr Ile Gly Phe Arg Gly Asp Phe Gly Asn Leu Ser Ala Leu Glu
705                 710                 715                 720

Lys Pro Ile Tyr Asp Ser Lys Asp Gly Ser Ser Tyr Tyr His Glu Ala
                725                 730                 735

Asn Ser Asp Ala Lys Asp Gln Leu Asp Gly Asp Gly Leu Gln Phe Tyr
            740                 745                 750

Ala Leu Lys Asn Asn Phe Thr Ala Leu Thr Thr Glu Ser Asn Pro Trp
        755                 760                 765

Thr Ile Ile Lys Ala Val Lys Glu Gly Val Glu Asn Ile Glu Asp Ile
    770                 775                 780

Glu Ser Ser Glu Ile Thr Glu Thr Ile Phe Ala Gly Thr Phe Ala Lys
785                 790                 795                 800

Gln Asp Asp Asp Ser His Tyr Tyr Ile His Arg His Ala Asn Gly Lys
                805                 810                 815

Pro Tyr Ala Ala Ile Ser Pro Asn Gly Asp Gly Asn Arg Asp Tyr Val
            820                 825                 830

Gln Phe Gln Gly Thr Phe Leu Arg Asn Ala Lys Asn Leu Val Ala Glu
        835                 840                 845

Val Leu Asp Lys Glu Gly Asn Val Val Trp Thr Ser Glu Val Thr Glu

850                 855                 860

Gln Val Val Lys Asn Tyr Asn Asn Asp Leu Ala Ser Thr Leu Gly Ser
865                 870                 875                 880

Thr Arg Phe Glu Lys Thr Arg Trp Asp Gly Lys Asp Lys Asp Gly Lys
                885                 890                 895

Val Val Ala Asn Gly Thr Tyr Thr Tyr Arg Val Arg Tyr Thr Pro Ile
            900                 905                 910

Ser Ser Gly Ala Lys Glu Gln His Thr Asp Phe Asp Val Ile Val Asp
        915                 920                 925

Asn Thr Thr Pro Glu Val Ala Thr Ser Ala Thr Phe Ser Thr Glu Asp
    930                 935                 940

Ser Arg Leu Thr Leu Ala Ser Lys Pro Lys Thr Ser Gln Pro Val Tyr
945                 950                 955                 960

Arg Glu Arg Ile Ala Tyr Thr Tyr Met Asp Glu Asp Leu Pro Thr Thr
                965                 970                 975

Glu Tyr Ile Ser Pro Asn Glu Asp Gly Thr Phe Thr Leu Pro Glu Glu
            980                 985                 990

Ala Glu Thr Met Glu Gly Ala Thr Val Pro Leu Lys Met Ser Asp Phe
        995                 1000                1005

Thr Tyr Val Val Glu Asp Met Ala Gly Asn Ile Thr Tyr Thr Pro Val
    1010                1015                1020

Thr Lys Leu Leu Glu Gly His Ser Asn Lys Pro Glu Gln Asp Gly Ser
1025                1030                1035                1040

Asp Gln Ala Pro Asp Lys Lys Pro Glu Ala Lys Pro Glu Gln Asp Gly
                1045                1050                1055

Ser Gly Gln Thr Pro Asp Lys Lys Lys Glu Thr Lys Pro Glu Lys Asp
            1060                1065                1070

Ser Ser Gly Gln Thr Pro Gly Lys Thr Pro Gln Lys Gly Gln Ser Ser
        1075                1080                1085

Arg Thr Leu Glu Lys Arg Ser Ser Lys Arg Ala Leu Ala Thr Lys
    1090                1095                1100

<210>   16
<211>   1078
<212>   PRT
<213>   Streptococcus agalactiae

<400>   16
Gln Ser Asp Ile Lys Ala Asn Thr Val Thr Glu Asp Thr Pro Ala Thr
1               5                   10                  15

Glu Gln Ala Val Glu Pro Pro Gln Pro Ile Ala Val Ser Glu Glu Ser
            20                  25                  30

Arg Ser Ser Lys Glu Thr Lys Thr Ser Gln Thr Pro Ser Asp Val Gly
        35                  40                  45

Glu Thr Val Ala Asp Asp Ala Asn Asp Leu Ala Pro Gln Ala Pro Ala
    50                  55                  60

Lys Thr Ala Asp Thr Pro Ala Thr Ser Lys Ala Thr Ile Arg Asp Leu
65                  70                  75                  80

Asn Asp Pro Ser His Val Lys Thr Leu Gln Glu Lys Ala Gly Lys Gly
                85                  90                  95

Ala Gly Thr Val Val Ala Val Ile Asp Ala Gly Phe Asp Lys Asn His
            100                 105                 110

Glu Ala Trp Arg Leu Thr Asp Lys Thr Lys Ala Arg Tyr Gln Ser Lys
        115                 120                 125

Glu Asn Leu Glu Lys Ala Lys Lys Glu His Gly Ile Thr Tyr Gly Glu
    130                 135                 140

Trp Val Asn Asp Lys Val Ala Tyr Tyr His Asp Tyr Ser Lys Asp Gly
145                 150                 155                 160

Lys Asn Ala Val Asp Gln Glu His Gly Thr His Val Ser Gly Ile Leu
                165                 170                 175

Ser Gly Asn Ala Pro Ser Glu Met Lys Glu Pro Tyr Arg Leu Glu Gly
            180                 185                 190

Ala Met Pro Glu Ala Gln Leu Leu Leu Met Arg Val Glu Ile Val Asn
        195                 200                 205

Gly Leu Ala Asp Tyr Ala Arg Asn Tyr Ala Gln Ala Ile Arg Asp Ala
    210                 215                 220

Val Asn Leu Gly Ala Lys Val Ile Asn Met Ser Phe Gly Asn Ala Ala
225                 230                 235                 240

Leu Ala Tyr Ala Asn Leu Pro Asp Glu Thr Lys Lys Ala Phe Asp Tyr
                245                 250                 255

Ala Lys Ser Lys Gly Val Ser Ile Val Thr Ser Ala Gly Asn Asp Ser
            260                 265                 270

Ser Phe Gly Gly Lys Pro Arg Leu Pro Leu Ala Asp His Pro Asp Tyr
        275                 280                 285

Gly Val Val Gly Thr Pro Ala Ala Ala Asp Ser Thr Leu Thr Val Ala
    290                 295                 300

Ser Tyr Ser Pro Asp Lys Gln Leu Thr Glu Thr Ala Thr Val Lys Thr
305                 310                 315                 320

Asp Asp His Gln Asp Lys Glu Met Pro Val Ile Ser Thr Asn Arg Phe
                325                 330                 335

Glu Pro Asn Lys Ala Tyr Asp Tyr Ala Tyr Ala Asn Arg Gly Thr Lys
            340                 345                 350

Glu Asp Asp Phe Lys Asp Val Glu Gly Lys Ile Ala Leu Ile Glu Arg
        355                 360                 365

Gly Asp Ile Asp Phe Lys Asp Lys Ile Ala Asn Ala Lys Lys Ala Gly
    370                 375                 380

Ala Val Gly Val Leu Ile Tyr Asp Asn Gln Asp Lys Gly Phe Pro Ile
385                 390                 395                 400

Glu Leu Pro Asn Val Asp Gln Met Pro Ala Ala Phe Ile Ser Arg Arg
                405                 410                 415

Asp Gly Leu Leu Leu Lys Asp Asn Pro Pro Lys Thr Ile Thr Phe Asn
            420                 425                 430

Ala Thr Pro Lys Val Leu Pro Thr Ala Ser Gly Thr Lys Leu Ser Arg
        435                 440                 445

Phe Ser Ser Trp Gly Leu Thr Ala Asp Gly Asn Ile Lys Pro Asp Ile
    450                 455                 460

Ala Ala Pro Gly Gln Asp Ile Leu Ser Ser Val Ala Asn Asn Lys Tyr
465                 470                 475                 480

Ala Lys Leu Ser Gly Thr Ser Met Ser Ala Pro Leu Val Ala Gly Ile
                485                 490                 495

Met Gly Leu Leu Gln Lys Gln Tyr Glu Thr Gln Tyr Pro Asp Met Thr
            500                 505                 510

Pro Ser Glu Arg Leu Asp Leu Ala Lys Lys Val Leu Met Ser Ser Ala
        515                 520                 525

Thr Ala Leu Tyr Asp Glu Asp Glu Lys Ala Tyr Phe Ser Pro Arg Gln
    530                 535                 540

Gln Gly Ala Gly Ala Val Asp Ala Lys Lys Ala Ser Ala Ala Thr Met
545                 550                 555                 560

Tyr Val Thr Asp Lys Asp Asn Thr Ser Ser Lys Val His Leu Asn Asn
                565                 570                 575

Val Ser Asp Lys Phe Glu Val Thr Val Thr Val His Asn Lys Ser Asp
            580                 585                 590

Lys Pro Gln Glu Leu Tyr Tyr Gln Val Thr Val Gln Thr Asp Lys Val
        595                 600                 605

Asp Gly Lys His Phe Ala Leu Ala Pro Lys Ala Leu Tyr Glu Thr Ser
    610                 615                 620

Trp Gln Lys Ile Thr Ile Pro Ala Asn Ser Ser Lys Gln Val Thr Val
625                 630                 635                 640

Pro Ile Asp Ala Ser Arg Phe Ser Lys Asp Leu Leu Ala Gln Met Lys
                645                 650                 655

Asn Gly Tyr Phe Leu Glu Gly Phe Val Arg Phe Lys Gln Asp Pro Thr
            660                 665                 670

Lys Glu Glu Leu Met Ser Ile Pro Tyr Ile Gly Phe Arg Gly Asp Phe
        675                 680                 685

Gly Asn Leu Ser Ala Leu Glu Lys Pro Ile Tyr Asp Ser Lys Asp Gly

690                 695                 700

Ser Ser Tyr Tyr His Glu Ala Asn Ser Asp Ala Lys Asp Gln Leu Asp
705                 710                 715                 720

Gly Asp Gly Leu Gln Phe Tyr Ala Leu Lys Asn Asn Phe Thr Ala Leu
                725                 730                 735

Thr Thr Glu Ser Asn Pro Trp Thr Ile Ile Lys Ala Val Lys Glu Gly
            740                 745                 750

Val Glu Asn Ile Glu Asp Ile Glu Ser Ser Glu Ile Thr Glu Thr Ile
        755                 760                 765

Phe Ala Gly Thr Phe Ala Lys Gln Asp Asp Asp Ser His Tyr Tyr Ile
    770                 775                 780

His Arg His Ala Asn Gly Lys Pro Tyr Ala Ala Ile Ser Pro Asn Gly
785                 790                 795                 800

Asp Gly Asn Arg Asp Tyr Val Gln Phe Gln Gly Thr Phe Leu Arg Asn
                805                 810                 815

Ala Lys Asn Leu Val Ala Glu Val Leu Asp Lys Glu Gly Asn Val Val
            820                 825                 830

Trp Thr Ser Glu Val Thr Glu Gln Val Val Lys Asn Tyr Asn Asn Asp
        835                 840                 845

Leu Ala Ser Thr Leu Gly Ser Thr Arg Phe Glu Lys Thr Arg Trp Asp
    850                 855                 860

Gly Lys Asp Lys Asp Gly Lys Val Val Ala Asn Gly Thr Tyr Thr Tyr
865                 870                 875                 880

Arg Val Arg Tyr Thr Pro Ile Ser Ser Gly Ala Lys Glu Gln His Thr
                885                 890                 895

Asp Phe Asp Val Ile Val Asp Asn Thr Thr Pro Glu Val Ala Thr Ser
            900                 905                 910

Ala Thr Phe Ser Thr Glu Asp Ser Arg Leu Thr Leu Ala Ser Lys Pro
        915                 920                 925

Lys Thr Ser Gln Pro Val Tyr Arg Glu Arg Ile Ala Tyr Thr Tyr Met
    930                 935                 940

Asp Glu Asp Leu Pro Thr Thr Glu Tyr Ile Ser Pro Asn Glu Asp Gly
945                 950                 955                 960

Thr Phe Thr Leu Pro Glu Glu Ala Glu Thr Met Glu Gly Ala Thr Val
                965                 970                 975

Pro Leu Lys Met Ser Asp Phe Thr Tyr Val Val Glu Asp Met Ala Gly
            980                 985                 990

Asn Ile Thr Tyr Thr Pro Val Thr Lys Leu Leu Glu Gly His Ser Asn
        995                 1000                1005

Lys Pro Glu Gln Asp Gly Ser Asp Gln Ala Pro Asp Lys Lys Pro Glu
    1010                1015                1020

Ala Lys Pro Glu Gln Asp Gly Ser Gly Gln Thr Pro Asp Lys Lys Lys
1025                1030                1035                1040

Glu Thr Lys Pro Glu Lys Asp Ser Ser Gly Gln Thr Pro Gly Lys Thr
                1045                1050                1055

Pro Gln Lys Gly Gln Ser Ser Arg Thr Leu Glu Lys Arg Ser Ser Lys
            1060                1065                1070

Arg Ala Leu Ala Thr Lys
        1075

<210>   17
<211>   392
<212>   PRT
<213>   Streptococcus agalactiae

<400>   17
Asp Leu Val Lys Gln Asp Asn Lys Ser Ser Tyr Thr Val Lys Tyr Gly
1               5                   10                  15

Asp Thr Leu Ser Val Ile Ser Glu Ala Met Ser Ile Asp Met Asn Val
            20                  25                  30

Leu Ala Lys Ile Asn Asn Ile Ala Asp Ile Asn Leu Ile Tyr Pro Glu
        35                  40                  45

Thr Thr Leu Thr Val Thr Tyr Asp Gln Lys Ser His Thr Ala Thr Ser
    50                  55                  60

Met Lys Ile Glu Thr Pro Ala Thr Asn Ala Ala Gly Gln Thr Thr Ala
65                  70                  75                  80

Thr Val Asp Leu Lys Thr Asn Gln Val Ser Val Ala Asp Gln Lys Val
                85                  90                  95

Ser Leu Asn Thr Ile Ser Glu Gly Met Thr Pro Glu Ala Ala Thr Thr
            100                 105                 110

Ile Val Ser Pro Met Lys Thr Tyr Ser Ser Ala Pro Ala Leu Lys Ser
        115                 120                 125

Lys Glu Val Leu Ala Gln Glu Gln Ala Val Ser Gln Ala Ala Ala Asn
    130                 135                 140

Glu Gln Val Ser Pro Ala Pro Val Lys Ser Ile Thr Ser Glu Val Pro
145                 150                 155                 160

Ala Ala Lys Glu Glu Val Lys Pro Thr Gln Thr Ser Val Ser Gln Ser
                165                 170                 175

Thr Thr Val Ser Pro Ala Ser Val Ala Ala Glu Thr Pro Ala Pro Val
            180                 185                 190

Ala Lys Val Ala Pro Val Arg Thr Val Ala Ala Pro Arg Val Ala Ser
        195                 200                 205

Val Lys Val Val Thr Pro Lys Val Glu Thr Gly Ala Ser Pro Glu His
    210                 215                 220

Val Ser Ala Pro Ala Val Pro Val Thr Thr Thr Ser Pro Ala Thr Asp
225                 230                 235                 240

Ser Lys Leu Gln Ala Thr Glu Val Lys Ser Val Pro Val Ala Gln Lys
                245                 250                 255

Ala Pro Thr Ala Thr Pro Val Ala Gln Pro Ala Ser Thr Thr Asn Ala
            260                 265                 270

Val Ala Ala His Pro Glu Asn Ala Gly Leu Gln Pro His Val Ala Ala
        275                 280                 285

Tyr Lys Glu Lys Val Ala Ser Thr Tyr Gly Val Asn Glu Phe Ser Thr
    290                 295                 300

Tyr Arg Ala Gly Asp Pro Gly Asp His Gly Lys Gly Leu Ala Val Asp
305                 310                 315                 320

Phe Ile Val Gly Thr Asn Gln Ala Leu Gly Asn Lys Val Ala Gln Tyr
                325                 330                 335

Ser Thr Gln Asn Met Ala Ala Asn Asn Ile Ser Tyr Val Ile Trp Gln
            340                 345                 350

Gln Lys Phe Tyr Ser Asn Thr Asn Ser Ile Tyr Gly Pro Ala Asn Thr
        355                 360                 365

Trp Asn Ala Met Pro Asp Arg Gly Gly Val Thr Ala Asn His Tyr Asp
    370                 375                 380

His Val His Val Ser Phe Asn Lys
385                 390

<210>   18
<211>   869
<212>   PRT
<213>   Streptococcus agalactiae

<400>   18
Met Arg Lys Tyr Gln Lys Phe Ser Lys Ile Leu Thr Leu Ser Leu Phe
1               5                   10                  15

Cys Leu Ser Gln Ile Pro Leu Asn Thr Asn Val Leu Gly Glu Ser Thr
            20                  25                  30

Val Pro Glu Asn Gly Ala Lys Gly Lys Leu Val Val Lys Lys Thr Asp
        35                  40                  45

Asp Gln Asn Lys Pro Leu Ser Lys Ala Thr Phe Val Leu Lys Thr Thr
    50                  55                  60

Ala His Pro Glu Ser Lys Ile Glu Lys Val Thr Ala Glu Leu Thr Gly
65                  70                  75                  80

Glu Ala Thr Phe Asp Asn Leu Ile Pro Gly Asp Tyr Thr Leu Ser Glu
                85                  90                  95

Glu Thr Ala Pro Glu Gly Tyr Lys Lys Thr Asn Gln Thr Trp Gln Val
            100                 105                 110

Lys Val Glu Ser Asn Gly Lys Thr Thr Ile Gln Asn Ser Gly Asp Lys

115                 120                 125

Asn Ser Thr Ile Gly Gln Asn Gln Glu Glu Leu Asp Lys Gln Tyr Pro
    130                 135                 140

Pro Thr Gly Ile Tyr Glu Asp Thr Lys Glu Ser Tyr Lys Leu Glu His
145                 150                 155                 160

Val Lys Gly Ser Val Pro Asn Gly Lys Ser Glu Ala Lys Ala Val Asn
                165                 170                 175

Pro Tyr Ser Ser Glu Gly Glu His Ile Arg Glu Ile Pro Glu Gly Thr
            180                 185                 190

Leu Ser Lys Arg Ile Ser Glu Val Gly Asp Leu Ala His Asn Lys Tyr
        195                 200                 205

Lys Ile Glu Leu Thr Val Ser Gly Lys Thr Ile Val Lys Pro Val Asp
    210                 215                 220

Lys Gln Lys Pro Leu Asp Val Val Phe Val Leu Asp Asn Ser Asn Ser
225                 230                 235                 240

Met Asn Asn Asp Gly Pro Asn Phe Gln Arg His Asn Lys Ala Lys Lys
                245                 250                 255

Ala Ala Glu Ala Leu Gly Thr Ala Val Lys Asp Ile Leu Gly Ala Asn
            260                 265                 270

Ser Asp Asn Arg Val Ala Leu Val Thr Tyr Gly Ser Asp Ile Phe Asp
        275                 280                 285

Gly Arg Ser Val Asp Val Val Lys Gly Phe Lys Glu Asp Asp Lys Tyr
    290                 295                 300

Tyr Gly Leu Gln Thr Lys Phe Thr Ile Gln Thr Glu Asn Tyr Ser His
305                 310                 315                 320

Lys Gln Leu Thr Asn Asn Ala Glu Glu Ile Ile Lys Arg Ile Pro Thr
                325                 330                 335

Glu Ala Pro Lys Ala Lys Trp Gly Ser Thr Thr Asn Gly Leu Thr Pro
            340                 345                 350

Glu Gln Gln Lys Glu Tyr Tyr Leu Ser Lys Val Gly Glu Thr Phe Thr
        355                 360                 365

Met Lys Ala Phe Met Glu Ala Asp Asp Ile Leu Ser Gln Val Asn Arg
    370                 375                 380

Asn Ser Gln Lys Ile Ile Val His Val Thr Asp Gly Val Pro Thr Arg
385                 390                 395                 400

Ser Tyr Ala Ile Asn Asn Phe Lys Leu Gly Ala Ser Tyr Glu Ser Gln
                405                 410                 415

Phe Glu Gln Met Lys Lys Asn Gly Tyr Leu Asn Lys Ser Asn Phe Leu
            420                 425                 430

Leu Thr Asp Lys Pro Glu Asp Ile Lys Gly Asn Gly Glu Ser Tyr Phe
        435                 440                 445

Leu Phe Pro Leu Asp Ser Tyr Gln Thr Gln Ile Ile Ser Gly Asn Leu
    450                 455                 460

Gln Lys Leu His Tyr Leu Asp Leu Asn Leu Asn Tyr Pro Lys Gly Thr
465                 470                 475                 480

Ile Tyr Arg Asn Gly Pro Val Lys Glu His Gly Thr Pro Thr Lys Leu
                485                 490                 495

Tyr Ile Asn Ser Leu Lys Gln Lys Asn Tyr Asp Ile Phe Asn Phe Gly
            500                 505                 510

Ile Asp Ile Ser Gly Phe Arg Gln Val Tyr Asn Glu Glu Tyr Lys Lys
        515                 520                 525

Asn Gln Asp Gly Thr Phe Gln Lys Leu Lys Glu Glu Ala Phe Lys Leu
    530                 535                 540

Ser Asp Gly Glu Ile Thr Glu Leu Met Arg Ser Phe Ser Ser Lys Pro
545                 550                 555                 560

Glu Tyr Tyr Thr Pro Ile Val Thr Ser Ala Asp Thr Ser Asn Asn Glu
                565                 570                 575

Ile Leu Ser Lys Ile Gln Gln Gln Phe Glu Thr Ile Leu Thr Lys Glu
            580                 585                 590

Asn Ser Ile Val Asn Gly Thr Ile Glu Asp Pro Met Gly Asp Lys Ile
        595                 600                 605

Asn Leu Gln Leu Gly Asn Gly Gln Thr Leu Gln Pro Ser Asp Tyr Thr
    610                 615                 620

Leu Gln Gly Asn Asp Gly Ser Val Met Lys Asp Gly Ile Ala Thr Gly
625                 630                 635                 640

Gly Pro Asn Asn Asp Gly Gly Ile Leu Lys Gly Val Lys Leu Glu Tyr
                645                 650                 655

Ile Gly Asn Lys Leu Tyr Val Arg Gly Leu Asn Leu Gly Glu Gly Gln
            660                 665                 670

Lys Val Thr Leu Thr Tyr Asp Val Lys Leu Asp Asp Ser Phe Ile Ser
        675                 680                 685

Asn Lys Phe Tyr Asp Thr Asn Gly Arg Thr Thr Leu Asn Pro Lys Ser
    690                 695                 700

Glu Asp Pro Asn Thr Leu Arg Asp Phe Pro Ile Pro Lys Ile Arg Asp
705                 710                 715                 720

Val Arg Glu Tyr Pro Thr Ile Thr Ile Lys Asn Glu Lys Lys Leu Gly
                725                 730                 735

Glu Ile Glu Phe Ile Lys Val Asp Lys Asp Asn Asn Lys Leu Leu Leu
            740                 745                 750

Lys Gly Ala Thr Phe Glu Leu Gln Glu Phe Asn Glu Asp Tyr Lys Leu
        755                 760                 765

Tyr Leu Pro Ile Lys Asn Asn Asn Ser Lys Val Val Thr Gly Glu Asn
    770                 775                 780

Gly Lys Ile Ser Tyr Lys Asp Leu Lys Asp Gly Lys Tyr Gln Leu Ile
785                 790                 795                 800

Glu Ala Val Ser Pro Glu Asp Tyr Gln Lys Ile Thr Asn Lys Pro Ile
                805                 810                 815

Leu Thr Phe Glu Val Val Lys Gly Ser Ile Lys Asn Ile Ile Ala Val
            820                 825                 830

Asn Lys Gln Ile Ser Glu Tyr His Glu Glu Gly Asp Lys His Leu Ile
        835                 840                 845

Thr Asn Thr His Ile Pro Pro Lys Gly Ile Ile Pro Met Thr Gly Gly
    850                 855                 860

Lys Gly Ile Leu Ser
865

<210>   19
<211>   858
<212>   PRT
<213>   Streptococcus agalactiae

<400>   19
Met Arg Lys Tyr Gln Lys Phe Ser Lys Ile Leu Thr Leu Ser Leu Phe
1               5                   10                  15

Cys Leu Ser Gln Ile Pro Leu Asn Thr Asn Val Leu Gly Glu Ser Thr
            20                  25                  30

Val Pro Glu Asn Gly Ala Lys Gly Lys Leu Val Val Lys Lys Thr Asp
        35                  40                  45

Asp Gln Asn Lys Pro Leu Ser Lys Ala Thr Phe Val Leu Lys Thr Thr
    50                  55                  60

Ala His Pro Glu Ser Lys Ile Glu Lys Val Thr Ala Glu Leu Thr Gly
65                  70                  75                  80

Glu Ala Thr Phe Asp Asn Leu Ile Pro Gly Asp Tyr Thr Leu Ser Glu
                85                  90                  95

Glu Thr Ala Pro Glu Gly Tyr Lys Lys Thr Asn Gln Thr Trp Gln Val
            100                 105                 110

Lys Val Glu Ser Asn Gly Lys Thr Thr Ile Gln Asn Ser Gly Asp Lys
        115                 120                 125

Asn Ser Thr Ile Gly Gln Asn Gln Glu Glu Leu Asp Lys Gln Tyr Pro
    130                 135                 140

Pro Thr Gly Ile Tyr Glu Asp Thr Lys Glu Ser Tyr Lys Leu Glu His
145                 150                 155                 160

Val Lys Gly Ser Val Pro Asn Gly Lys Ser Glu Ala Lys Ala Val Asn
                165                 170                 175

Pro Tyr Ser Ser Glu Gly Glu His Ile Arg Glu Ile Pro Glu Gly Thr

            180                 185                 190

Leu Ser Lys Arg Ile Ser Glu Val Gly Asp Leu Ala His Asn Lys Tyr
        195                 200                 205

Lys Ile Glu Leu Thr Val Ser Gly Lys Thr Ile Val Lys Pro Val Asp
    210                 215                 220

Lys Gln Lys Pro Leu Asp Val Val Phe Val Leu Asp Asn Ser Asn Ser
225                 230                 235                 240

Met Asn Asn Asp Gly Pro Asn Phe Gln Arg His Asn Lys Ala Lys Lys
                245                 250                 255

Ala Ala Glu Ala Leu Gly Thr Ala Val Lys Asp Ile Leu Gly Ala Asn
            260                 265                 270

Ser Asp Asn Arg Val Ala Leu Val Thr Tyr Gly Ser Asp Ile Phe Asp
        275                 280                 285

Gly Arg Ser Val Asp Val Val Lys Gly Phe Lys Glu Asp Asp Lys Tyr
    290                 295                 300

Tyr Gly Leu Gln Thr Lys Phe Thr Ile Gln Thr Glu Asn Tyr Ser His
305                 310                 315                 320

Lys Gln Leu Thr Asn Asn Ala Glu Glu Ile Ile Lys Arg Ile Pro Thr
                325                 330                 335

Glu Ala Pro Lys Ala Lys Trp Gly Ser Thr Thr Asn Gly Leu Thr Pro
            340                 345                 350

Glu Gln Gln Lys Glu Tyr Tyr Leu Ser Lys Val Gly Glu Thr Phe Thr
        355                 360                 365

Met Lys Ala Phe Met Glu Ala Asp Asp Ile Leu Ser Gln Val Asn Arg
    370                 375                 380

Asn Ser Gln Lys Ile Ile Val His Val Thr Asp Gly Val Pro Thr Arg
385                 390                 395                 400

Ser Tyr Ala Ile Asn Asn Phe Lys Leu Gly Ala Ser Tyr Glu Ser Gln
                405                 410                 415

Phe Glu Gln Met Lys Lys Asn Gly Tyr Leu Asn Lys Ser Asn Phe Leu
            420                 425                 430

Leu Thr Asp Lys Pro Glu Asp Ile Lys Gly Asn Gly Glu Ser Tyr Phe
        435                 440                 445

Leu Phe Pro Leu Asp Ser Tyr Gln Thr Gln Ile Ile Ser Gly Asn Leu
    450                 455                 460

Gln Lys Leu His Tyr Leu Asp Leu Asn Leu Asn Tyr Pro Lys Gly Thr
465                 470                 475                 480

Ile Tyr Arg Asn Gly Pro Val Lys Glu His Gly Thr Pro Thr Lys Leu
                485                 490                 495

Tyr Ile Asn Ser Leu Lys Gln Lys Asn Tyr Asp Ile Phe Asn Phe Gly
            500                 505                 510

Ile Asp Ile Ser Gly Phe Arg Gln Val Tyr Asn Glu Glu Tyr Lys Lys
        515                 520                 525

Asn Gln Asp Gly Thr Phe Gln Lys Leu Lys Glu Glu Ala Phe Lys Leu
    530                 535                 540

Ser Asp Gly Glu Ile Thr Glu Leu Met Arg Ser Phe Ser Ser Lys Pro
545                 550                 555                 560

Glu Tyr Tyr Thr Pro Ile Val Thr Ser Ala Asp Thr Ser Asn Asn Glu
                565                 570                 575

Ile Leu Ser Lys Ile Gln Gln Gln Phe Glu Thr Ile Leu Thr Lys Glu
            580                 585                 590

Asn Ser Ile Val Asn Gly Thr Ile Glu Asp Pro Met Gly Asp Lys Ile
        595                 600                 605

Asn Leu Gln Leu Gly Asn Gly Gln Thr Leu Gln Pro Ser Asp Tyr Thr
    610                 615                 620

Leu Gln Gly Asn Asp Gly Ser Val Met Lys Asp Gly Ile Ala Thr Gly
625                 630                 635                 640

Gly Pro Asn Asn Asp Gly Gly Ile Leu Lys Gly Val Lys Leu Glu Tyr
                645                 650                 655

Ile Gly Asn Lys Leu Tyr Val Arg Gly Leu Asn Leu Gly Glu Gly Gln
            660                 665                 670

Lys Val Thr Leu Thr Tyr Asp Val Lys Leu Asp Asp Ser Phe Ile Ser
        675                 680                 685

Asn Lys Phe Tyr Asp Thr Asn Gly Arg Thr Thr Leu Asn Pro Lys Ser
    690                 695                 700

Glu Asp Pro Asn Thr Leu Arg Asp Phe Pro Ile Pro Lys Ile Arg Asp
705                 710                 715                 720

Val Arg Glu Tyr Pro Thr Ile Thr Ile Lys Asn Glu Lys Lys Leu Gly
                725                 730                 735

Glu Ile Glu Phe Ile Lys Val Asp Lys Asp Asn Asn Lys Leu Leu Leu
            740                 745                 750

Lys Gly Ala Thr Phe Glu Leu Gln Glu Phe Asn Glu Asp Tyr Lys Leu
        755                 760                 765

Tyr Leu Pro Ile Lys Asn Asn Asn Ser Lys Val Val Thr Gly Glu Asn
    770                 775                 780

Gly Lys Ile Ser Tyr Lys Asp Leu Lys Asp Gly Lys Tyr Gln Leu Ile
785                 790                 795                 800

Glu Ala Val Ser Pro Glu Asp Tyr Gln Lys Ile Thr Asn Lys Pro Ile
                805                 810                 815

Leu Thr Phe Glu Val Val Lys Gly Ser Ile Lys Asn Ile Ile Ala Val
            820                 825                 830

Asn Lys Gln Ile Ser Glu Tyr His Glu Glu Gly Asp Lys His Leu Ile
        835                 840                 845

Thr Asn Thr His Ile Pro Pro Lys Gly Ile
    850                 855